# EUROPEAN PATENT APPLICATION

(11) **EP 4 595 977 A1**
(43) Date of publication of application: **06.08.2025**
(21) Application number: 23870632.9
(22) Date of filing: 22.09.2023
(51) Int. Cl.: A61K 47/54, A61K 47/68, A61K 38/26, C07K 19/00, C07K 16/00, C07K 16/22, A61P 3/10

(54) **ULTRA-LONG-ACTING PLATFORM COMPRISING FC-ADVANCED FATTY ACID CHAIN**

(30) Priority: 26.09.2022 WO PCT/CN2022/121350
(71) Applicant: Waterstone Pharmaceuticals (Wuhan) Co., Ltd., Wuhan, Hubei 430075 (CN)
(72) Inventor: ZHANG, Faming, Wuhan, Hubei 430075 (CN); HU, Yimin, Wuhan, Hubei 430075 (CN); ZHOU, Yuan, Wuhan, Hubei 430075 (CN); YU, Yao, Wuhan, Hubei 430075 (CN); HU, Minglong, Wuhan, Hubei 430075 (CN); WANG, Xiaolong, Wuhan, Hubei 430075 (CN); ZHAO, Along, Wuhan, Hubei 430075 (CN); LIANG, Ying, Wuhan, Hubei 430075 (CN)
(74) Representative: Nederlandsch Octrooibureau
(86) International application number: PCT/CN2023/120731
(87) International publication number: WO 2024/067401

(57) **Abstract**

The present invention relates to an ultra-long-acting platform for improving the half-life of an active molecule of a drug, which comprises immunoglobulin Fc and an advanced advanced fatty acid chain. The present invention also relates to preparation of the conjugate platform, a composition compsring the conjugate, and a treatment application thereof. The present invention specifically relates to a conjugated molecule having a structure of "active molecule-Fc-Cn", wherein the active molecule is selected from any molecules beneficial to an organism, Fc is immunoglobulin IgG Fc, and Cn is a modified moiety comprising a C₁₄-₂₄ fatty acid chain. The present invention also relates to preparation of the conjugated molecule, a composition compsring the conjugate and a treatment application thereof.

## Description

### TECHNICAL FIELD

The present invention relates to an ultra-long-acting platform for improving the half-life of an active molecule of a drug, which comprises immunoglobulin Fc and an advanced fatty acid chain. The present invention also relates to the preparation of the conjugate platform, a composition ccomprising the conjugate and a treatment application thereof.

### BACKGROUND OF THE INVENTION

Polypeptide drugs, which are more similar to endogenous substances of an organism than chemical drugs, have the advantages of less toxic and side effects, stable curative effect and the like, thus being widely applied to various clinical treatments such as cancers, cardiovascular diseases, and autoimmune diseases, having a wide application prospect, and also being popular with pharmaceutical manufacturers and scientific researchers. However, classical polypeptide drugs will be degraded quickly after entering the body due to low tolerance to proteases in vivo and poor stability, causing a short plasma half-life; most bioactive peptide substances with poor bioavailability cannot be orally taken, so that the clinical application of the polypeptide drugs is greatly hindered by these problems. In addition, numerous of protein and polypeptide drugs are given more frequently to maintain clinically effective drug concentrations due to limited target tissue exposure.

In order to solve these problems, polypeptide drugs are modified currently. The modification of the polypeptide drugs can be classified into two types, one type is the modification of a peptide chain framework; the second type is the introduction of other groups to carry out structure optimization and performance modification on the basis of keeping the polypeptide framework unchanged, including polyethylene glycol modification, glycosylation modification, protein fusion strategy, advanced fatty acid modification, site-specific mutagenesis, cholesterol modification, and the like.

A Fc-fusion protein that is formed by the fusion of a biologically active molecules to a Fc-region of immunoglobulin combines the beneficial pharmacological properties of the biologically active molecule with additional properties of the Fc-region, thereby increasing the serum half-life of the physiologically active molecule and thereby reducing the administration frequency. Currently, the fusion of an Fc-region to an active peptide as a ligand or receptor or to the extracellular domain (ECD) greatly enhances the clinical potential of active protein drugs. In particular, for a product with a molecular weight of less than 60 kDa, it would be readliy cleared by the kidney, causing a short serum half-life; conjugating or fusing to the Fc-region will increase the size of the product, thus exceed the renal filtration threshold, and will increase the circulation time of the product. When entering into the acidified endosome after being uptaken by endothelial cells, the Fc-fusion protein is protected from lysosomal degradation via the binding of Fc to FcRn in the endosome; and when is transported to the cell surface by the recycling endosome, Fc dissociates from FcRn under neutral and slightly alkaline conditions, releasing the Fc-fusion protein back into the blood circulation, thereby extending the half-life of the Fc-fusion protein, allowing longer exposure of the target tissue to the pharmacologically active moiety of the Fc-fusion protein, thus increasing the therapeutic potential of the Fc-fusion protein.

Fc-fusion proteins represent a successful class of biopharmaceutical products, with 13 drugs approved in the Europe and the US, and 3 etanercept biosimilars. The potential biologically active molecules are of great diversity, including the extracellular domains of natural receptors, functionally active peptides, recombinases, and genetically engineered binding structures as cytokine traps. Most Fc-fusion proteins are produced by the fusion of biologically active molecules to the N-terminus of Fc domains. The strong interaction of the IgG-CH3 domains creates a stable Fc structure and allows for more complex structures to be fused to position such as flexible hinge regions, disulfide bonds, etc. Dulaglutide, a hypoglycemic drug developed via the fusion of GLP-1 to IgG4 (Fc) by Eli Lilly and Company, has an extended biological half-life due to the reduced renal clearance of GLP-1 caused by a remarkably increase of molecular size.

Fatty acids, which serve as important constituents of human body fats, lipids, and cell membrane phospholipids, have low immunogenicity as endogenous components, and thus are also used to modify biologically active molecules (e.g., polypeptide drugs). When a specific amino acid residue of a polypeptide drug is modified with a fatty acid, the serum half-life of the polypeptide drug is increased by the reversible binding of the fatty acid to serum albumin. However, the fatty acid modifications also have their own limitations: for example, it is readily to produce non-specific modification site products of fatty acids; polypeptide drugs that dissociate from albumin are readily eliminated by the kidney due to the reversible binding of fatty acids to serum albumin, thus affecting or reducing the half-life of the fatty acid-modified polypeptide drugs.

On the premise of retaining the curative effect of a polypeptide drug, there is always a demand on the increase of the serum half-life of the polypeptide drug and the reduction of the immunogenicity of the drug in the fields of scientific research and pharmaceuticals, which has been met in this application by providing an innovative ultra-long-acting platform for improving an active molecule of a drug.

### SUMMARY

The present invention provides an ultra-long-acting platform for improving the half-life of an active molecule of a drug, which has the following structures: an active molecule-fusion protein-Cn conjugate platform, an active molecule-Fc-Cn conjugate platform, and an antibody-Cn conjugate platform, wherein Cn represents a modified moiety comprising a fatty acid chain of n =14-24, and Fc is an Fc-region of an immunoglobulin molecule. The ultra-long-acting platform provided by the present application has the following advantages:
1.Both the Fc component and the advanced fatty acid chain component of the conjugated molecule can bind to FcRn directly or indirectly, with Fc binding directly to FcRn and the advanced fatty acid binding indirectly to FcRn via serum albumin, wherein Fc and serum albumin do not interfere with each other as they bind to different sites of FcRn, thereby providing a longer half-life for the active molecule conjugated thereto than that of Fc alone or the advanced fatty acid alone;
2.All of the fusion protein, the antibody, Fc and advanced fatty acid are endogenous substances *in vivo* with low immunogenicity, thus reducing the heterology of conjugated molecules to an organism, and reducing the possibility of generating corresponding antibodies;
3.The conjugated molecule can increase the size of the contained active molecule and reduce the renal excretion rate, thereby extending the circulation time of the active molecule *in vivo;*
4.The hydrophobic nature of the fatty acid chains contributes to improve the membrane permeability of the conjugated molecules, thereby allowing more conjugated molecules to enter the circulatory system;
5.The Fc in the conjugated molecule, via its CH2-3 domain, can pair with a homologous Fc fragment, thus increasing stability and improving the concentration of locally conjugated/active molecules.

In a first aspect, the present invention provides a conjugated molecule having a structure of "active molecule-Fc-Cn", a conjugated molecule having a structure of "active molecule-fusion protein-Cn", and a conjugated molecule having a structure of "antibody-Cn", wherein the active molecule is selected from any molecules beneficial to an organism, Fc is derived from the heavy chain constant region of an immunoglobulin IgG, and Cn is a modified moiety comprising a C₁₄-₂₄ fatty acid chain.

In some embodiments, the present invention provides a conjugated molecule having a structure of "active molecule-Fc-Cn", a conjugated molecule having a structure of "active molecule-fusion protein-Cn", and a conjugated molecule having a structure of "antibody-Cn", wherein Cn has the structure of Formula (I) below:

-Z-Y (I),

wherein
Z has the following structure:

   -Z₁-Z₂-Z₃-Z₄-,
wherein Z₁ is a sulfur atom or a nitrogen atom or an oxygen atom in Fc,
   Z₂ is -C(=O)- or 5-10 membered heterocyclyl, preferably containing 1 or 2 heteroatoms selected from N, S, and O;
   Z₃ is selected from the group consisting of a bond, -C(=O)-, -C₁-C₁₀ alkylene-C(=O)-, -C₃-C₁₀ alkynylene-C(=O)-, -C₃-C₁₀ alkenylene-C(=O)-, -C₁-C₁₀ heteroalkylene-C(=O)-, -C₃-C₈ cycloalkylene-C( = O)-, -O-C₁-C₈ alkylene-C( = O)-, -arylidene-C( = O)-, -C₁-C₁₀ alkylene-arylidene-C(=O)-, -arylidene-C₁₋C₁₀ alkylene-C(=O)-, -C₁-C₁₀ alkylene-C₃-C₈ cycloalkylene-C(= O)-, -C₃-C₈ cycloalkylene-C₁-C₁₀ alkylene-C( = O)-, -C₃-C₈ heterocyclylene-C( = O)-, -C₁-C₁₀ alkylene-C₃-C₈ heterocyclylene-C(=O)-, -C₃-C₈ heterocyclylene-C₁-C₁₀ alkylene-C(=O)-, wherein the alkylene, alkynylene, alkenylene, heteroalkylene, cycloalkylene, arylidene, and heterocyclylene are substituted optionally;
   Z₄ is a bond or a PEG unit represented by the following Formula, 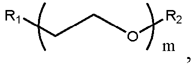 wherein R₁ is selected from the group consisting of C₁₋₄ alkylene, -NH-, -NH-C₁₋₄ alkylene-, -NH-C₁₋₄ alkylene-heteroaryl-, wherein heteroaryl is 5-membered or 6-membered nitrogen-containing heteroaryl; R₂ is-C(=O)-, -C₁₋₄ alkylene, -C₁₋₄ alkylene-C(=O)-, -C₁₋₄ alkylene-NH-C(=O)-(CH₂OCH₂)ₚ-C₁₋₄ alkylene-, -C₁₋₄ alkylene-C(=O)-NH-(CH₂OCH₂)ₚ-C₁₋₄ alkylene-, wherein m is an integer from 2 to 6, p is an integer from 1 to 3,
Y is 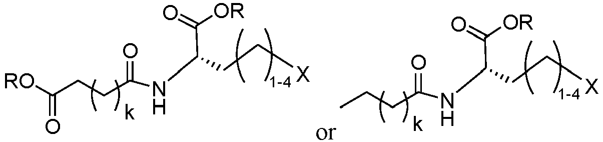
wherein Y is linked to Z₄ via X, k is an integer from 10 to 30,
wherein R independently represents hydrogen, C₁₋₆ alkyl, C₁₋₆ aminoalkyl, C₁₋₆ haloalkyl, C₁₋₆ hydroxyalkyl.

In some embodiments, Z₂ is maleimido group 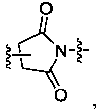 wherein the wavy line on the left side indicates the position to which Z₁ is linked; and the wavy line on the right side indicates the position to which Z₃ is linked.

In some embodiments, Z₃ is -C₁-C₁₀ alkylene-C(=O)-, wherein the alkylene is optionally substituted and wherein Z₃ is linked to Z₄ via -C-(=O)-.

In some preferred embodiments, Z₂ is a maleimido group, and Z₃ is -C₁₋₆ alkylene-C(=O)-.

In some embodiments, Z₄ is a bond and Z₃ is directly linked to Y in Formula (I).

In some embodiments, Z₄ is a PEG unit represented by the following Formula, 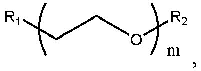 wherein R₁ is selected from the group consisting of -NH- and -NH-C₁₋₄ alkylene-; R₂ is -C₁₋₄ alkylene or -C₁₋₄ alkylene-NH-C(=O)-(CH₂OCH₂)ₚ-C₁₋₄ alkylene-, wherein m is an integer from 2 to 6 and p is an integer from 1 to 3.

In some embodiments, Z₄ is a unit comprising 2-6 PEGs. In some embodiments, Z₄ is 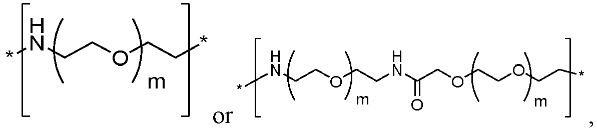 wherein m =1-4, the asterisk on the left side indicates the position to which Z₃ is linked; and the asterisk on the right side indicates the position to which Y in Formula II is linked.

In some embodiments, Z in Formula (I) of the present invention has the following structures: 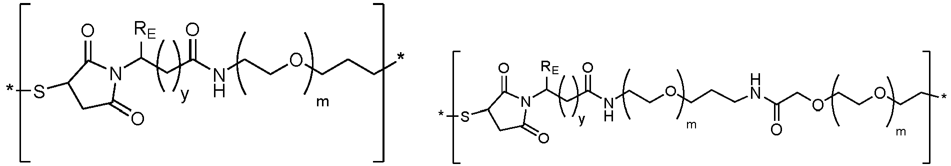 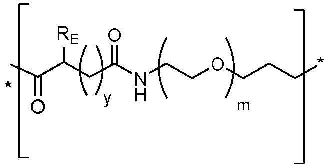 or 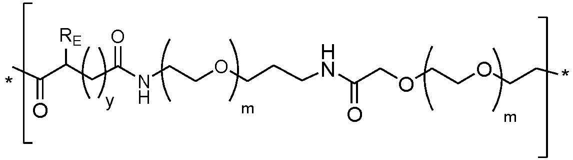 wherein R_{E} is hydrogen, C₁₋₆ alkyl, C₁₋₆ aminoalkyl, C₁₋₆ haloalkyl, C₁₋₆ hydroxyalkyl, wherein y =0-4, m =1-4, wherein the asterisk on the left side indicates the position to which Ab is linked and the asterisk on the right side indicates the position to which Y is linked.
Y is 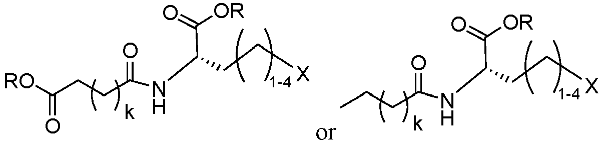
wherein Y is linked to Z₄ via X, and X is -NH-(C=O)- or -(C=O)-NH-,
k is an integer from 10 to 30,
wherein R independently represents hydrogen, C₁₋₆ alkyl, C₁₋₆ aminoalkyl, C₁₋₆ haloalkyl, C₁₋₆ hydroxyalkyl.

In one particular embodiment, Cn is selected from

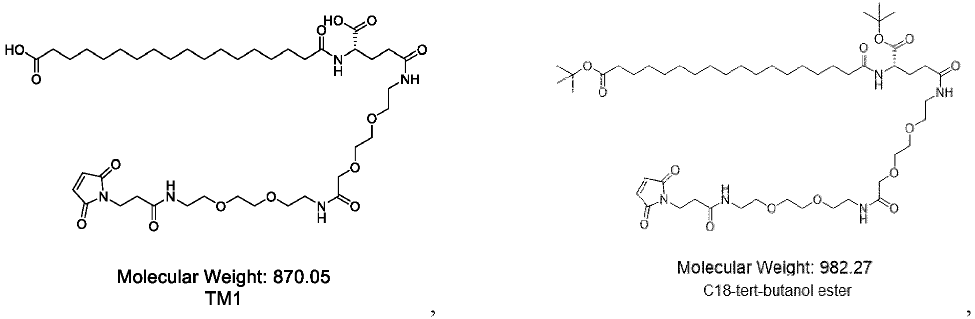

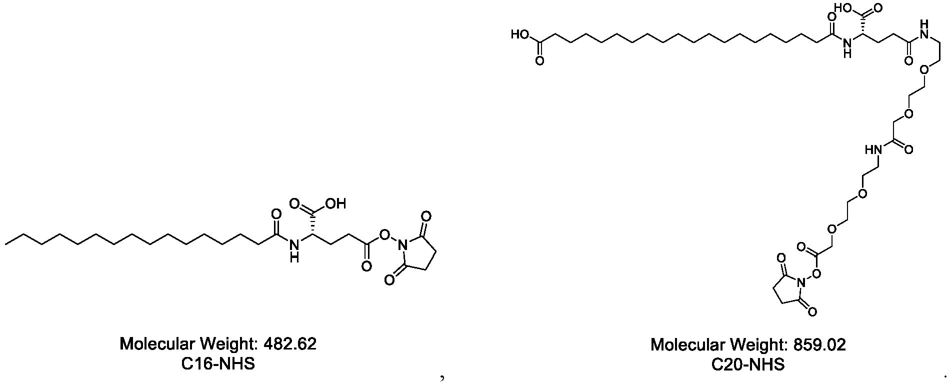

In one embodiment, Fc is drived from the heavy chain constant region of IgG1, IgG2, IgG3, or IgG4. In one particular embodiment, Fc is drived from the heavy chain constant region of IgG1 or IgG4. In yet another embodiment, the Fc-region may further comprise a hinge region. In one particular embodiment, Fc comprises amino acid modifications. In one particular embodiment, the modification of the Fc-region is a modification at positions 254, 308, and 434 (according to EU numbering). In another particular embodiment, the modification of the Fc-region is replacing amino acids at positions 254, 308, and 434 with Thr, Pro, and Ala, respectively. In one particular embodiment, the modification of the Fc-region is a modification at positions 228, 234, 235 and/or 447, such as the modifications S228P, F234A, L235A or the modifications S228P, F234A, L235A and a deletion at position 447. In one particular embodiment, the Fc-region is selected from the sequences of SEQ ID NO: 10, 15, or 16.

In one embodiment, the active molecule is an active peptidic molecule. In one embodiment, the active peptidic molecule is fused to an antibody, a fusion protein, or a Fc-region directly or via a peptide linker. In one embodiment, the C-terminus of the active peptidic molecule is fused to the N-terminus of the antibody, the fusion protein, or the Fc-region. Alternatively, the N-terminus of the active peptidic molecule is fused to the C-terminus of the antibody, the fusion protein, or the Fc-region. In yet another particular embodiment, the active peptidic molecule is linked to the antibody, the fusion protein, or the Fc-region in monomeric form. In yet further particular embodiment, the active peptidic molecule is linked to the antibody, the fusion protein, or the Fc-region in a multimeric form.

In one embodiment, the active molecule is selected from the group consisting of an enzyme, an enzyme inhibitor, an antigen, an antibody or an antibody fragment, hormone, glucagon-like peptide-1 (GLP-1), glucagon, an interferon, a cytokine, a growth factor and/or a differentiation factor, a factor involved in cell motility or migration, a factor involved in bone formation/resorption, a chemokine, a plasma or interstitial adhesion molecule or extracellular matrix, a bactericidal or antifungal factor, and the like.

In one particular embodiment, the active molecule is selected from the group consisting of GLP-1, an antibody Fab fragment, and an antibody F(ab')₂ fragment. In another particular embodiment, the active molecule is selected from the group consisting of an anti-PD-1 antibody Fab fragment, an anti-PD-1 antibody F(ab')₂ fragment, an anti-VEGF antibody Fab fragment, and an anti-VEGF antibody F(ab')₂ fragment.

In one embodiment, the peptide linker comprises the amino acid sequence (G4S)n, wherein n is an integer equeal to or greater than 1. In one particular embodiment, the peptide linker comprises (G₄S)₃, (G₄S)₄, (G₄S)₆, GS(G₄S)₄, DAAALEAAALDAAAREAAARDAAAL, NVDHLPSNTLVDLA, (G₃S)2, (G₄S)₂, (G₃S)₃, (G₄S)₃, (G₃S)₄, (G₄S)₄, (G₃S)₅, (G₄S)₅, (G₃S)₆, (G₄S)₆, GGG, DGGGS, TGEKP, GGRR, EGKSSGSGSESKVD, KESGSVSSEQLAQFRSLD, GGRRGGGS, LRQRDGERP, LRQKDGGGSERP, and GSTSGSGK PGSGEGSTKG.

In one embodiment, a conjugated molecule having a structure of "active molecule-Fc-Cn" or "antibody-Cn" provided in the present application is homodimerized via its Fc-region.

In one particular embodiment, the present application provides a conjugated molecule having a structure of "active molecule-Fc-Cn", wherein Fc is selected from IgG1 or IgG4, and Cn comprises a fatty acid chain of 16, 18, or 20 carbons. In one preferred embodiment, the Fc comprises a modification, such as those mentioned herein. In one particular embodiment, Cn comprises a fatty acid chain of 16 carbons, in another particular embodiment, Cn comprises a fatty acid chain of 18 carbons, and in yet another particular embodiment, Cn comprises a fatty acid chain of 20 carbons.

In one particular embodiment, the present application provides a conjugated molecule having a structure of "antibody-Cn", wherein the antibody is selected from IgG1 or IgG4, and Cn comprises a fatty acid chain of 16, 18, or 20 carbons. In one preferred embodiment, the Fc of the antibody comprises a modification, such as those mentioned herein. In one particular embodiment, Cn comprises a fatty acid chain of 16 carbons, in another particular embodiment, Cn comprises a fatty acid chain of 18 carbons, and in yet another particular embodiment, Cn comprises a fatty acid chain of 20 carbons.

In one particular embodiment, the present application provides a conjugated molecule having a structure of "active molecule-Fc-Cn", wherein Fc is selected from IgG1 or IgG4, and Cn comprises a fatty acid chain of 16, 18, or 20 carbons and is coupled/conjugated to a free thiol group of Fc. In one preferred embodiment, the Fc comprises a modification, such as those mentioned herein. **In** one particular embodiment, Cn comprises a fatty acid chain of 16 carbons, in another particular embodiment, Cn comprises a fatty acid chain of 18 carbons, and in yet another particular embodiment, Cn comprises a fatty acid chain of 20 carbons.

**In** one particular embodiment, the present application provides a conjugated molecule having a structure of "antibody-Cn", wherein the antibody is selected from IgG1 or IgG4, and Cn comprises a fatty acid chain of 16, 18, or 20 carbons and is coupled/conjugated to the sulfur atom of the free thiol group of the antibody Fc. **In** one preferred embodiment, the Fc of the antibody comprises a modification, such as those mentioned herein. In one particular embodiment, Cn comprises a fatty acid chain of 16 carbons, in another particular embodiment, Cn comprises a fatty acid chain of 18 carbons, and in yet another particular embodiment, Cn comprises a fatty acid chain of 20 carbons.

In one particular embodiment, the present application provides a conjugated molecule having a structure of "active molecule-fusion protein-Cn", wherein Cn comprises a fatty acid chain of 16, 18, or 20 carbons and is coupled/conjugated to the sulfur atom of the free thiol group of the fusion protein. In one particular embodiment, Cn comprises a fatty acid chain of 16 carbons, in another particular embodiment, Cn comprises a fatty acid chain of 18 carbons, and in yet another particular embodiment, Cn comprises a fatty acid chain of 20 carbons.

In one particular embodiment, the present application provides a conjugated molecule having a structure of "active molecule-IgG4 Fc-Cn", wherein Cn comprises a fatty acid chain of 16, 18 or 20 carbons and is coupled/conjugated to the free thiol group of IgG4 Fc. In one preferred embodiment, the IgG4 Fc comprises a modification, such as those mentioned herein. In one particular embodiment, Cn comprises a fatty acid chain of 16 carbons, in another particular embodiment, Cn comprises a fatty acid chain of 18 carbons, and in yet another particular embodiment, Cn comprises a fatty acid chain of 20 carbons.

In one particular embodiment, the present application provides a conjugated molecule having a structure of "antibody-Cn", wherein Cn comprises a fatty acid chain of 16, 18, or 20 carbons and is coupled/conjugated to the sulfur atom of the free thiol group of IgG4 antibody. In one preferred embodiment, the Fc of the IgG4 antibody comprises a modification, such as those mentioned herein. In one particular embodiment, Cn comprises a fatty acid chain of 16 carbons, in another particular embodiment, Cn comprises a fatty acid chain of 18 carbons, and in yet another particular embodiment, Cn comprises a fatty acid chain of 20 carbons.

In one particular embodiment, the present application provides a conjugated molecule having a structure of "active molecule-fusion protein-Cn", wherein Cn comprises a fatty acid chain of 16, 18, or 20 carbons, and is coupled/conjugated to the sulfur atom of the free thiol group of the fusion protein. In one particular embodiment, Cn comprises a fatty acid chain of 16 carbons, in another particular embodiment, Cn comprises a fatty acid chain of 18 carbons, and in yet another particular embodiment, Cn comprises a fatty acid chain of 20 carbons.

In one particular embodiment, the present application provides a conjugated molecule having a structure of "active molecule-IgG1 Fc-Cn", wherein Cn comprises a fatty acid chain of 16, 18, or 20 carbons and is coupled/conjugated to the sulfur atom of the free thiol group of IgG1 Fc. In one preferred embodiment, the IgG1 Fc comprises a modification, such as those mentioned herein. In one particular embodiment, Cn comprises a fatty acid chain of 16 carbons, in another particular embodiment, Cn comprises a fatty acid chain of 18 carbons, and in yet another particular embodiment, Cn comprises a fatty acid chain of 20 carbons.

In one particular embodiment, the present application provides a conjugated molecule having a structure of "antibody-Cn", wherein Cn comprises a fatty acid chain of 16, 18, or 20 carbons and is coupled/conjugated to the sulfur atom of the free thiol group of IgG1 antibody. In one preferred embodiment, the Fc of the IgG1 antibody comprises a modification, such as those mentioned herein. In one particular embodiment, Cn comprises a fatty acid chain of 16 carbons, in another particular embodiment, Cn comprises a fatty acid chain of 18 carbons, and in yet another particular embodiment, Cn comprises a fatty acid chain of 20 carbons.

In one particular embodiment, the present application provides a conjugated molecule having a structure of "active molecule-fusion protein-Cn", wherein Cn comprises a fatty acid chain of 16, 18, or 20 carbons and is coupled/conjugated to the sulfur atom of the free thiol group of the fusion protein. In one particular embodiment, Cn comprises a fatty acid chain of 16 carbons, in another particular embodiment, Cn comprises a fatty acid chain of 18 carbons, and in yet another particular embodiment, Cn comprises a fatty acid chain of 20 carbons.

In one particular embodiment, the conjugated molecule having a structure of "active molecule-Fc-Cn" in the present application is a GLP-1-Fc-Cn conjugated molecule, wherein the GLP-1 is any active GLP-1 known in the art. In one embodiment, Fc is selected from Fc of IgG1 or Fc of IgG4. In one particular embodiment, Cn in the conjugated molecule GLP-1-Fc-Cn comprises a fatty acid chain of 16, 18, or 20 carbons and is coupled/conjugated to the sulfur atom of the free thiol group of Fc or the nitrogen atom of Fc. In one particular embodiment, Cn in the conjugated molecule GLP-1-Fc-Cn is selected from TM1, C18-tert-butanol ester, C16-NHS, or C20-NHS. In one preferred technical solution, the conjugated molecule GLP-1-Fc-Cn is GLP-1-IgG4 Fc-TM1, GLP-1-IgG4 Fc-C18 tert-butanol ester, GLP-1-IgG4 Fc-C16-NHS, GLP1-IgG4 Fc-C20-NHS. In one embodiment, Fc comprises a sequence as set forth in SEQ ID NO: 10, 15, or 16. In one more preferred embodiment, the GLP-1-IgG4 Fc in GLP-1-IgG4 Fc-TM1, GLP-1-IgG4 Fc-C18, GLP-1-IgG4 Fc-C16-NHS, and GLP1-IgG4 Fc-C20-NHS conjugated molecules is derived from dulaglutide, preferably is dulaglutide, e.g., having a structure as disclosed in CN1802167, preferably having a structure of Gly⁸-Glu²²-Gly³⁶-GLP-1(7-37)-1L-IgG4 (S228P, F234A, L235A). In one embodiment, the amino acid sequence of dulaglutide is as set forth in SEQ ID NO. 1. In one particular embodiment, the GLP-1-Fc-Cn conjugated molecule is dulaglutide-Cn, such as dulaglutide-TM1, dulaglutide-C18 tert-butanol ester, dulaglutide-C16-NHS, dulaglutide-C20-NHS.

In one particular embodiment, the active molecule in the conjugated molecule having a structure of "active molecule-Fc-Cn" in the present application is an anti-PD-1 antibody or antigen-binding fragment thereof, which can be any known anti-PD-1 antibody or antigen-binding fragment thereof. In one particular embodiment, the antibody in the conjugated molecule having a structure of "antibody-Cn" in the present application is an anti-PD-1 antibody or antigen-binding fragment thereof, which can be any known anti-PD-1 antibody or antigen-binding fragment thereof. In one preferred embodiment, the Fc is selected from IgG1 Fc or IgG4 Fc. In one preferred embodiment, the antibody is selected from IgG1 or IgG4. In one preferred technical solution, the conjugated molecule is antigen binding fragment of anti-PD-1 antibody-IgG4 Fc-TM1, antigen binding fragment of anti-PD-1 antibody-IgG4 Fc-C18, antigen binding fragment of anti-PD-1 antibody-IgG4 Fc-C16-NHS, and antigen binding fragment of anti-PD-1 antibody-IgG4 Fc-C20-NHS. In one preferred technical solution, the conjugated molecule is antigen binding fragment of anti-PD-1 antibody-IgG1 Fc-TM1, antigen binding fragment of anti-PD-1 antibody-IgG1 Fc-C18, antigen binding fragment of anti-PD-1 antibody-IgG1 Fc-C16-NHS, and antigen binding fragment of anti-PD-1 antibody-IgG1 Fc-C20-NHS. In one preferred embodiment, the anti-PD-1 antibody or antigen-binding fragment thereof comprises a heavy chain variable region as set forth in SEQ ID NO: 9 and a light chain variable region as set forth in SEQ ID NO: 11. In one preferred embodiment, the Fc comprises a sequence as set forth in SEQ ID NO: 10. In one more preferred embodiment, the anti-PD-1 antibody or antigen-binding fragment thereof comprises a heavy chain as set forth in SEQ ID NO: 8 and a light chain as set forth in SEQ ID NO: 12. In one more preferred embodiment, the Cn is selected from the group consisting of TM1, C18-tert-butanol ester, C16-NHS, and C20-NHS. In a preferred embodiment, the Cn is selected from TM1.

In one particular embodiment, the active molecule in the conjugated molecule having a structure of "active molecule-Fc-Cn" in the present application is an anti-VEGF antibody or antigen-binding fragment thereof, which can be any known anti-VEGF antibody or antigen-binding fragment thereof. In one particular embodiment, the antibody in the conjugated molecule having a structure of "antibody-Cn" in the present application is an anti-VEGF antibody or antigen-binding fragment thereof, which can be any known anti-VEGF antibody or antigen-binding fragment thereof. In one preferred embodiment, the Fc is selected from IgG1 Fc or IgG4 Fc. In one preferred embodiment, the antibody is selected from IgG1 or IgG4. In one preferred technical solution, the conjugated molecule is antigen binding fragment of anti-VEGF antibody-IgG4 Fc-TM1, antigen binding fragment of anti-VEGF antibody-IgG4 Fc-C18, antigen binding fragment of anti-VEGF antibody-IgG4 Fc-C16-NHS, and antigen binding fragment of anti-VEGF antibody-IgG4 Fc-C20-NHS. In one preferred technical solution, the conjugated molecule is antigen binding fragment of anti-VEGF antibody-IgG1 Fc-TM1, antigen binding fragment of anti-VEGF antibody-IgG1 Fc-C18-tert-butanol ester, antigen binding fragment of anti-VEGF antibody-IgG1 Fc-C16-NHS, and antigen binding fragment of anti-VEGF antibody-IgG1 Fc-C20-NHS. In one preferred embodiment, the anti-VEGF antibody or antigen-binding fragment thereof comprises 3 heavy chain CDRs as set forth in SEQ ID NOs: 2, 3, and 4, and 3 light chain CDRs as set forth in SEQ ID NO: 5, 6, and 7. In one preferred embodiment, the Fc comprises a sequence as set forth in SEQ ID NO: 15. In one more preferred embodiment, the anti-VEGF antibody or antigen-binding fragment thereof comprises a heavy chain as set forth in SEQ ID NO: 13 and a light chain as set forth in SEQ ID NO: 14. In one more preferred embodiment, the Cn is selected from the group consisting of TM1, C18-tert-butanol ester, C16-NHS, and C20-NHS. In a preferred embodiment, the Cn is selected from TM1.

In a second aspect, the present application provides a method for preparing a conjugated molecule having a structure of "active molecule-Fc-Cn", comprising (a) linking an active molecule polypeptide to an immunoglobulin Fc-region to prepare an "active molecule-Fc" fusion; and (b) subjecting the "active molecule-Fc" fusion and a Cn containing a fatty acid chain to a conjugating reaction under the conditions that allow the conjugation of the Fc-region to Cn, resulting in an "active molecule-Fc-Cn" conjugated molecule.

The present application also provides a method for preparing a conjugated molecule having a structure of "antibody-Cn", comprising (a) subjecting an antibody and a Cn containing a fatty acid chain to a conjugating reaction under the conditions that allow the conjugation of the antibody to Cn, resulting in an "antibody-Cn" conjugated molecule.

The present application also provides a method for preparing a conjugated molecule having a structure of "active molecule-fusion protein-Cn", comprising (a) linking an active molecule polypeptide to a fusion protein to prepare an "active molecule-fusion protein" fusion; and (b) subjecting the "active molecule-fusion protein" fusion and a Cn containing a fatty acid chain to a conjugating reaction under the conditions that allow the conjugation of the fusion protein to Cn, resulting in an "active molecule-fusion protein-Cn" conjugated molecule.

In an alternative embodiment, the present application provides a method for preparing a conjugated molecule having a structure of "active molecule-Fc-Cn", comprising the steps of (a) linking an antibody Fab fragment to an immunoglobulin Fc-region to prepare a "Fab-Fc" fusion, and (b) subjecting the "Fab-Fc" fusion to a conjugating reaction under the conditions that allow the conjugation of the Fc-region to Cn containing a fatty acid chain, resulting in an "Fab-Fc-Cn" conjugated molecule. In one embodiment, the Fab and Fc are derived from the same molecule or different antibody molecules.

In an alternative embodiment, the present application provides a method for preparing a conjugated molecule having a structure of "active molecule-Fc-Cn", comprising the step of subjecting a whole antibody to a conjugating reaction under the conditions that allow the conjugation of the Fc-region to Cn containing a fatty acid chain, resulting in an "active molecule-Fc-Cn" conjugated molecule.

In an alternative embodiment, the present application provides a method for preparing a conjugated molecule having a structure of "antibody-Cn", comprising the step of subjecting a whole antibody to a conjugating reaction under the conditions that allow the conjugation of the Fc-region to Cn containing a fatty acid chain, resulting in an "active molecule-Fc-Cn" conjugated molecule.

In a third aspect, the present application provides a composition comprising the conjugated molecule as described in the first aspect, for example, a pharmaceutical composition. **In** one embodiment, the pharmaceutical composition further comprises a pharmaceutically acceptable carrier.

In a fourth aspect, the present application provides a method for effectively extending the serum half-life of an active molecule, comprising the step of constructing the active molecule into a conjugated molecule having a structure of "active molecule-Fc-Cn", a conjugated molecule having a structure of "antibody-Cn", a conjugated molecule having a structure of "active molecule-fusion protein-Cn" according to the method of the second aspect, thereby effectively increasing the serum half-life of the active molecule.

In a fifth aspect, the present application provides the use of the conjugated molecule of the first aspect, or the composition of the third aspect, in the manufacture of a medicament for the treatment of a human disease.

In one embodiment, the present invention provides the conjugated molecule of the first aspect, or the composition of the third aspect, for use in therapy.

In a sixth aspect, the present application provides a method for treating a human disease, comprising administering to a subject an effective amount of the conjugated molecule of the first aspect, or the composition of the third aspect.

### BRIEF DESCRIPTION OF THE DRAWINGS

FIG. 1 shows the results of HIC-HPLC for a GLP1-Fc-TM1 conjugated product, wherein the upper panel is the HIC-HPLC result of GLP1-Fc without conjugating to TM1s, and the lower panel is the HIC-HPLC result of GLP1-Fc-TM1 after conjugating to TM1s, wherein in the lower panel, "0" indicates GLP1-Fc to which no TM1 is conjugated, "2" indicates GLP1-Fc to which 2 TM1s are conjugated, and "4" indicates GLP1-Fc to which 4 TM1s are conjugated.
FIG. 2 shows the results of HIC-HPLC for a HX006-TM1-1 conjugated product, wherein the upper panel is the HIC-HPLC result of HX006 without conjugating to TM1s, and the lower panel is the HIC-HPLC result of HX006-TM1-1 after conjugating to TM1s, wherein in the lower panel, "0" indicates HX006 to which no TM1 is conjugated, "2" indicates HX006 to which 2 TM1s are conjugated, "4" indicates HX006 to which 4 TM1s are conjugated, and "6" indicates HX006 to which 6 TM1s are conjugated.
FIG. 3 shows the results of HIC-HPLC for a HX006-TM1-2 conjugated product, wherein in the panel, "0" indicates HX006 to which no TM1 is conjugated, "2" indicates HX006 to which 2 TM1s are conjugated, "4" indicates HX006 to which 4 TM1s are conjugated, "6" indicates HX006 to which 6 TM1s are conjugated, and "8" indicate HX006 to which 8 TM1s are conjugated.

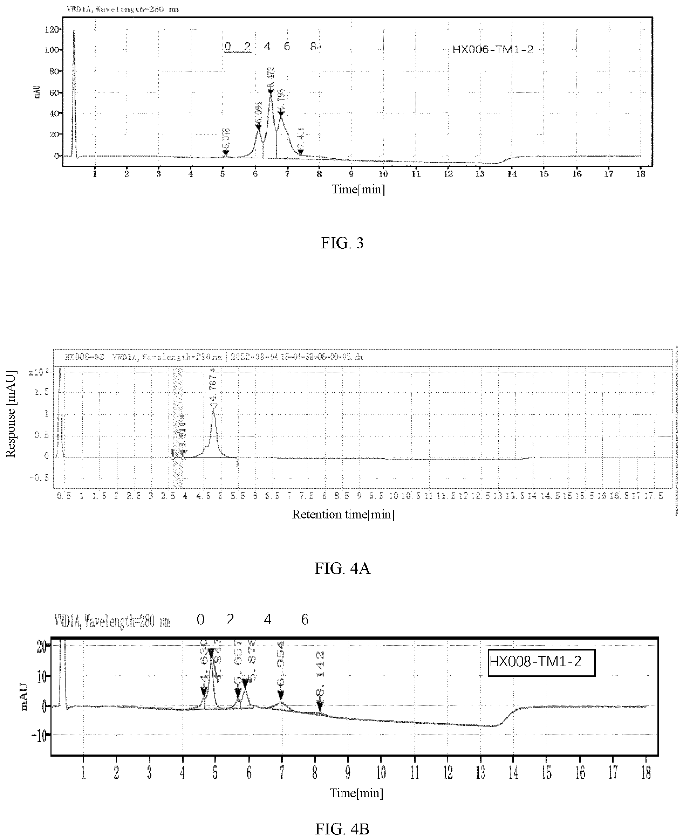

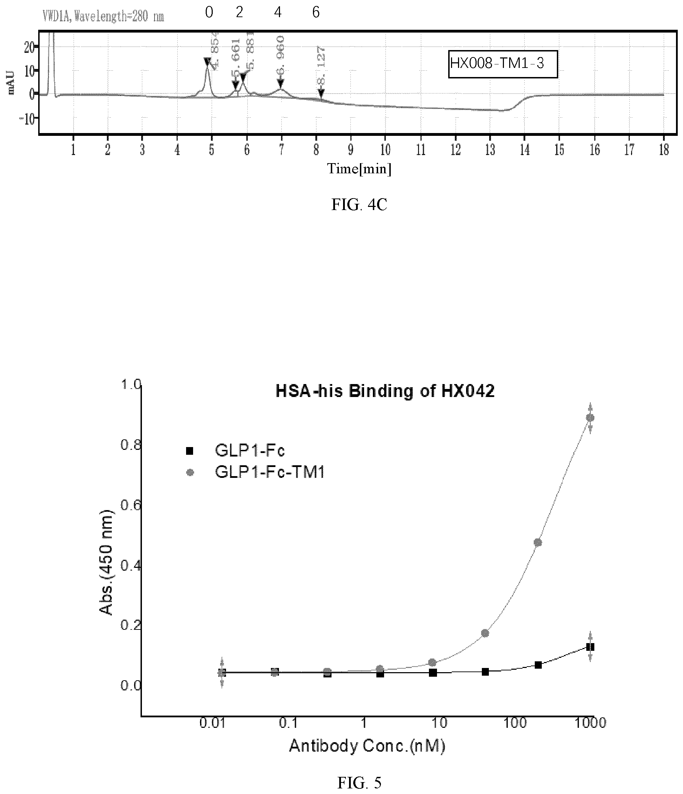

FIG. 4 shows the results of HIC-HPLC for a HX008-TM1 conjugated product, wherein FIG. 4A shows the HIC-HPLC result of HX008 without conjugating to TM1s, FIG. 4B shows the HIC-HPLC result of HX008-TM1-2 after conjugating to TM1s, wherein in the panel, "0" indicates HX008 to which no TM1 is conjugated, "2" indicates HX008 to which 2 TM1s are conjugated, "4" indicates HX008 to which 4 TM1s are conjugated, "6" indicates HX008 to which 6 TM1s are conjugated; and FIG. 4C shows the HIC-HPLC result of HX008-TM1-3 after conjugating to TM1s, wherein in the panel, "0" indicates HX008 to which no TM1 is conjugated, "2" indicates HX008 to which 2 TM1s are conjugated, "4" indicates HX008 to which 4 TM1s are conjugated, and "6" indicates HX008 to which 6 TM1s are conjugated.
FIG. 5 shows the results of an ELISA assay for the binding of GLP1-Fc-TM1 to HSA, wherein HX042 represents GLP1-Fc.
FIG. 6 shows the results of an ELISA assay for the binding of HX006-TM1 to HSA.
FIG. 7 shows the results of an ELISA assay for the binding of HX008-TM1 to HSA.
FIG. 8 shows the results of HIC-HPLC for GLP1-Fc after conjugating to C18-tert-butanol ester, wherein in the panle, "0" indicates GLP1-Fc to which no C18-tert-butanol ester is conjugated and "2" indicates GLP1-Fc to which 2 C18-tert-butanol esters are conjugated.
FIG. 9 shows the results of HIC-HPLC for a GLP1-Fc sample (upper panel) and a GLP1-Fc-C16-NHS sample (lower panel).
FIG. 10 shows the results of HIC-HPLC for a GLP1-Fc-C20-NHS sample.
FIG. 11 shows the results of an ELISA assay for the binding of GLP1-Fc-C16-NHS and GLP1-Fc-C20-NHS to HSA, respectively, wherein HX042 represents GLP 1-Fc.
FIG. 12 shows the results of an ELISA assay for the binding of HX006-C16-NHS and HX006-C20-NHS to HSA, respectively.
FIG. 13 shows the binding activity of GLP1-Fc-C18-tert-butanol ester to HSA.
FIG. 14 shows the binding activity of HX006-C18-tert-butanol ester to HSA.
FIG. 15 shows the results of HIC-HPLC for HX006 after conjugating to C18-tert-butanol ester.
FIG. 16 shows that GLP-1-Fc-TM1 can effectively activate the biological activity of a reporter gene.
FIG. 17 shows the plasma concentration-time curves of GLP1-Fc-TM1 after a single dosing in rats, with dulaglutide as a positive control.
FIG. 18 shows the plasma concentration-time curves of GLP1-Fc-TM1 after a single dosing in cynomolgus monkeys, with dulaglutide as a positive control.

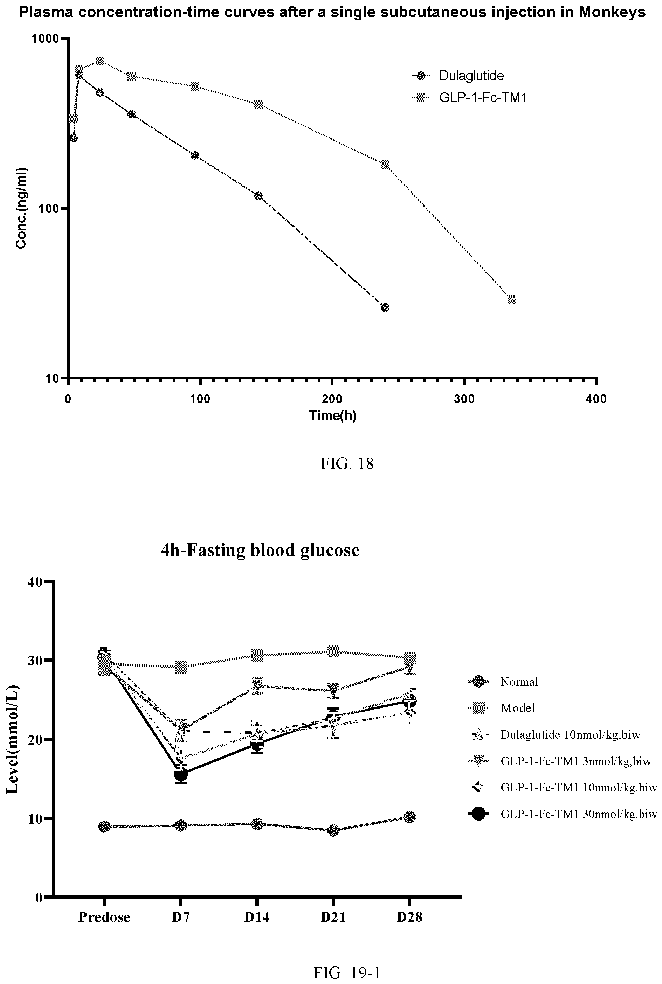

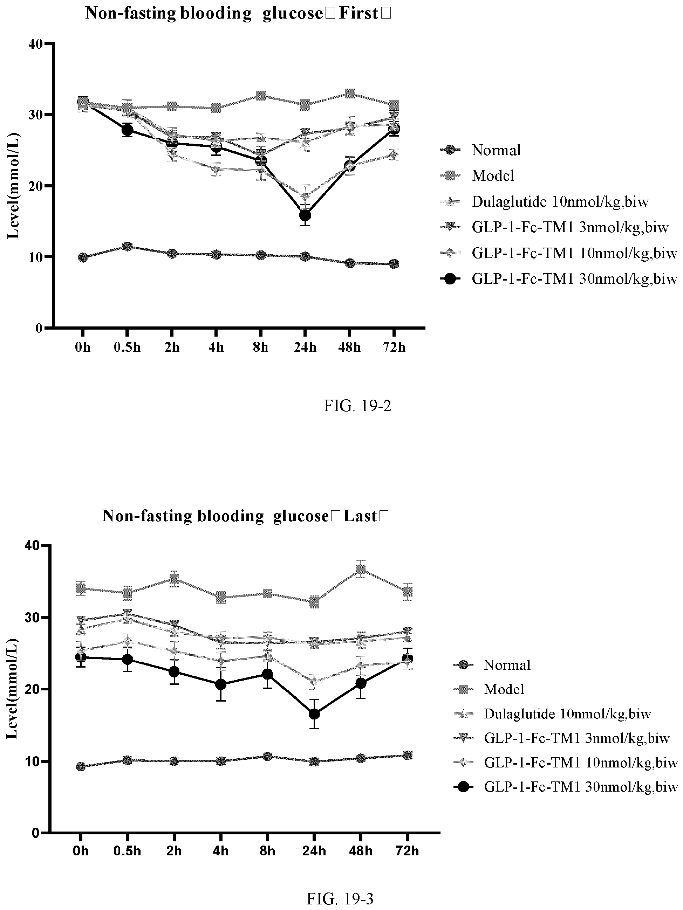

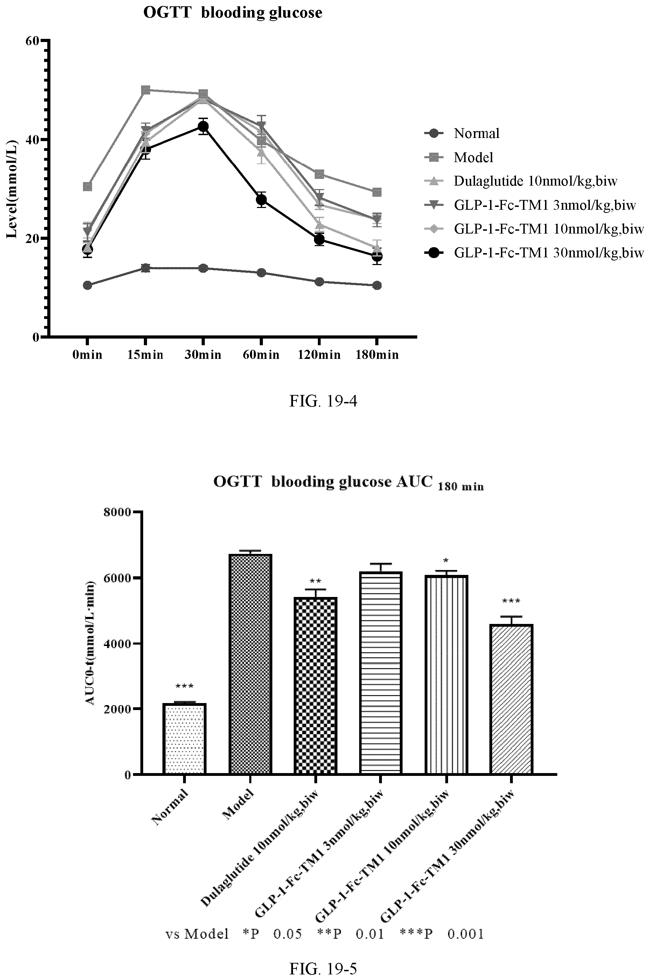

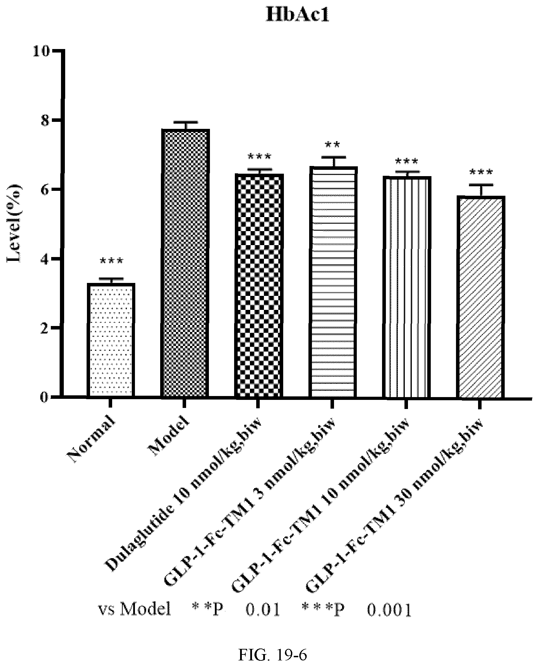

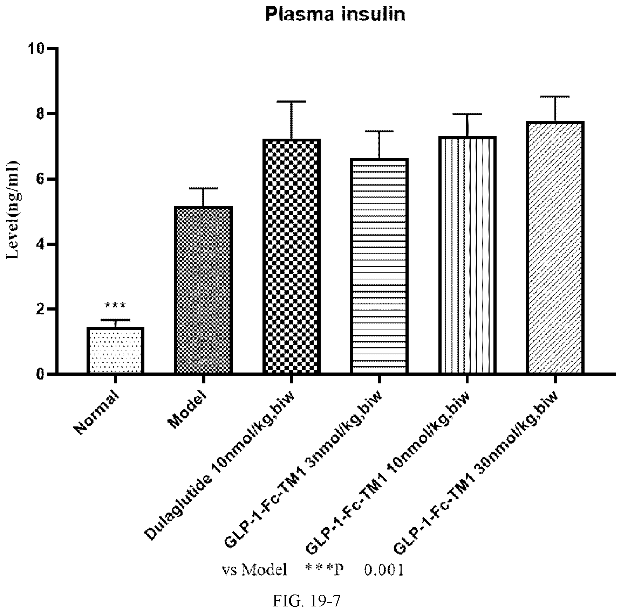

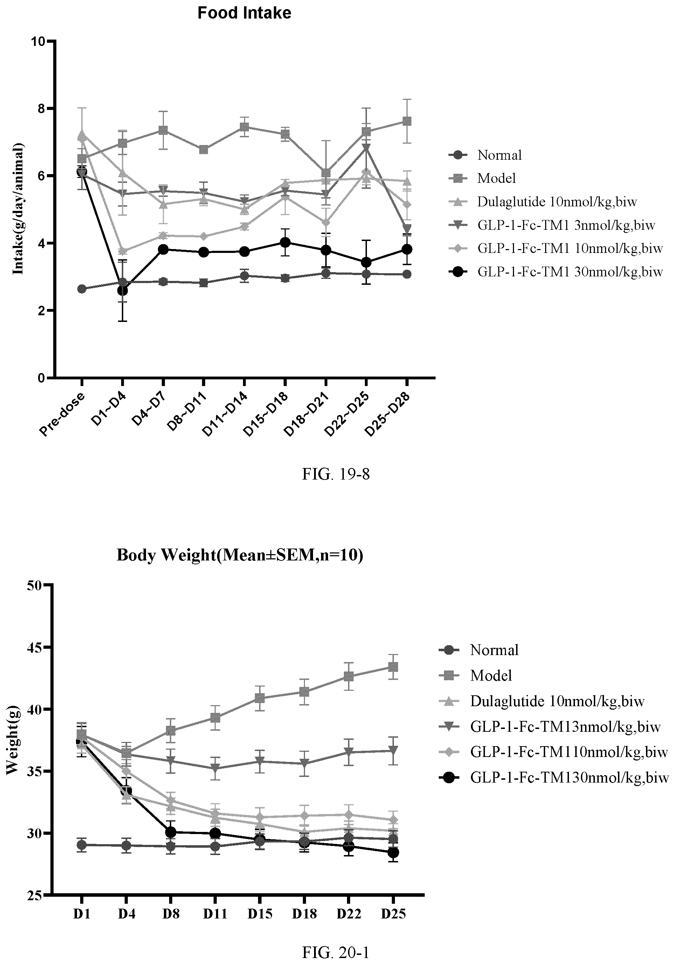

FIG. 19 shows the efficacy results of GLP-1-Fc-TM1 on type II diabetes db/db mice, wherein FIG. 19-1 shows the results of lowering 4h-fasting blood glucose in db/db mice; FIGS. 19-2 and 19-3 show the results of random blood glucose after the first and last dosing; FIGS. 19-4 and 19-5 show the results of blood glucose AUC₀₋₁₈₀ₘᵢₙ during the OGTT test after the last dosing; FIG. 19-6 shows the results of reducing glycated hemoglobin levels in db/db mice; FIG. 19-7 shows the results of increasing insulin levels in db/db mice; FIG. 19-8 shows the results of reducing average daily food intake in db/db mice.

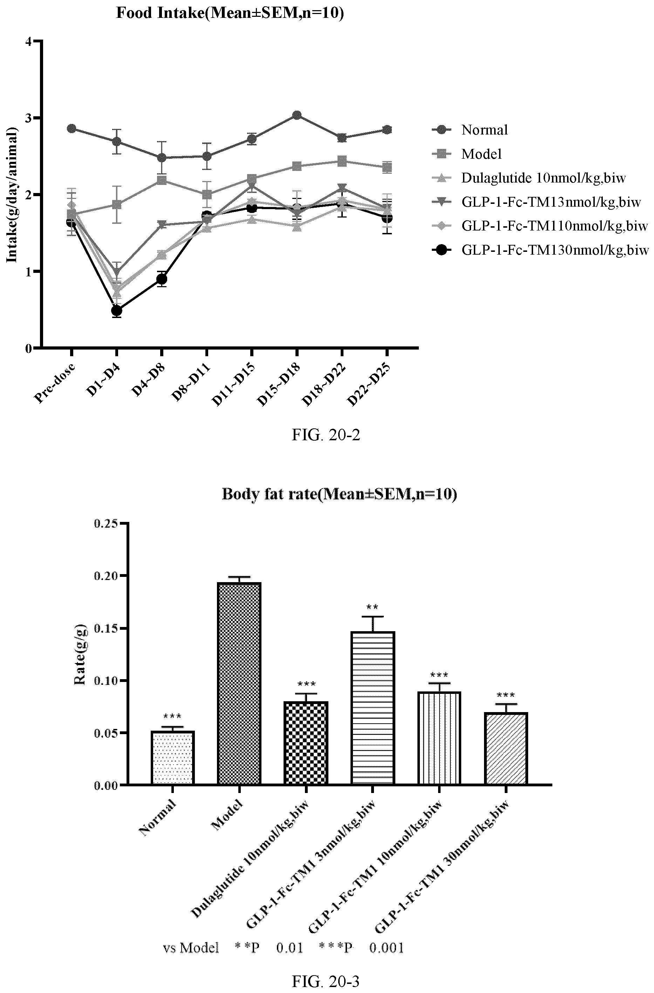

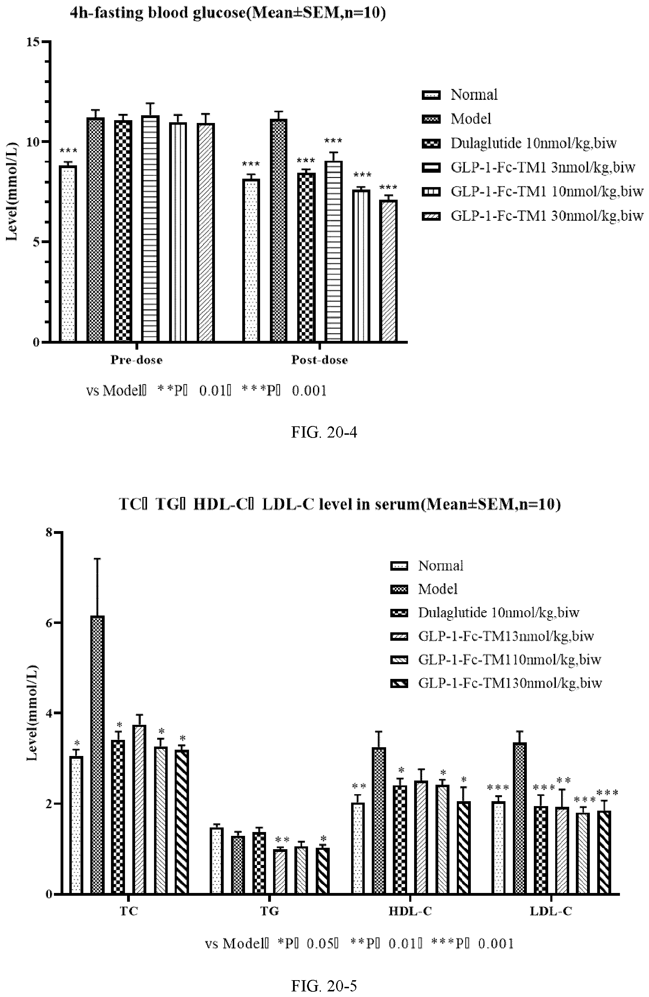

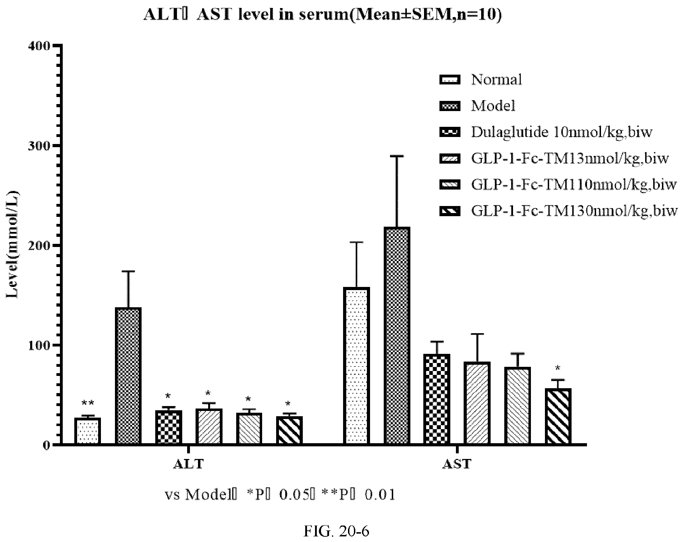

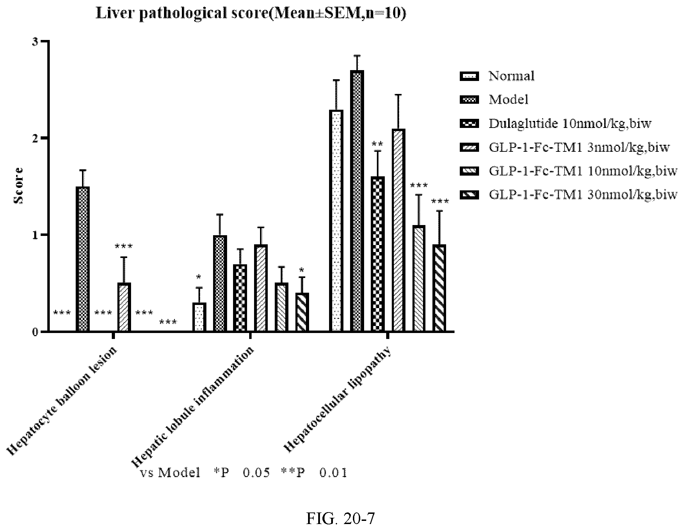

FIG. 20 shows the efficacy results of GLP-1-Fc-TM1 on DIO model mice, wherein FIG. 20-1 shows the results of reducing the body weight of DIO mice by GLP-1-Fc-TM1; FIG. 20-2 shows the results of reducing food intake in DIO mice by GLP-1-Fc-TM1; FIG. 20-3 shows the results of reducing body fat content in mice by GLP-1-Fc-TM1; FIG. 20-4 shows the results of reducing fasting blood glucose in mice by GLP-1-Fc-TM1; FIG. 20-5 shows the results of reducing the blood lipid levels in mice by GLP-1-Fc-TM1; FIG. 20-6 shows the results of liver function indices ALT and AST levels in serum of mice; and FIG. 20-7 shows the results of improving hepatocyte balloon lesion and hepatocellular lipopathy in DIO mice by GLP-1-Fc-TM1.
FIG. 21 shows the efficacy results of GLP-1-Fc-TM1 on rats, wherein FIGS. 21-1 to 21-4 show the results of lowering the blood glucose in SD rats by GLP-1-Fc-TM1, and FIGS. 21-5 to 21-8 show the results of elevating serum insulin by GLP-1-Fc-TM1.

### DETAILED DESCRIPTION OF THE INVENTION

### I. Definitions

Unless otherwise defined, all technical and scientific terms used herein have the same meaning as commonly understood by one of ordinary skill in the art. For the purposes of the present invention, the following terms are defined below.

When a trade name is used herein, the trade name includes the product formulation, generic drugs and active pharmaceutical ingredients of the trade name product, unless the context indicates otherwise.

The term "about" when used in conjunction with a numerical value is intended to encompass the numerical value within a range having a lower limit that is 5% less than the numerical value specified, and an upper limit that is 5% greater than the numerical value specified.

The term "and/or" should be understood as meaning any one of the alternatives or a combination of any two or more of the alternatives.

The terms "comprising" or "including" means containing the recited elements, integers, or steps, but not excluding any other elements, integers, or steps. When the term "comprising" or "including" is used herein, unless otherwise indicated, it also encompasses the situation consisting of the stated elements, integers, or steps. For example, reference to an antibody variable region "comprising" a particular sequence is also intended to encompass antibody variable regions consisting of that particular sequence.

The term "antibody" is used herein in the broadest sense and encompasses a variety of antibody constructs, including but not limited to, a monoclonal antibody, a polyclonal antibody, a recombinant antibody, a humanized antibody, a chimeric antibody, a multispecific antibody (e.g., a bispecific antibody), a single chain antibody, an intact antibody, or an antibody fragment thereof that exhibits the desired antigen-binding activity. A complete antibody will typically comprise at least two full length heavy chains and two full length light chains, but in some cases may comprise fewer chains, for example, antibodies naturally occurring in camels may comprise only heavy chains.

The term "whole antibody" refers to an immunoglobulin molecule comprising at least two heavy chains (H) and two light chains (L). Each heavy chain may consist of a heavy chain variable region (abbreviated as VH herein) and a heavy chain constant region. Each light chain may consist of a light chain variable region (abbreviated as VL herein) and a light chain constant region. The heavy chains of antibodies can be divided into 5 major distinct classes based on the amino acid sequence of their constant regions: IgA, IgD, IgE, IgG, and IgM, and several of these classes can be further divided into subclasses, e.g., IgG1, IgG2, IgG3 and IgG4, IgA1, and IgA2.

The terms "antibody fragment" and "antigen-binding fragment" of an antibody, which are used interchangeably, refer to a molecule that is not an intact antibody, comprising an portion of the intact antibody that is used to bind the antigen to which the intact antibody binds. As understood by those skilled in the art, for the purpose of antigen binding, antibody fragments typically comprise amino acid residues from a "complementarity determining regions" or "CDR". The antibody fragment may be prepared by recombinant DNA techniques, or by enzymatic or chemical cleavage of intact antibodies. Examples of antibody fragments include, but are not limited to, Fab, scFab, disulfide-linked scFab, Fab', F(ab')2, Fab' -SH, Fv, scFv, disulfide-linked scFv. In some embodiments, the antibody fragment comprises cysteine residues introduced into the Fc-region to provide amino acid residue sites used for thiol conjugation chemistry.

When an antibody is referred to herein as an IgG antibody, it refers to a heterotetrameric protein with an IgG class immunoglobulin structure. In IgG antibodies, the VH-CH1 of the heavy chain is typically paired with the VL-CL of the light chain to form a Fab fragment which specifically binds to the antigen. Therefore, an IgG antibody is essentially composed of two Fab molecules tethered by an immunoglobulin hinge region and two dimerized Fc-regions. IgG immunoglobulins can be divided to subclasses based on the sequence of the heavy chain constant region, e.g., γ1(IgG1), γ2(IgG2), γ3(IgG3), and γ4(IgG 4). In some embodiments, the antibody according to the present invention is an IgG antibody, for example an IgG1, IgG2, IgG3 or IgG4 antibody.

The term "complementary determining region"or "CDR region" or "CDR" or "hypervariable region" is a region of an antibody variable domain that is highly variable in sequence and forms a structurally defined loop ("hypervariable loop") and/or comprises an antigen-contacting residue ("antigen-contacting point"). The CDRs are primarily responsible for the binding to antigenic epitopes.

The term "variable region" or "variable domain" refers to the domain of an antibody heavy or light chain that is involved in the binding of the antibody to its antigen. The heavy chain variable region (VH) and light chain variable region (VL) can be further subdivided into hypervariable regions (HVRs, also known as Complementarity Determining Regions (CDRs)) with more conserved regions (i.e., Framework Regions (FRs)) interposed therebetween. Each VH and VL is composed of three CDRs and four FRs, arranged from amino-terminus to carboxy-terminus in the following order: FR1, CDR1, FR2, CDR2, FR3, CDR3, FR4.

The term "affinity" or "binding affinity" refers to the intrinsic binding affinity that reflects the interaction between members of a binding pair. Affinity can be measured by common methods known in the art. One method for measuring affinity is an ELISA assay, and the other method is a surface plasmon resonance technique (SPR) assay as described in the examples herein.

The term "Fc-region" refers to the C-terminal region of an immunoglobulin heavy chain, including a native sequence Fc-region and variant Fc-region, e.g., Fc-region sequences of various Ig subtypes and their allotypes (Gestur Vidarsson et al.,IgG subclasses and allotypes: from structure to effector functions, 20 October 2014, doi: 10.3389/fimmu.2014.00520.). In some embodiment, a Fc-region of human IgG heavy chain has amino acid sequences extending from Cys226 or from Pro230 to the carboxy-terminus of heavy chain. However, the C-terminal lysine (Lys447) of the Fc-region may or may not be present. In still other embodiments, the human IgG heavy chain Fc-region bears a hinge sequence or a partial hinge sequence of a native immunoglobulin at the N-terminus, e.g., the sequence from E216 to T225 or the sequence from D221 to T225 according to EU numbering. In certain embodiments, the Fc-region of an immunoglobulin comprises two constant domains, i.e., CH2 and CH3, and in other embodiments, the Fc-region of an immunoglobulin comprises three constant domains, i.e., CH2, CH3, and CH4.

The EU numbering scheme is a widely adopted standard for numbering the residues in an antibody in a consistent manner, developed based on sequence alignments. In the context of the present application, unless otherwise indicated, amino acid residues in the antibody or fusion protein region are numbered and referenced using the Kabat EU numbering scheme (as described by Kabat et al., Sequences of Proteins of Immunological Interest, 5 th edition, Public Health Service, National Institutes of Health, Bethesda, Md.(1991)).

The similar terms such as "Fc-region mutant," "mutant Fc-region," and "mutated Fc-region" are used interchangeably to refer to an Fc-region that comprises at least one amino acid modification that differs from a native sequence Fc-region/wild-type Fc-region. For example, amino acids at various positions in the Fc-region of a wild-type immunoglobulin IgG are modified to reduce the formation of hapten, to reduce or eliminate effector function, and to increase serum half-life. Further modifications to Fc-regions known in the art, Fc-regions provided by the present application, fusion proteins comprising Fc-regions (e.g., antibodies), and the like may be performed using a variety of methods that have been disclosed in the art, e.g., other modifications for reducing immunogenicity, increasing stability, solubility, function, and clinical benefit. Such modifications include, but are not limited to, modifications at the following positions, for example, of IgG Fc, e.g., modifications to amino acid residues at positions 214-238, 250-260, 297-299, 307-318, 322 or 327-331, 380-390, etc., in particular, for example, at positions 228, 233, 234, 235, 252, 254, 256, 297, 307, 308, 311, 380, 385, 386, 389, 428, 434, and 447. The hinge sequence of native human IgG can be modified so that the Fc-region is expressed as a homogeneous product. To improve the chemical stability of the Fc-region, asparagine, which is susceptible to deamidation, may be modified, for example, by replacement with glutamine, aspartic acid, or glutamic acid, for example, substitutions including N297E, N315Q, and N384Q. To improve the physical stability of the Fc-region, the leucine at position 235 of the Fc-region may be modified, for example, by a L235K substitution. In some embodiments, the immunoglobulin Fc-region may also be modified by phosphorylation, sulfation, glycosylation, methylation, acetylation, amidation, etc., to meet specific needs.

In one embodiment, the modification to the Fc-region is a modification at positions 254, 308 and 434, for example the modification disclosed in CN108299560A. In another embodiment, the modification to the Fc-region is a modification as described in CN1802167. In one particular embodiment, the modification to the Fc-region is a modification at positions 228, 234, 235 and/or 447, such as the modifications S228P, F234A, L235A or the modifications S228P, F234A, L235A and a deletion at position 447. In the present application, modifications to the Fc-region are shown in parentheses, for example, IgG4 Fc (S228P, F234A, L235A) indicates that amino acids at positions 228, 234, and 235 of the wild-type IgG4 Fc are substituted correspondingly.

The wild-type immunoglobulin Fc-region may be obtained from humans and animals (e.g., cows, goats, pigs, mice, rabbits, hamsters, rats, guinea pigs, and the like), or a recombinant form or a derivative of Fc-region may be obtained from transformed animal cells or microorganisms. For example, the Fc-region can be obtained by isolating a gene encoding an immunoglobulin from a corresponding cDNA library using a PCR method, or by subjecting an intact immunoglobulin to protease treatment. Techniques for making derivatives of the Fc-region are described, for example, in WO 97/34631 and WO 96/32478.

In some embodiments, the Fc-region used in the present application is an Fc-region derived from an IgG immunoglobulin, e.g., from the subclasses IgG1, IgG2, IgG3 and IgG4, preferably from the subclasses IgG1 and IgG4. In some embodiments, the Fc-region used in the present application is an Fc-region derived from human IgG1 and IgG4 to reduce the immunogenicity of the conjugates of the present application.

In some embodiments, the variant Fc-region comprises an amino acid sequence that differs from the amino acid sequence of the native sequence Fc-region by one or more amino acid substitutions, deletions, or additions. In some embodiments, the variant Fc-region has at least about 80%, 90%, 95%, 96%, 97%, 98%, 99% or more homology to a wild-type Fc-region and/or a parent Fc-region.

The term "neonatal Fc receptor (FcRn)" refers to an IgG antibody receptor located on the surface of a cell membrane. FcRn is responsible for the transfer of maternal IgG to the fetus and regulates the homeostasis of immunoglobulins in vivo. The FcRn can bind to the Fc moiety of the IgG, preventing the IgG molecule from being cleavaged by lysosomes, thereby increasing the half-life of the IgG in vivo, and involving in the metabolic processes of transportation, maintenance, and distribution of IgG in vivo.

The term "receptor-mediated endocytosis" refers to a process whereby a ligand/receptor complex is internalized and delivered into the cytosol or transferred to a suitable intracellular compartment, triggered by binding of the ligand to the corresponding receptor on the cell surface.

The term "sequence identity" refers to the extent to which sequences are identical on a nucleotide-by-nucleotide or amino acid-by-amino acid basis over a comparison window. The "percentage of sequence identity" can be calculated by comparing the two optimally aligned sequences over a comparison window, determining the number of positions at which the identical nucleic acid bases (e.g., A, T, C, G, I) or the identical amino acid residues (e.g., Ala, Pro, Ser, Thr, Gly, Val, Leu, Ile, Phe, Tyr, Trp, Lys, Arg, His, Asp, Glu, Asn, Gln, Cys, and Met) occurs in the two sequences to yield the number of matched positions, dividing the number of matched positions by the total number of positions in the comparison window (i.e., the window size), and multiplying the result by 100 to yield the percentage of sequence identity. Optimal alignment to determine the percentage of sequence identity can be accomplished in a variety of ways known in the art, for example, using publicly available computer software such as BLAST, BLAST-2, ALIGN, or Megalign (DNASTAR) software. Those skilled in the art can determine suitable parameters for the aligning sequences, including any algorithms required to achieve maximum alignment over the full length of the sequences being compared or over a region of the target sequence.

In the present invention, with respect to antibody sequences, the percentage of amino acid sequence identity is determined by optimally aligning the candidate antibody sequence with the given antibody sequence, and in a preferred embodiment, optimally aligning according to the Kabat numbering convention. Herein, without specifying the comparison window (i.e. the target antibody region to be compared), it will be applicable to alignment over the full length of a given antibody sequence. In some embodiments, with respect to antibodies, the sequence identity may be distributed over the heavy chain variable region and/or the light chain variable region, or the sequence percent identity may be limited to the framework regions only, while the sequences of the corresponding CDR regions remain 100% identical.

The term "amino acid substitution" refers to the replacement of at least one amino acid residue in a predetermined amino acid sequence with another different "substituted" amino acid residues.

The term "conservative substitution" refers to a substitution of one amino acid with another within the same class, e.g., a substitution of one acidic amino acid with another acidic amino acid, a substitution of one basic amino acid with another basic amino acid, or a substitution of one neutral amino acid with another neutral amino acid.

In the context of the present application referring to conjugates, the term "peptide linker" refers to a short amino acid sequence which links the active ingredient and the Fc-region, and consists of amino acids (such as glycine (G) and/or serine (S) and/or threonine residues (T) used alone or in combination), or is a hinge region from an immunoglobulin.

Peptide linkers that can be used in the present invention can be readily determined by those skilled in the art. For example, the peptide linkers comprising the amino acid sequence (G₄S)ₙ, wherein n is an integer equal to or greater than 1. In one preferred embodiment, the peptide linker comprises (G₄S)₃, (G₄S)₄, (G₄S)₆, GS(G₄S)₄, DAAALEAAALDAAAREAAARDAAAL, and NVDHLPSNTLVDLA. Peptide linkers that can be used in the antibody molecules of the present invention can also be, for example, but not limited to, the following amino acid sequences: (G₃S)2, (G₄S)₂, (G₃S)₃, (G₄S)₃, (G₃S)₄, (G₄S)₄, (G₃S)₅, (G₄S)₅, (G₃S)₆, (G₄S)₆, GGG, DGGGS, TGEKP, GGRR, EGKSSGSGSESKVD, KESGSVSSEQLAQFRSLD, GGRRGGGS, LRQRDGERP, LRQKDGGGSERP, and GSTSGSGKPGSGEGSTKG.

The term "alkyl" refers to a straight-chain or branched saturated hydrocarbon group consisting of carbon and hydrogen atoms. Specifically, an alkyl has 1 to 10 carbon atoms, such as 1 to 8, 1 to 6, 1 to 5, 1 to 4, 1 to 3, or 1 to 2 carbon atoms. For example, as used herein, the term "C₁-C₆ alkyl" refers to a straight or branched chain saturated hydrocarbon group having 1 to 6 carbon atoms, examples of which are, for example, methyl, ethyl, propyl (including n-propyl and isopropyl), butyl (including n-butyl, isobutyl, sec-butyl or tert-butyl), pentyl (including n-pentyl, isopentyl, neopentyl), hexyl (including n-hexyl, 2-methylpentyl, 3-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl), and the like.

The term "alkylene" refers to a divalent group derived by the removal of two hydrogen atoms from the same or two different carbon atoms from a straight or branched chain saturated alkane. Specifically, an alkylene has 1 to 10 carbon atoms, such as 1 to 6, 1 to 5, 1 to 4, 1 to 3, or 1 to 2 carbon atoms. For example, as used herein, the term "C₁-C₆ alkylene" refers to a straight or branched chain alkylene group having 1 to 6 carbon atoms, including, but not limited to, methylene, ethylidene, propylidene, butylidene, and the like.

The term "cycloalkyl" refers to a monocyclic, fused polycyclic, bridged polycyclic, or spiro non-aromatic monovalent hydrocarbon ring structure having the indicated number of ring atoms, which may be saturated or unsaturated, e.g., containing 1 or more double bonds. The cycloalkyl group may contain 3 or more carbon atoms, for example, 3 to 18, 3 to 10, or 3 to 8 carbon atoms in the ring, e.g., C₃₋₁₀ cycloalkyl, C₃₋₈ cycloalkyl, C₃₋₆ cycloalkyl, C₅₋₆ cycloalkyl. Non-limiting examples of cycloalkyl group include, but are not limited to, cyclopropyl, cyclobutyl, cyclopentyl, cyclohexyl, cycloheptyl, and the like.

The term "alkenyl" refers to a straight or branched chain unsaturated hydrocarbon group consisting of carbon atoms and hydrogen atoms containing at least one double bond. **In** particular, an alkenyl has 2 to 8 carbon atoms, such as 2 to 6, 2 to 5, 2 to 4, or 2 to 3 carbon atoms. For example, as used herein, the term "C₂-C₆ alkenyl" refers to a straight or branched chain alkenyl group having 2 to 6 carbon atoms, such as ethenyl, propenyl, allyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, and the like.

The term "alkenylene" refers to a divalent group derived by the removal of two hydrogen atoms from the same or two different carbon atoms from a straight or branched chain unsaturated alkene containing at least one double bond. **In** particular, the alkenylene group has 2-8 carbon atoms, for example 2 to 6, 2 to 5, 2 to 4 or 2 to 3 carbon atoms. For example, as used herein, the term "C₂-C₆ alkenylene" refers to a straight or branched chain alkenylene group having 2 to 6 carbon atoms, such as ethenylene, propenylene, allylidene, butenylene, pentenylene, and hexenylene.

The term "alkynyl" refers to a straight or branched unsaturated hydrocarbon group consisting of carbon and hydrogen atoms containing at least one triple bond. In particular, an alkynyl has 2-8, e.g., 2 to 6, 2 to 5, 2 to 4, or 2 to 3 carbon atoms. For example, as used herein, the term "C₂-C₆ alkynyl" refers to a straight or branched chain alkynyl group having 2 to 6 carbon atoms, such as ethynyl, propynyl, propargyl, 1-butynyl, 2-butynyl, 1-pentynyl, 2-pentynyl, 3-pentynyl, 4-methyl-1-pentynyl, 1-hexynyl, 2-hexynyl, 3-hexynyl, 5-methyl-2-hexynyl, and the like.

The term "alkynylene" refers to a divalent group derived by the removal of two hydrogen atoms from the same or two different carbon atoms from a straight or branched chain unsaturated alkyne containing at least one triple bond. In particular, an alkynylene has 2 to 8 carbon atoms, e.g., 2 to 6, 2 to 5, 2 to 4, or 2 to 3 carbon atoms. For example, as used herein, the term "C₂-C₆ alkynylene" refers to a straight or branched chain alkynylene group having 2 to 6 carbon atoms, such as ethynylene, propynylene, propargylene, butynylene, pentynylene, and hexynylene.

The term "heterocycle" or "heterocyclyl" refers to a 5-20 membered (e.g., 5-14 membered, 5-8 membered, 5-6 membered) aromatic or non-aromatic monocyclic, bicyclic, or polycyclic ring system having 1-4 heteroatom ring members independently selected from N, O, or S. One or more of the N, C, or S atoms in the heterocycle may be oxidized. Preferably, an heterocycle is a 5-10 membered ring system, either a monocyclic or fused bicyclic ring. Representative examples include, but are not limited to, pyrrolidine, azetidine, piperidine, morpholine, tetrahydrofuran, tetrahydropyran, benzofuran, benzothiophene, indole, benzopyrazole, pyrrole, thiophene (thiophene), furan, thiazole, imidazole, pyrazole, pyrimidine, pyridine, pyrazine, pyridazine, isothiazole, and isoxazole. It should be understood that this term encompasses heteroaryl as defined herein.

The term "aryl" refers to a monocyclic or polycyclic aromatic hydrocarbon group having 6 to 20, for example, 6 to 12 carbon atoms in the ring portion. Preferably, an aryl is a C₆-C₁₀ aryl group. Non-limiting examples include phenyl, biphenyl, naphthyl, or tetrahydronaphthyl, each of which may be optionally substituted with 1-4 substituents such as alkyl, trifluoromethyl, cycloalkyl, halogen, hydroxyl, alkoxy, acyl, alkyl-C(O)-O-, aryl-O-, heteroaryl-O-, amino, thiol, alkyl-S-, aryl-S-, nitro, cyano, carboxy, alkyl-O-C(O)-, carbamoyl, alkyl-S(O)-, sulfonyl, sulfonamido, heterocyclyl, and the like.

The term "heteroaryl" refers to a 5-20 membered (e.g., 5-14 membered, 5-8 membered, 5-6 membered) aromatic monocyclic or polycyclic ring system containing 1-4 heteroatoms selected from N, O, or S, which may be substituted or unsubstituted. Preferably, an heteroaryl is a 5-10 membered ring system, either a monocyclic or fused bicyclic ring. Representative heteroaryl groups include 2- or 3-thienyl, 2- or 3-furyl, 2- or 3-pyrrolyl, 2-, 4- or 5-imidazolyl, 3-, 4- or 5-pyrazolyl, 2-, 4- or 5-thiazolyl, 3-, 4- or 5-isothiazolyl, 2-, 4- or 5-oxazolyl, 3-, 4- or 5-isoxazolyl, 3- or 5-1,2, 4-triazolyl, 4- or 5-1,2, 3-triazolyl, tetrazolyl, 2-, 3- or 4-pyridyl, 3- or 4-pyridazinyl, 3-, 4- or 5-pyrazinyl, 2-pyrazinyl, 2-, 4- or 5-pyrimidinyl.

The term "heteroalkyl" refers to a stable straight or branched chain hydrocarbon which is fully saturated or contains from 1 to 3 degrees of unsaturation and consists of the indicated number of carbon atoms and from one to ten, preferably from one to three, heteroatoms selected from O, N, Si and S, wherein the nitrogen and sulfur atoms may optionally be oxidized and the nitrogen heteroatom may optionally be quaternized. The heteroatoms O, N, Si, and S may be located at any internal position of the heteroalkyl group or at the position where the heteroalkyl group is attached to the remainder of the molecule. Representative examples of heteroalkyl groups include -CH2-CH2-O-CH3, -CH2-CH2-NH-CH3, -CH2-CH2-N(CH3)-CH3, -CH2-S-CH2-CH3, -CH2-CH2-S(O)-CH3, -NH-CH2-CH2-NH-C(O)-CH2-CH3, -CH2-CH2-S(O)2-CH3, -CH = CH-O-CH3, -Si(CH3)3, -CH2-CH = N-O-CH3, and -CH = CH-N(CH3)-CH3. Up to two heteroatoms may be consecutive, for example, -CH2-NH-OCH3 and -CH2-O-Si(CH3)3. Typically, the C1 to C4 heteroalkyl or heteroalkylene group has 1 to 4 carbon atoms and 1 or 2 heteroatoms, and the C1 to C3 heteroalkyl or heteroalkylene group has 1 to 3 carbon atoms and 1 or 2 heteroatoms. In some aspects, the heteroalkyl and heteroalkylene groups are saturated.

Unless otherwise indicated, the term "being substituted" as used herein in defining the respective groups means that the respective group may be substituted by, for example, but not limited to the following groups: alkyl, alkenyl, alkynyl, cycloalkyl, aryl, heteroaryl, heterocyclyl, halogen, cyano, nitro, azido, carboxyl, hydroxyl, thiol, amino, mono- or dialkylamino, mono- or dicycloalkylamino, mono- or diarylamino, mono- or diheteroylamino, mono -or diheteroarylamino, alkyl -or cycloalkyl- or heterocyclyl- or heteroaryl- or aryl-oxy, alkyl- or cycloalkyl- or heterocyclyl- or heteroaryl- or aryl-thio, alkyl- or cycloalkyl- or heterocyclyl- or heteroaryl- or aryl-acyl, alkyl- or cycloalkyl- or heterocyclyl- or heteroaryl- or aryl-acylamino, alkyl- or cycloalkyl- or heterocyclyl- or heteroaryl- or aryl-acyloxy, alkyl- or cycloalkyl- or heterocyclyl- or heteroaryl- or aryl-sulfonyl, alkyl- or cycloalkyl- or heterocyclyl- or heteroaryl- or aryl-sulfonyloxy, alkyl- or cycloalkyl- or heterocyclyl- or heteroaryl- or aryl-sulfonylamino, or the above optionally substituted amino-formyl, and groups each of which is further substituted by other optional substituents, wherein each group is as defined herein. Examples of substituents include, but are not limited to, one or more groups independently selected from: halogen, OH, SH, CN, NH₂, NHCH₃, N(CH₃)₂, NO₂, N₃, C(O)CH₃, COOH, C(O)-amino, OCOCH₃, methyl, ethyl, propyl, isopropyl, butyl, isobutyl, sec-butyl, tert-butyl, cyclopropyl, methoxy, ethoxy, propoxy, oxo, trifluoromethyl, difluoromethyl, sulfonylamino, methylsulfonylamino, SO, SO₂, phenyl, piperidinyl, piperazinyl, and pyrimidinyl.

The term "PEG unit" refers to an organic moiety comprising repeating ethyleneoxy subunits (PEG or PEG subunits), which may be polydisperse, monodisperse, or discrete (i.e., having a discrete number of ethyleneoxy subunits). A polydisperse PEG is a mixture of PEG with heterogeneous size and molecular weight, whereas a monodisperse PEG is typically purified from a heterogeneous mixture and thus has a single chain length and molecular weight. Preferred PEG units comprise discrete PEGs, which are compounds synthesized in a stepwise manner rather than via a polymerization process. The discrete PEGs provide a single molecule with a defined and specified chain length.

"Pharmaceutically acceptable" and "for pharmaceutical use" are used interchangeably hereinunless context dictates otherwise.

The term "DAR" in the present application refers to a ratio of Cn moiety conjugated to an Fc as described herein to the Fc in a conjugate, or a ratio of Cn moiety conjugated to an antibody as described herein to the antibody in a conjugate, or a ratio of Cn moiety conjugated to a fusion protein as described herein to the fusion protein in a conjugate. In some embodiments as described herein, the DAR may be from 1 to 20, such as 1-18, 4-16, 5-12, 6-10, 1-8, 2-8, 1-6, 2-6, 2-4, such as 1, 2, 3, 4, 5, 6, 7, 8, 9, 10, 11, 12, 13, 14, or 15. DAR may also be calculated as an average DAR of a population of molecules in a product, i.e., an overall ratio of Cn conjugated to an Fc moiety as described herein to the Fc moiety in the product, an overall ratio of Cn moiety conjugated to an antibody as described herein to the antibody in the product, or an overall ratio of Cn moiety conjugated to a fusion protein as described herein to the fusion protein in the product, as measured by detection methods (e.g., by conventional methods such as mass spectrometry, an ELISA assay, electrophoresis, and/or HPLC), such DAR being referred to herein as an average DAR. In some embodiments, the average DAR value of a conjugate in the present invention is from 1 to 20, for example, 2-18, 4-16, 5-12, 6-10, 2-8, 3-8, 2-6, 4-6, 6-10, e.g., 1.0-8.0, 2.0-6.0, such as 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8.0, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0, and the ranges with two of these values as endpoints.

The term "drug" as used herein encompasses any substance effective in preventing or treating diseases associated with glucose metabolism disorders, cardiovascular and cerebrovascular diseases, kidney diseases, retinopathy. The diseases associated with glucose metabolism disorders include, for example, diabetes (e.g., type I diabetes, type II diabetes), obesity, hypertension, dyslipidemia, obesity, glucose intolerance, hyperglycemia, hyperinsulinemia, cardiovascular diseases, and the like.

The term "active ingredient" as used herein refers to a substance, usually an active peptide substance, having any physiologically active function that is beneficial to cells and/or organisms (e.g., regulating gene expression and physiological function, correcting abnormal conditions caused by deficient or excess secretion of ingredients involved in regulating in vivo functions). For example, the active ingredient includes, but not limited to, an enzyme, an enzyme inhibitor, an antigen, an antibody, an antibody fragment, a hormone, glucagon-like peptide-1 (GLP-1), glucagon, an interferon, a cytokine, a growth factor and/or a differentiation factor, a factor involved in cell motility or migration, a factor involved in bone formation/resorption, a chemokine, a plasma or interstitial adhesion molecule or extracellular matrix, a bactericidal or antifungal factor, and the like.

The term "pharmaceutical composition" refers to a composition that is present in a form that allows the biological activity of the active ingredients contained therein to be effective, and which does not contain additional ingredients having unacceptable toxicity to the subject to which the composition is administered. In some embodiments, the pharmaceutical composition of the present invention comprises a conjugated molecule of the present invention and a pharmaceutical excipient.

The term "pharmaceutical excipient" refers to a diluent, an adjuvant (e.g., a Freund's adjuvant (complete and incomplete)), an excipient, a buffer, a surfactant, a carrier or a stabilizer, etc., with which the active substance is administered.

The term "pharmaceutical combination" refers to a non-fixed combination product or a fixed combination product, including but not limited to a kit, a pharmaceutical composition. The term "non-fixed combination" means that the active ingredients are administered to a patient as separate entities either simultaneously, without specific time limitations, or sequentially at the same or different time intervals, wherein such administration provides prophylactically or therapeutically effective levels of the two or more active agents in the patient. In some embodiments, the molecule of the present invention and the other therapeutic agents used in the pharmaceutical combination are administered at a level no greater than that when they are used alone. The term "fixed combination" means that two or more active agents are administered to a patient simultaneously in the form of a single entity. The dosage and/or time intervals of the two or more active agents are preferably selected such that the combined use of the components results in a greater effect in the treatment of the disease or disorder than that would be achieved by use of either component alone. The ingredients may each be in separate formulations, which may be the same or different.

The term "combination therapy" refers to the administration of two or more therapeutic agents or treatment modalities (e.g., radiation therapy or surgery) to treat the diseases described herein. Such administration includes co-administering the therapeutic agents in a substantially simultaneous manner, for example, in a single capsule having a fixed ratio of active ingredients. Alternatively, such administration includes co-administration of the active ingredients in multiple or separate containers (e.g., tablets, capsules, powders, and liquids). The powder and/or liquid may be reconstituted or diluted to the desired dosage prior to administration. **In** addition, such administration also includes the use of each type of therapeutic agent at approximately the same time or at different times in a sequential manner. **In** either case, the treatment regimen will provide a beneficial effect of the drug combination in treating the disorders or conditions described herein.

The terms "individual" or "subject" are used interchangeably herein to refer to mammals. Mammals include, but are not limited to, domesticated animals (e.g., cows, sheep, cats, dogs, and horses), primates (e.g., humans and non-human primates such as monkeys), rabbits, and rodents (e.g., mice and rats). **In** particular, the subject is a human.

As used herein, "treating" refers to slowing, interrupting, arresting, relieving, stopping, reducing, or reversing the progression or severity of an existing symptom, disorder, condition, or disease.

As used herein, "preventing" includes the inhibition of the occurrence or development of a disease or disorder or the symptoms of a particular disease or disorder. In some embodiments, a subject with a family history of diseases associated with glucose metabolism disorders (e.g., diabetes) is a candidate for a prophylactic regimen. In general, in the context of diseases associated with glucose metabolism disorders (e.g., diabetes), the term "prevention" refers to the administration of a drug prior to the onset of a sign or symptom of diseases associated with glucose metabolism disorders (e.g., diabetes), particularly in a subject at risk for diseases associated with glucose metabolism disorders (e.g., diabetes).

The term "effective amount" used herein refers to an amount or dose of a conjugate of the present invention or a composition or a combination thereof which, upon administration to a patient in a single or multiple dose, produces the desired effect in the patient in need of treatment or prevention.

The term "therapeutically effective amount" used herein refers to an amount effective, at dosages and for periods of time required, to achieve the desired therapeutic result. A therapeutically effective amount is also one in which any toxic or adverse effect of the conjugate of the present invention or a composition or a combination thereof is less than a therapeutically beneficial effect. A "therapeutically effective amount" preferably inhibits or reduces a measurable parameter (e.g., glucagon secretion, blood glucose concentration) by at least about 30%, even more preferably at least about 40%, 45%, 50%, 55%, 60%, 65%, 70%, 75%, 80%, 85%, 90% or even 100% relative to untreated individuals.

A "prophylactically effective amount" used herein refers to an amount effective, at dosages and for periods of time required, to achieve the desired prophylactic result. Generally, a prophylactically effective amount will be less than a therapeutically effective amount because a prophylactic dose is used in an subject prior to or at an earlier stage of the disease.

### II. Pharmaceutical composition and kit

A pharmaceutically acceptable salt form of a conjugated molecule of the present invention falls within the scope of the present invention. In some embodiments, the present invention provides a composition, preferably a pharmaceutical composition or a pharmaceutical formulation, comprising any of the conjugated molecules described herein and pharmaceutically acceptable salts thereof.

In one embodiment, the pharmaceutical composition further comprises a pharmaceutical excipient. In one embodiment, a composition, e.g., a pharmaceutical composition, comprises a conjugated molecule of the present invention in combination with one or more other therapeutic agents.

The composition disclosed in the present invention also contains suitable pharmaceutical excipients such as a pharmaceutical carrier, a excipient, including a buffer, as known in the art.

As used herein, "pharmaceutically acceptable carrier" includes any and all solvents, dispersion media, isotonic agent and absorption delaying agents, and the like that are physiologically compatible.

See also "Handbook of Pharmaceutical Excipients", 8th Edition, R.C.Rowe,P.J.Seskey and S.C. Owen, Pharmaceutical Press, London, Chicago for the use of pharmaceutical excipients and uses thereof.

The compositions of the present invention can be in a variety of forms. Such forms include, for example, liquid, semi-solid, and solid dosage forms, such as liquid solutions (e.g., injectable and infusible solutions), powders or suspensions, liposomal formulations, and suppositories. The preferred form depends on the intended mode of administration and therapeutic use.

Medicaments comprising a conjugated molecule of the present invention as described herein can be prepared by mixing the conjugated molecules of the present invention having the desired purity with one or more optional pharmaceutical excipients, preferably in the form of lyophilized formulations or aqueous solutions.

The pharmaceutical compositions or formulations of the present invention may also contain more than one active ingredient as required for the particular indication being treated, preferably those having complementary activities that do not adversely affect each other. For example, it is desirable to also provide other therapeutic agents, including chemotherapeutic agents, angiogenesis inhibitors, cytokines, cytotoxic agents, other antibodies, small molecule drugs, or immunomodulators (e.g., immune checkpoint inhibitors or agonists), etc. The active ingredients are suitably present in combination in an amount effective for the intended use.

Sustained release formulations may be prepared. Suitable examples of sustained release formulations include semipermeable matrices of solid hydrophobic polymers containing the conjugated molecule, which matrices are in the form of shaped articles, e.g., films, or microcapsules.

In some embodiments, the present invention also provides a pharmaceutical combination or a pharmaceutical combination product comprising a conjugated molecule of the present invention, and one or more other therapeutic agents.

A further object of the present invention is to provide a pharmaceutical kit comprising the pharmaceutical combination of the present invention, preferably the pharmaceutical kit is in a form of pharmaceutical dosage unit. Dosage units may thus be provided according to a dosing regimen or interval of drug administration.

In one embodiment, a pharmaceutical kit according to the present invention comprises in the same package:
- a first container containing a conjugated molecule of the present invention;
- a second container containing a pharmaceutical composition comprising a further therapeutic agent.

### III. Use and therapeutic method

The long-acting platform, namely the active molecule-fusion protein-Cn conjugate platform, the active molecule-Fc-Cn conjugate platform and the antibody-Cn conjugate platform, provided by the present invention, can be used for effectively extendeding the serum half-life of the active molecules. Specifically, the active molecule is constructed into a conjugated molecule having a structure of "active molecule-Fc-Cn", a conjugated molecule having a structure of "active molecule-fusion protein-Cn", and a conjugated molecule having a structure of "antibody-Cn" by the method disclosed by the present application, so that the serum half-life of the active molecules is effectively improved, thereby reducing the dosing frequency, reducing the dosage, and saving the cost.

According to the present invention, the long-acting platform will extend the serum half-life of the active molecules without affecting the biological function of the active molecules, and the resulting conjugated molecule having a structure of "active molecule-Fc-Cn", the conjugated molecule having a structure of "active molecule-fusion protein-Cn" and the conjugated molecule having a structure of "antibody-Cn" have low immunogenicity to an organism, thereby effectively avoiding the negative effect that the common conjugated molecules easily cause the immune response of the organism.

The conjugated molecule of "active molecule-Fc-Cn", the conjugated molecule of "active molecule-fusion protein-Cn" and the conjugated molecule having a structure of "antibody-Cn" obtained by the present application can be used for preventing or treating various diseases in a subject. One skilled in the art would be readily determine the diseases that can be treated or prevented by the conjugated molecule based on the biological function of the active molecule. For example, when the active molecule is GLP-1, the conjugated molecule may be used for the effective treatment of metabolic diseases and/or disorders, e.g., diabetes, such as type I diabetes, type II diabetes, impaired glucose tolerance, hyperglycemia, dyslipidemia, obesity, metabolic syndrome, cardiovascular diseases, and the like.

When the active molecule is an anti-PD-1 antibody or antigen-binding fragment thereof, the conjugated molecule can be used to effectively prevent or treat diseases associated with aberrant PD-1 expression, such as various tumors or cancers, e.g., melanoma, non-small cell lung cancer, renal cell carcinoma, bladder cancer , Hodgkin's lymphoma, head and neck cancer, ovarian cancer, and brain cancer.

When the active molecule is an anti-VEGF antibody or antigen-binding fragment thereof, the conjugated molecule may be used to effectively prevent or treat diseases associated with aberrant VEGF expression, for example, various angiogenesis-related diseases, e.g., ophthalmic diseases, such as wet or neovascular age-related macular degeneration (AMD) and diabetic macular edema (DME); most cancers; cardiovascular diseases.

The present invention provides a method for preventing or treating a disease in a subject, comprising administering to the subject an effective amount of a conjugated molecule of the present invention or a pharmaceutically acceptable salt thereof, a pharmaceutical composition, a pharmaceutical combination or a kit.

**In** the therapeutic methods of the present invention, administration of the conjugated molecules of the present invention may include 1) therapeutic measures that cure, slow, alleviate symptoms of, and/or halt progression of a diagnosed pathological condition or disorder; or 2) prophylactic or preventative measures that prevent and/or slow the development of a pathological condition or disorder. **In** some embodiments, in the therapeutic methods of the present invention, the subject will benefit from the therapeutic or prophylactic measure and exhibit a reduction or improvement in the occurrence, recurrence or development of a disease, disorder, condition, and/or symptom as compared to a subject not receiving the treatment.

The pharmaceutical compositions of the present invention may be administrated by any suitable method, including parenteral, intratumoral and intranasal dosing. Parenteral infusion includes intramuscular, intravenous, intraarterial, intraperitoneal or subcutaneous dosing. Administration may be by any suitable route, e.g., by injection, e.g., intravenous or subcutaneous, depending in part on whether administration is short-term or long-term. Various administration schedules are encompassed herein, including, but not limited to, single dosing or multiple dosing at multiple time points, bolus dosing, and pulse infusion.

For the prevention or treatment of diseases, the appropriate dosage of the pharmaceutical composition of the present invention (when used alone or in combination with one or more other therapeutic agents) will depend on the type of disease to be treated, the specific type of the active molecules of the conjugated molecules, the severity and course of the disease, therapeutic purposes, previous therapy, the patient's clinical history and response to the drugs, and the discretion of the attending physician.

**In** some embodiments, the present invention also provides the use of a pharmaceutical composition of the present invention in the manufacture of a medicament for use in the aforementioned methods of treatment and prevention.

These and other aspects and embodiments of the present invention are exemplified in the following examples. Any or all of the features described above and throughout this application may be combined in various embodiments of the present invention. The following examples further illustrate the present invention, however, it should be understood that the examples are for purposes of illustration and are not to be construed as limiting in any way.

The abbreviations used in the specification and claims have the following meanings:
- AUC: Area under the curve
- CV: Column volume
- HSA: Human serum albumin
- PBS: Phosphate buffered saline
- tBu: Tert-butyl
- Pbf: 2,2,4,6,7-Pentamethylbenzofuran-5-sulfonyl
- Trt: Trityl
- Mmt: 4-Methoxytriphenyl
- Mtt: Methyl trityl
- Alloc: (2-Propyleneoxy)carbonyl
- DCM: Dichloromethane
- DCC: Dicyclohexylcarbodiimide
- DMF: N,N-dimethylformamide
- DMAP: 4-Dimethylaminopyridine
- DIPEA: N,N-Diisopropylethyl amine
- DIC: N,N-Diisopropylcarbodiimide
- HBTU: Benzotriazole-N,N,N',N'-tetramethylurea hexafluorphosphate
- HATU: 2-(7-Azabenzotriazol-1-yl)-N,N,N',N'-tetramethyluronium hexafluorophosphate
- HPLC: High performance liquid chromatography
- TBTU: O-Benzotriazole-N,N,N',N'-tetramethylurea tetrafluoroborate
- HOBT: 1-Hydroxybenzotriazole
- HOAT: 1-Hydroxy-7-azobenzotriazole
- TFA: Trifluoroacetic acid
- TIS: Tri-isopropyl silane
- TCEP: Tris(2-carboxyethyl) phosphine hydrochloride
- TSTU: O-(N-succinimide)-1,1,3,3-tetramethyluronium tetrafluoroborate

### Example

### Example 1. Preparation of advanced fatty acid chain

In this example, examples of a variety of different advanced fatty acid chains that can react with different sites on the Fc-region (or fusion protein), e.g., free thio groups, and amino groups, were constructed.

### 1.1 Preparation of advanced fatty acid chain TM1

Fatty acid chain TM1 was prepared according to the following technical route: 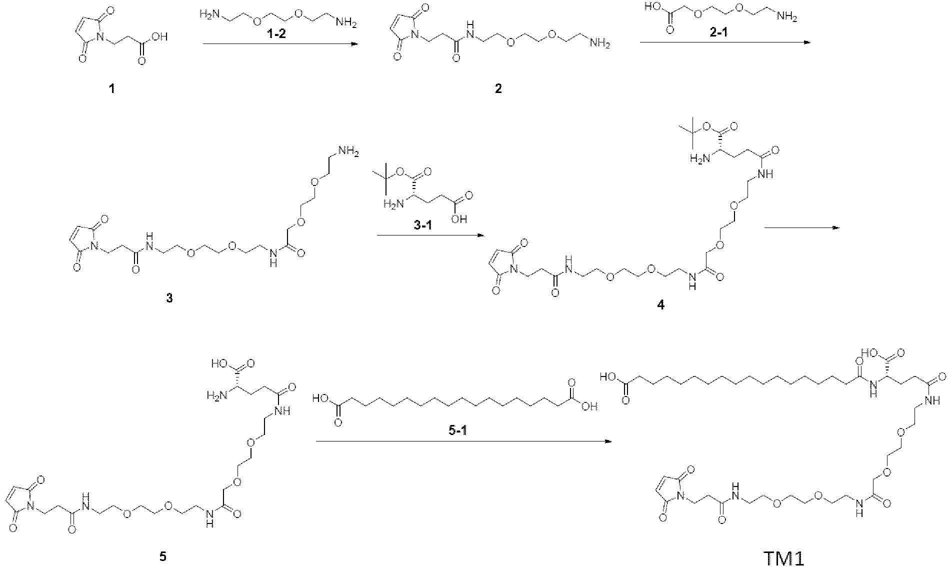
**(1)** Compound 1 (5.0 g, 29.40 mmol) was dissolved in anhydrous DCM (50 mL) under stirring well, then EDCI (5.62 g, 29.4 mmol) and Compound 1-2 (4.35 g, 29.40 mmol) were added and allowed to react at 25°C for 2 h. After the reaction was completed, the reaction mixture was isolated directly by preparative HPLC, and concentrated to give 2 as a colorless oil (5.5 g, 62.5%).
**(2)** Compound 2 (5.5 g, 18.30 mmol) was dissolved in anhydrous DCM (60 mL) under stirring well, then EDCI (3.50 g, 18.30 mmol) and Compound 2-1 (2.98 g, 18.30 mmol) were added and allowed to react at 25°C for 2 h. After the reaction was completed, the reaction mixture was isolated directly by preparative HPLC, and concentrated to give 3 as a colorless oil (4.8 g, 59.3%).
**(3)** Compound 3 (4.8 g, 10.78 mmol) was dissolved in anhydrous DCM (40 mL) under stirring well, then EDCI (2.06 g, 10.78 mmol) and Compound 3-1 (2.18 g, 10.78 mmol) were added and allowed to react at 25°C for 12 h. After the reaction was completed, the reaction mixture was isolated directly by preparative HPLC, and concentrated to give 4 as a colorless oil (4.0 g, 58.9%).
**(4)** Compound 4 (4.0 g, 6.34 mmol) was dissolved in anhydrous DCM (30 mL) under stirring well, then TFA (5 mL) was added and allowed to react at 25°C for 0.5 h. After the reaction was completed, the reaction mixture was concentrated directly to give 5 as a brownish-black oil (3.2 g, 88%).
**(5)** Compound 5 (3.2 g, 5.58 mmol) was dissolved in anhydrous DMF (30 mL) under stirring well, then DCC (1.14 g, 5.58 mmol) and DMAP (0.14 g, 1.16 mmol) and Compound 5-1 (1.75 g, 5.58 mmol) were added and allowed to react at 25°C for 2 h. After the reaction was completed, the reaction mixture was isolated directly by preparative HPLC, and concentrated to give TM1 as a colorless oil (2.1g, 43.7%), with a molecular weight of 870.05.

Structure elucidation of TM1:
Mass spectrometry:
   MS-ESI (m/z): 870.4 [M+H]+
Nuclear magnetic hydrogen spectrum:
   ¹H NMR (400MHz, DMSO-d₆) δ 8.02 (S, 2H, NH) 7.88 (S, 1H, COOH) 7.65 (S, 1H, COOH) 6.97 (S, 2H, CH) 4.13 (S, 1H, CH) 3.67~3.69 (S, 2H, CH₂) 3.65~3.15 (m, 22H, CH₂) 2.33~1.17 (m, 10H, CH₂) 1.16 (S, 4H, CH₂) 1.21 (S, 26H, CH₂)
Nuclear magnetic carbon spectrum:
   ¹³C NMR (100MHz, DMSO-d₆) δ 174.91, 173.91, 172.88, 171.95, 171.13, 169.77, 134.94, 70.63, 70.38, 69.33, 51.94, 40.45, 40.25, 39.83, 39.20, 35.53, 34.20, 32.15, 29.57, 27.49, 25.70, 24.95

### 1.2 Preparation of advanced fatty acid chain C18 ester

The fatty acid chain C18 ester (i.e., C18-tert-butanol ester) was prepared according to the following technical route: 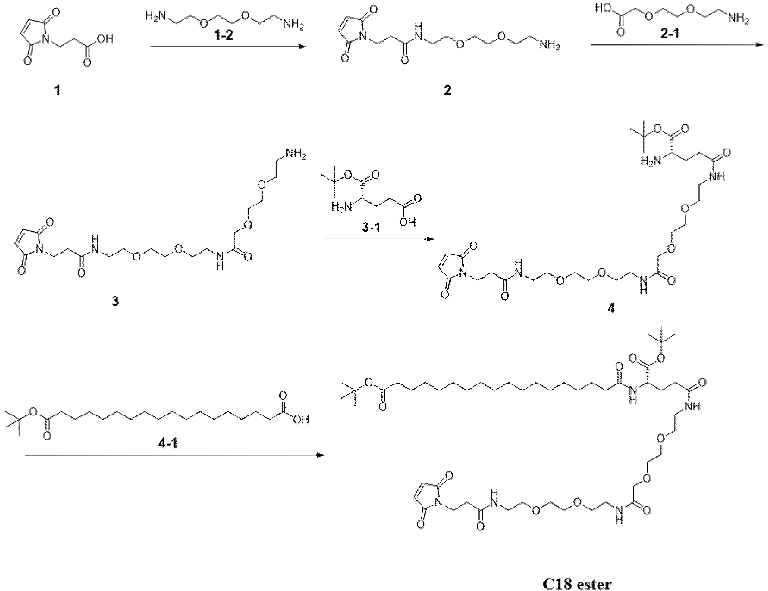
**(1)** Compound 1 (5.0 g, 29.40 mmol) was dissolved in anhydrous DCM (50 mL) under stirring well, then EDCI (5.62 g, 29.4 mmol) and Compound 1-2 (4.35 g, 29.40 mmol) were added and allowed to react at 25°C for 2 h. After the reaction was completed, the reaction mixture was isolated directly by preparative HPLC, and concentrated to give 2 as a colorless oil (5.5 g, 62.5%).
**(2)** Compound 2 (5.5 g, 18.30 mmol) was dissolved in anhydrous DCM (60 mL) under stirring well, then EDCI (3.50 g, 18.30 mmol) and Compound 2-1 (2.98 g, 18.30 mmol) were added and allowed to react at 25°C for 2 h. After the reaction was completed, the reaction mixture was isolated directly by preparative HPLC, and concentrated to give 3 as a colorless oil (4.8 g, 59.3%).
**(3)** Compound 3 (4.8 g, 10.78mmol) was dissolved in anhydrous DCM (40 mL) under stirring well, then EDCI (2.06 g, 10.78 mmol) and Compound 3-1 (2.18 g, 10.78 mmol) were added and allowed to react at 25°C for 12 h. After the reaction was completed, the reaction mixture was isolated directly by preparative HPLC, and concentrated to give 4 as a colorless oil (4.0 g, 58.9%).
**(4)** Compound 4 (4.0 g, 6.35 mmol) was dissolved in anhydrous DMF (40 mL) under stirring well, then DCC (1.31 g, 6.35 mmol) and DMAP (0.155 g, 1.27 mmol) and Compound 4-1 (2.35 g, 6.35 mmol) were added and allowed to react at 25°C for 2 h. After the reaction was completed, the reaction mixture was isolated directly by preparative HPLC, and concentrated to give C18 ester as a colorless oil (1.8 g, 28.8%), with a molecular weight of 982.27.

Structure elucidation of C18 ester:
Mass spectrometry:
   MS-ESI (m/z): 982.5 [M+H]+
Nuclear magnetic hydrogen spectrum:
   ¹H NMR (400MHz, DMSO-d₆) δ 8.11 (m, 2H, NH) 7.91~7.62 (m, 2H, NH) 7.01 (S, 2H, CH) 3.87~3.59 (m, 1H, CH) 3.57 (S, 2H, CH₂) 3.56~3.55 (m, 10H, CH₂) 3.50~3.28 (m, 6H, CH₂) 3.21~3.16 (m, 6H, CH₂) 2.25 (S, 2H, CH₂) 2.16~2.10 (m, 6H, CH₂) 1.92~1.85 (m, 2H, CH₂) 1.40~1.35 (m, 22H, CH₂, CH₃) 1.30~1.23 (m, 24H, CH₂, CH₃)
Nuclear magnetic carbon spectrum:
   ¹³C NMR (100MHz, DMSO-d₆) δ 172.78, 171.78, 171.19, 169.95, 169.70, 135.02, 80.75, 79.76, 70.62, 70.42, 69.94, 69.81, 69.47, 69.35, 52.77, 38.94, 35.23, 34.53, 29.50, 29.38, 29.09, 28.81, 28.23, 28.09, 25.06

### 1.3 Preparation of advanced fatty acid chain C16-NHS

The fatty acid chain C16-NHS was prepared according to the following technical route: 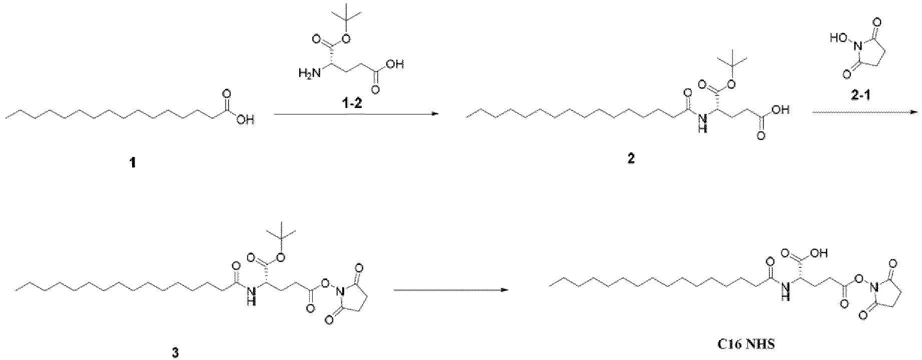
**(1)** Compound 1 (3.0 g, 11.7 mmol) was dissolved in anhydrous DCM (30mL) under stirring well, then DCC (2.41 g, 11.7 mmol) and DMAP (0.285 g, 2.34 mmol) were added and allowed to react at 25°C for 12 h. After the reaction was completed, the reaction mixture was isolated directly by preparative HPLC, and concentrated to give 2 as a colorless oil (2.1 g, 41.2%).
**(2)** Compound 2 (2.1 g, 4.76 mmol) was dissolved in anhydrous DCM (20 mL) under stirring well, then TSTU (1.43 g, 4.76 mmol) and DIPEA (0.614 g, 4.76 mmol) and Compound 2-1 (1.64 g, 14.28 mmol) were added and allowed to react at 25°C for 1 h. After the reaction was completed, the reaction mixture was concentrated directly to give 3 as a brownish-black oil (2.0 g, 78.1%).
**(3)** Compound 3 (2.0 g, 3.71 mmol) was dissolved in anhydrous DCM (20 mL) under stirring well, then TFA 2 mL was added and allowed to react at 25°C for 1 h. After the reaction was completed, the reaction mixture was isolated directly by preparative HPLC, and concentrated to give C16 ester (also named as C16-NHS) as a colorless oil (1.2 g, 70.5%), with a molecular weight of 482.26.

Structure elucidation of C16-NHS ester:
Mass spectrometry:
   MS-ESI (m/z): 483.3 [M+H]+
Nuclear magnetic hydrogen spectrum:
   ¹H NMR (400MHz, DMSO-d₆) δ 12.71 (S, 1H, COOH) 8.20 (S, 1H, NH) 4.32~4.31 (m, 1H, CH) 2.88~2.71 (m, 6H, CH₂) 2.18~2.17 (m, 4H, CH₂) 1.61~1.52 (m, 2H, CH₂) 1.30~1.28 (m, 24H, CH₂) 0.94~0.90 (m, 3H, CH₃)
Nuclear magnetic carbon spectrum:
   ¹³C NMR (100MHz, DMSO-d₆) δ 173.43, 172.95, 170.59, 168.87, 51.21, 35.52, 31.77, 29.52, 29.18, 29.05, 27.60, 26.42, 25.90, 25.64, 22.56, 14.41

### 1.4 Preparation of advanced fatty acid chain C20-NHS

The fatty acid chain C20-NHS was prepared according to the following technical route: 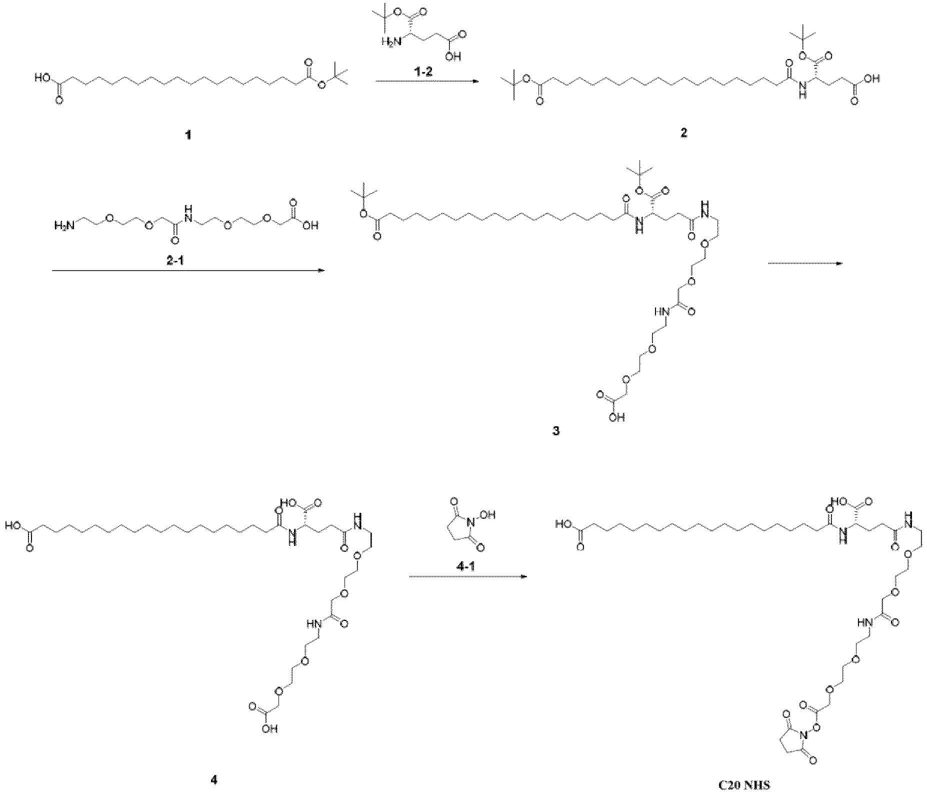
**(1)** Compound 1 (5.0 g, 12.56 mmol) was dissolved in anhydrous DCM (50 mL) under stirring well, then DCC (2.58 g, 12.56 mmol) and DMAP (0.306 g, 2.51 mmol) and Compound 1-2 (2.54 g, 12.56 mmol) were added and allowed to react at 25°C for 2 h. After the reaction was completed, the reaction mixture was isolated directly by preparative HPLC, and concentrated to give 2 as a colorless oil (6.1g, 83.56%).
**(2)** Compound 2 (6.1 g, 10.4 mmol) was dissolved in anhydrous DCM (60mL) under stirring well, then DCC (2.14 g, 10.4 mmol) and DMAP (0.53 g, 2.08 mmol) and Compound 2-1 (3.2 g, 10.4 mmol) were added and allowed to react at 25°C for 2 h. After the reaction was completed, the reaction mixture was isolated directly by preparative HPLC, and concentrated to give 3 as a colorless oil (4.5g, 51%).
**(3)** Compound 3 (4.5 g, 5.15 mmol) was dissolved in anhydrous DCM (40 mL) under stirring well, then TFA 4 mL was added and allowed to react at 25°C for 1 h. After the reaction was completed, the reaction mixture was isolated directly by preparative HPLC, and concentrated to give 4 as a brown oil (3.2 g, 82%).
**(4)** Compound 4 (3.2 g, 4.20 mmol) was dissolved in anhydrous DMF (40 mL) under stirring well, then DCC (0.865 g, 4.20 mmol) and DMAP (0.103 g, 0.84 mmol) and Compound 4-1 (0.483 g, 4.2 mmol) were added and allowed to react at 25°C for 2 h. After the reaction was completed, the reaction mixture was isolated directly by preparative HPLC, and concentrated to give C20 ester (also named as C20-NHS) as a white solid (1.5 g, 41.7%), with a molecular weight of 859.02.

Structure elucidation of C20-NHS ester:
Mass spectrometry:
   MS-ESI (m/z): 859.4 [M+H]+
Nuclear magnetic hydrogen spectrum:
   ¹H NMR (400MHz, DMSO-d₆) δ 12.41 (S, 2H, COOH) 8.11~7.72(m, 3H, NH) 4.66 (S, 1H, CH) 3.92~3.61(m, 4H, CH₂) 3.49~3.40(m, 18H, CH₂) 3.44~3.33(m, 4H, CH₂) 2.88~2.21(m, 6H, CH₂) 2.18~2.10(m, 2H, CH₂) 1.52~1.28(m, 30H, CH₂)
Nuclear magnetic carbon spectrum:
   ¹³C NMR (100MHz, DMSO-d₆) δ 174.95, 174.00, 172.81, 171.87, 170.50, 169.72, 167.05, 70.84, 70.62, 70.41, 69.80, 69.56, 69.33, 66.23, 39.88, 34.12, 29.54, 29.37, 29.30, 29.01, 25.94, 25.69, 24.96

### Example 2. Preparation of GLP-1-Fc-TM1 sample and detection of its DAR value

In this example, with the commercial GLP-1-Fc fusion protein Dulaglutide as an example, a conjugate based on the Fc-advanced fatty acid chain platform of the present application was constructed. Dulaglutide consists of 2 identical long chains, one of which has the amino acid sequence shown below:

HGEGTFTSDV SSYLEEQAAK EFIAWLVKGG GGGGGSGGGG SGGGGSAESK YGPPCPPCPA
PEAAGGPSVF LFPPKPKDTL MISRTPEVTC VVVDVSQEDP EVQFNWYVDG VEVHNAKTKP
REEQFNSTYR VVSVLTVLHQ DWLNGKEYKC KVSNKGLPSS IEKTISKAKG QPREPQVYTL
PPSQEEMTKN QVSLTCLVKG FYPSDIAVEW ESNGQPENNY KTTPPVLDSD GSFFLYSRLT
VDKSRWQEGN VFSCSVMHEA LHNHYTQKSL SLSLG (SEQ ID NO: 1)

The Fc contained in Dulaglutide has the amino acid sequence shown below:

AESKYGPPCPPCPAPEAAGGPSVFLFPPKPKDTLMISRTPEVTCVVVDVSQEDPEVQFNWYVDG

VEVHNAKTKPREEQFNSTYRVVSVLTVLHQDWLNGKEYKCKVSNKGLPSSIEKTISKAKGQPREPQV
YTLPPSQEEMTKNQVSLTCLVKGFYPSDIAVEWESNGQPENNYKTTPPVLDSDGSFFLYSRLTVDKS
RWQEGNVFSCSVMHEALHNHYTQKSLSLSLG (SEQ ID NO: 16)

### 1.1 Conjugation of GLP-1-Fc fusion protein to TM1

7.5 mg GLP-1-Fc fusion protein (Dulaglutide, Homemade) was exchanged into a reducing buffer (25 mM sodium borate, 30 mM NaCl, 5 mM EDTA, pH8.0) via a 15 ml of 30KD ultrafiltration tube, repeated 4 times; the resulting sample with a final volume of about 1 ml was detected for the concentrations of proteins. To the sample was added a 3-fold molar excess of TCEP and allowed to react in a water bath at 25°C for 2 h; followed by exchanging into a conjugation buffer (50 mM Tris, 150 mM NaCl, 5 mM EDTA, pH 7.5) via 15 ml of 30KD ultrafiltration tube, repeat 4 times. The sample was then detected for the concentrations of proteins and the number of free thiol groups.

To the GLP-1-Fc fusion protein sample was added a 5-fold molar excess of TM1 according to the amount of proteins, the mixture was blended uniformly and allowed to react at 25°C for 2 h under shaking by a microplate shaker. The sample was purified by cation chromatography immediately after conjugation.

The purified sample was designated as GLP-1-Fc-TM1 (also known as GLP1-Fc-TM1) and exchanged into a temporary buffer (25 mM phosphate, 150 m NaCl, pH 7.0) for further detection.

### 1.2 Detection of DAR value of GLP1-Fc-TM1 sample

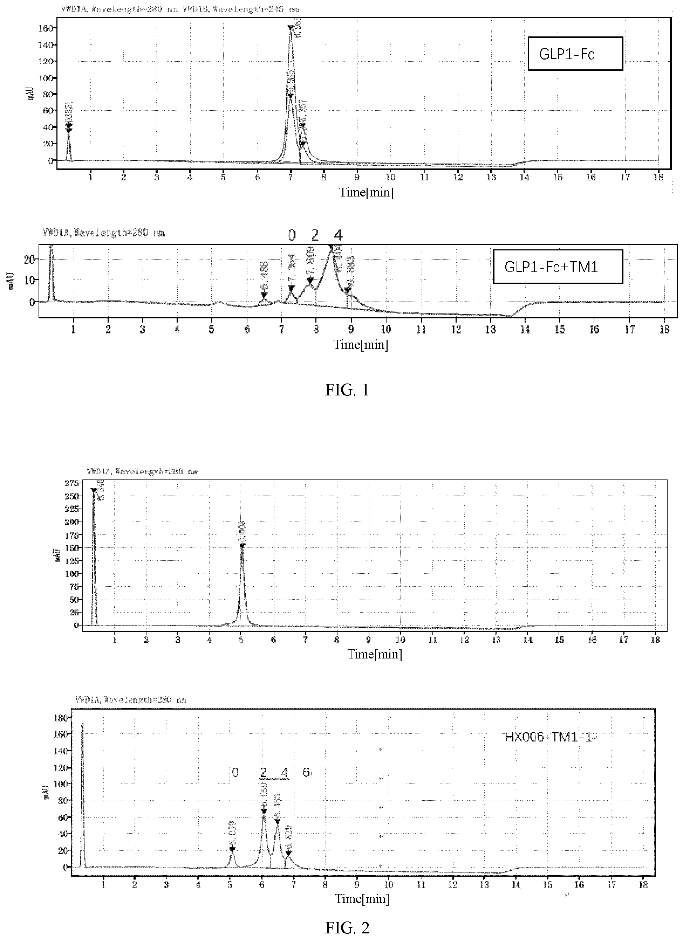

The conjugation ratio (DAR) of GLP1-Fc and TM1 was determined by HIC-HPLC assay in this example. The used analytical column was TSKgel Butyl-NPR (4.6 mm*3.5 cm), and the analytical method was known method recorded in Drug-to-Antibody Ratio(DAR) and Drug Load Distribution by Hydrophobic Interaction Chromatography and Reversed Phase High-Performance Liquid Chromatography. The detection results were shown in FIG. 1, and an average DAR value of the TM1 conjugated to the GLP1-Fc protein is 3.3.

### Example 3. Preparation and detection of GLP-1-Fc-C18

In this example, with the commercial GLP-1-Fc fusion protein Dulaglutide as an example, a conjugate based on the Fc-advanced fatty acid chain platform of the present application was constructed.

8.34 mg GLP1-Fc fusion protein (Dulaglutide, Homemade) was exchanged into a reducing buffer (25 mM sodium borate, 30 mM NaCl, 5 mM EDTA, pH8.0) via a 15 ml of 30KD ultrafiltration tube, repeated 4 times; the resulting sample with a final volume of about 3 ml was detected for the concentrations of proteins. To the antibody was added a 3-fold molar excess of TCEP and allowed to react in a water bath at 25°C for 2 h; followed by exchanging into a conjugation buffer (50 mM Tris, 150 mM NaCl, 5 mM EDTA, pH 7.5) via a 15 ml of 30KD ultrafiltration tube, repeat 4 times. The sample was then detected for the concentrations of proteins and the number of free thiol groups.

To the GLP-1-Fc fusion protein sample was added a 3-fold molar excess of C18-tert-butanol ester according to the amount of proteins, the mixture was blended uniformly and allowed to react at 25°C for 2 h under shaking by a miniature shaker. The sample was purified by cation chromatography immediately after conjugation.

The purified protein was designated as GLP-1-Fc-C18-tert-butanol ester (also known as GLP1-Fc-C18 tert-butanol ester) and exchanged into a temporary buffer for further detection.

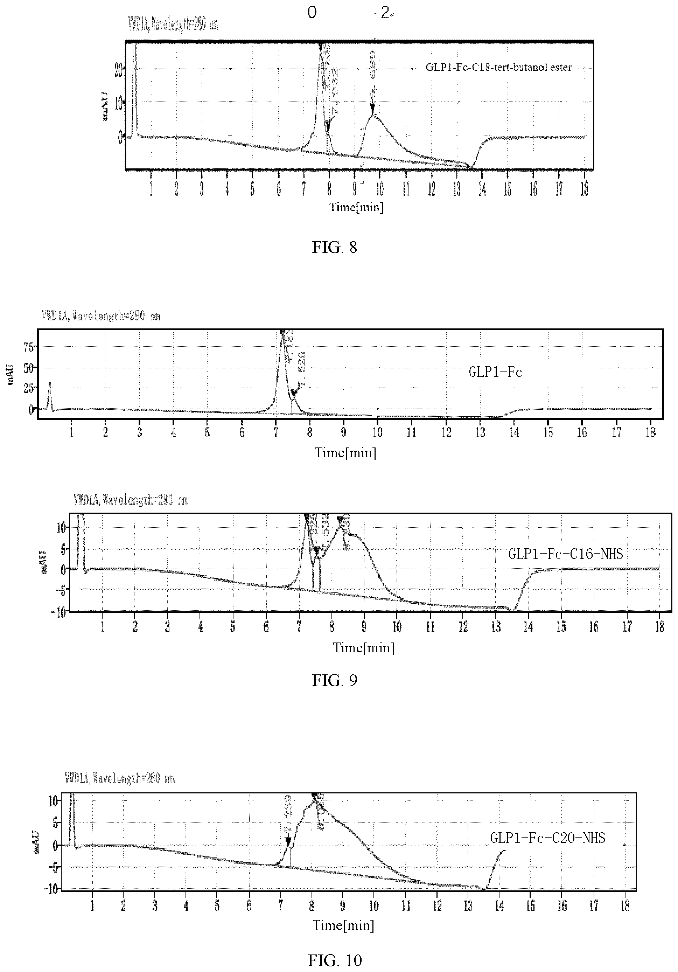

In this example, the conjugation ratio (DAR) of GLP1-Fc and C18-tert-butanol ester was determined by HIC-HPLC assay according to the method in Example 2. The results were shown in FIG. 8, with an average DAR value of 1.26.

### Example 4. Preparation and detection of sample GLP1-Fc-C16-NHS

In this example, with the commercial GLP-1-Fc fusion protein Dulaglutide as an example, a conjugate, which is based on the Fc-advanced fatty acid chain platform of the present application and linked through amino groups of lysine residues on Fc, was constructed.

4.56 mg GLP-1-Fc fusion protein was exchanged into a conjugation buffer (0.1M MOPS, 20 mM Tris, pH 7.5) via a 30KD ultrafiltration tube, the resulting sample with a final volume of 0.9 ml was detected for the concentrations of proteins. To the fusion protein sample was added a 6-fold molar excess of C16-NHS. The mixture was blended uniformly and allowed to react at 25°C for 2 h under shaking by a miniature shaker. The sample was purified by cation chromatography immediately after conjugation, then exchanged into a temporary buffer via a 30KD ultrafiltration tube, and detected for the concentrations. The purified product was designated as GLP1-Fc-C16-NHS or GLP1-Fc-C16.

In this example, the detection was carried out by HIC-HPLC assay according to the method in Example 2. The results were shown in FIG. 9, and the conjugation ratio of C16-NHS to GLP1-Fc is 83.07%.

### Example 5. Preparation and detection of sample GLP1-Fc-C20-NHS

In this example, with the commercial GLP-1-Fc fusion protein Dulaglutide as an example, a conjugate, which is based on the Fc-advanced fatty acid chain platform of the present application and linked through amino groups of lysine residues on Fc, was constructed.

4.56 mg of GLP1-Fc fusion protein was exchanged into a conjugation buffer (0.1M MOPS, 20 mM Tris, pH 7.5) via a 30KD ultrafiltration tube, the resulting sample with a final volume of 0.9 ml was detected for the concentrations of proteins. To the fusion protein sample was added a 6-fold molar excess of C20-NHS. The mixture was blended uniformly and allowed to react at 25°C for 2 h under shaking by a miniature shaker. The sample was purified by cation chromatography immediately after conjugation, then exchanged into a temporary buffer via a 30KD ultrafiltration tube, and detected for the concentrations. The purified product was designated as GLP1-Fc-C20-NHS or GLP1-Fc-C20.

In this example, the detection was carried out by HIC-HPLC assay according to the method in Example 2. The results were shown in FIG. 10, and the conjugation ratio of C20-NHS to GLP1-Fc is 96.38%.

### Example 6. Binding activity of GLP1-Fc-C16-NHS and GLP1-Fc-C20-NHS to HSA

In this example, the binding activity of each of GLP1-Fc-C16-NHS and GLP1-Fc-C20-NHS, which were obtained by conjugating lysine of GLP1-Fc fusion protein, to HSA was detected by ELISA.

8 ELISA plates were coated with HSA-his that was diluted in a coating solution (carbonate buffer) to 0.5 µg/ml, and incubated overnight at 4°C. The plates were washed with PBST (PBS containing 0.05% Tween 20) for 3 times, then added with a blocking solution (1% BSA in PBST) at 200 µl/well, and incubated at 37°C for 1 h. The plates were washed with PBST for 4 times. GLP1-Fc-C16-NHS and GLP1-Fc-C20-NHS were respectively diluted with diluent (PBST solution containing 1‰ BSA) to 1000 nM as the initial concentration, and then 7 dilutions of 5-fold gradient were made, for a total of 8 gradients. Each of the dilutions was added in the plate at 100 µl/well and incubated at 37°C for 1 h. The plates were washed with PBST for 5 times, added with a working solution of HRP-Goat anti-Huamn 10000X at 100 µl/well, and incubated at 37°C for 1 h. The plates were washed with PBST for 6 times, added with a freshly prepared color-developing solution (5 ml substrate solution, 250 µl TMB stock solution, 16 µl 0.75% H₂O₂) at 100 µl/well, and incubated at 37°C for 10 min. The OD value was measured at 450 nm after adding a stop solution (2M H₂SO₄) at 50 µl/well.

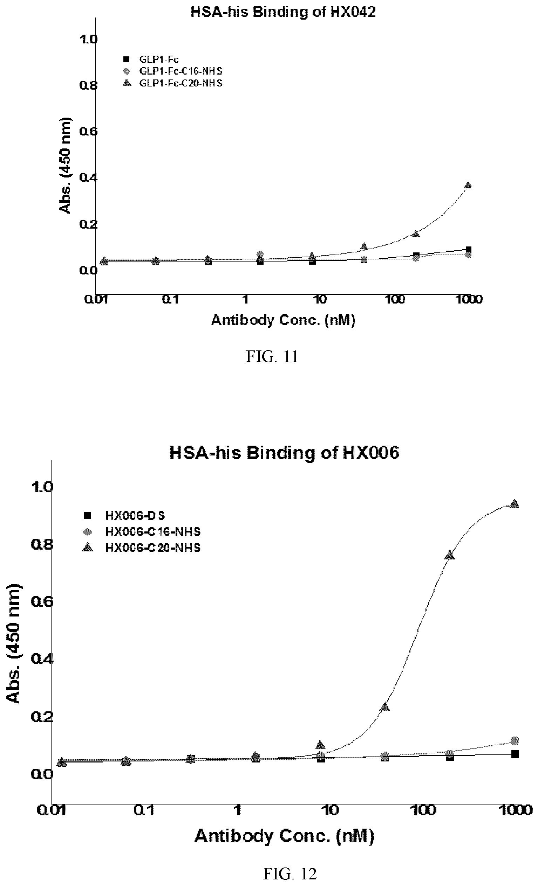

The results were shown in FIG. 11. The results showed that the sample GLP1-Fc-C20-NHS after conjugating to C20-NHS exhibited strong binding to HSA-his. The sample GLP1-Fc-C16-NHS after conjugating to C16-NHS exhibited weaker binding to HSA-his than GLP1-Fc-C20-NHS.

### Example 7. Preparation of a sample conjugated by TM1and HX006 antibody

In this example, with the anti-VEGF antibody HX006 disclosed in the CN 104804088A patent as an example, a conjugate was constructed based on the Fc-advanced fatty acid chain platform of the present application, wherein the HX006 antibody is of the IgG1 type, with the heavy chain sequence and light chain sequence shown in the following table.

| Heavy chain CDR | HCDR1: TYGMS (SEQ ID NO: 2) | Light chain CDR | LCDR1: KASQSVSNDVV (SEQ ID NO: 5) |
|---|---|---|---|
| | HCDR2: TISNGGSYTFYPDSVKG (SEQ ID NO: 3) | | LCDR2: YASNRYT (SEQ ID NO: 6) |
| | HCDR3 : HGSVTTRGFDY (SEQ ID NO: 4) | | LCDR3: LQDYSSPFT (SEQ ID NO: 7) |
| Heavy chain | EVQLVQSGSELKKPGASVKVSCKASGYTFTNYGLNWVRQAPGQGLEWMGWINTYTGEPTYA DDFKGRFVFSLDTSASTAYLQISSLKAEDMAVYYCAKYGITYEWYFDVWGQGTMVTVSSAS TKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSRWQQGNVFS CSVMHEALHNHYTQKSLSLSPGK (SEQ ID NO: 13) | | |
| Fc | EPKSCDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTIS | | |
| | KAKGQPREPQVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK (SEQ ID NO: 15) | | |
| Light chain | DIQMTQSPSSLSASVGDRVTITCRASQDISNSLNWYQQKPGKAPKLLIYYTSRLHSGVPSR FSGSGSGTDFTFTISSLQPEDIATYYCQQGNTFPWTFGSGTKLEIKRTVAAPSVFIFPPSD EQLKSGTASVVCLLNNFYPREAKVQWKVDNALQSGNSQESVTEQDSKDSTYSLSSTLTLSK ADYEKHKVYACEVTHQGLSSPVTKSFNRGEC (SEQ ID NO: 14) | | |

6.53 mg HX006 antibody protein was exchanged into a reducing buffer (25 mM sodium borate, 30 mM NaCl, 5 mM EDTA, pH8.0) via a 15 ml of 30KD ultrafiltration tube, repeat 4 times; the resulting sample with a final volume of about 2 ml was detected for the concentrations of proteins. To the antibody was added a 2-fold molar excess of TCEP and allowed to react in a water bath at 25°C for 2 h; followed by exchanging into a conjugation buffer (50 mM Tris, 150 mM NaCl, 5 mM EDTA, pH 7.5) via a 15 ml of 30KD ultrafiltration tube, repeat 4 times. The sample was then detected for the concentrations of proteins and the number of free thiol groups.

To the antibody sample was added a 2-fold molar excess of TM1 according to the amount of proteins, the mixture was blended uniformly and allowed to react at 25°C for 2 h under shaking by a microplate shaker. The sample was purified by cation chromatography immediately after conjugation.

The purified protein was designated as HX006-TM1-1 and exchanged into a temporary buffer for further detection.

In this example, the conjugation ratio (DAR) of HX006 and TM1 in the molecule HX006-TM1-1 was determined by HIC-HPLC assay according to the method in Example 2.

The detection results were shown in FIG. 2. An average DAR value of the TM1 conjugated to HX006 antibody in the molecule HX006-TM1-1 is 3.0.

### Example 8. Preparation of a sample conjugated by HX006 antibody and TM1

In this example, a conjugation of HX006 antibody and TM1 was further prepared.

6.56 mg HX006 antibody protein was exchanged into a reducing buffer (25 mM sodium borate, 30 mM NaCl, 5 mM EDTA, pH 8.0) via a 15 ml of 30KD ultrafiltration tube, repeat 4 times; the resulting sample with a final volume of about 2 ml was detected for the concentrations of proteins. To the antibody was added a 3-fold molar excess of TCEP and allowed to react in a water bath at 25°C for 2.5 h; followed by exchanging into a conjugation buffer (50 mM Tris, 150 mM NaCl, 5 mM EDTA, pH 7.5) via a 15 ml of 30KD ultrafiltration tube, repeat 4 times. The sample was then detected for the concentrations of proteins and the number of free thiol groups.

To the antibody sample was added a 4-fold molar excess of TM1 according to the amount of proteins, the mixture was blended uniformly and allowed to react at 25°C for 2 h under shaking by a microplate shaker. The sample was purified by cation chromatography immediately after conjugation.

The purified protein was designated as HX006-TM1-2 and exchanged into a temporary buffer for further detection.

In this example, the conjugation ratio (DAR) of HX006 and TM1 in the molecule HX006-TM1-2 was determined by HIC-HPLC assay according to the method in Example 2.

The detection results were shown in FIG. 3, an average DAR value of the TM1 conjugated to HX006 antibody in the molecule HX006-TM1-2 is 4.67.

### Example 9 Preparation of a sample conjugated by C18-tert-butanol ester and HX006 antibody

In this example, a conjugation of HX006 antibody and C18 tert-butanol ester was prepared.

4.3 mg HX006 antibody protein was exchanged into a reducing buffer (25 mM sodium borate, 30 mM NaCl, 5 mM EDTA, pH 8.0) via a 15 ml of 30KD ultrafiltration tube, repeat 4 times; the resulting sample with a final volume of about 2 ml was detected for the concentrations of proteins. To the antibody was added a 3-fold molar excess of TCEP and allowed to react in a water bath at 25°C for 2 h; followed by exchanging into a conjugation buffer (50 mM Tris, 150 mM NaCl, 5 mM EDTA, pH 7.5) via a 15 ml of 30KD ultrafiltration tube, repeat 4 times. The sample was then detected for the concentrations of proteins and the number of free thiol groups.

To the antibody sample was added a 3-fold molar excess of C18 tert-butanol ester according to the amount of proteins, the mixture was blended uniformly and allowed to react at 25°C for 2 h under shaking by a microplate shaker. The sample was purified by cation chromatography immediately after conjugation.

The purified protein was designated as HX006-C18-tert-butanol ester (also known as HX006-C18) and exchanged into a temporary buffer for further detection.

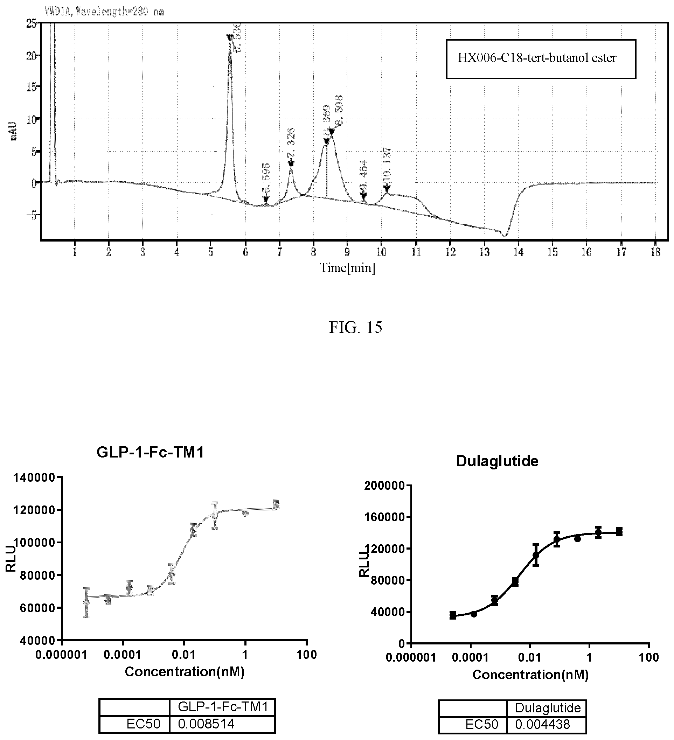

In this example, the conjugation ratio (DAR) of HX006 and C18-tert-butanol ester was determined by HIC-HPLC assay according to the method in Example 2. The results were shown in FIG. 15, with an average DAR value of 2.95.

### Example 10 Preparation of a sample conjugated by C16-NHS and HX006 antibody

4 mg HX006 antibody protein was exchanged into a conjugation buffer (0.1M MOPS, 20 mM Tris, pH 7.5) via a 30KD ultrafiltration tube, the resulting sample with a final volume of 0.9 ml was detected for the concentrations of protein. To the fusion protein sample was added a 6-fold molar excess of C16-NHS. The mixture was blended uniformly and allowed to react at 25°C for 2 h under shaking by a miniature shaker. The sample was purified by cation chromatography immediately after conjugation, and then exchanged into a temporary buffer via a 30KD ultrafiltration tube, and detected for the concentrations of proteins. The protein was designated as HX006-C16-NHS.

### Example 11. Preparation of a sample conjugated by C20-NHS and HX006 antibody

4 mg HX006 antibody protein was exchanged into a conjugation buffer (0.1M MOPS, 20 mM Tris, pH 7.5) via a 30KD ultrafiltration tube, the resulting sample with a final volume of 0.9 ml was detected for the concentrations of proteins. To the fusion protein sample was added a 6-fold molar excess of C20-NHS. The mixture was blended uniformly and allowed to react at 25°C for 2 h under shaking by a miniature shaker. The sample was purified by cation chromatography immediately after conjugation, and then exchanged into a temporary buffer via a 30KD ultrafiltration tube, and detected for the concentrations of proteins. The protein was designated as HX006-C20-NHS.

### Example 12. Binding activity of HX006-C16-NHS and HX006-C20-NHS to HSA

In this example, the binding activity of samples HX006-C16-NHS and HX006-C20-NHS, which were obtained by conjugating lysine of Fc of the HX006 antibody protein, to HSA was detected by ELISA.

8 ELISA plates were coated with HSA-his that was diluted in a coating solution to 0.5 µg/ml, and incubated overnight at 4°C. The plates were washed with PBST (PBS containing 0.05% Tween 20) for 3 times, then added with a blocking solution at 200 µl/well, and incubated at 37°C for 1 h. The plates were washed with PBST for 4 times. HX006-C16-NHS and HX006-C20-NHS were respectively diluted with diluent to 1000 nM as the initial concentration, and then 7 dilutions of 5-fold gradient were made, for a total of 8 gradients. Each of the dilutions was added in the plate at 100 µl/well, and incubated at 37°C for 1 h. The plates were washed with PBST for 5 times, added with a working solution of HRP-Goat anti-Huamn 10000X at 100 µl/well, and incubated at 37°C for 1 h. The plates were washed with PBST for 6 times, added with a freshly prepared color-developing solution at 100 µl/well, and incubated at 37°C for 10 min. The OD value was measured at 450 nm after adding a stop solution (2M H₂SO₄) at 50 µl/well.

The results were shown in FIG. 12. The results showed that the sample HX006-C20-NHS after conjugating to C20-NHS exhibited strong binding to HSA-his. The sample HX006-C16-NHS after conjugating to C16-NHS exhibited weaker binding to HSA-his than HX006-C20-NHS.

### Example 13. Preparation of a sample conjugated by TM1 and HX008 antibody

In this example, with the anti-PD-1 antibody H8L2 (referred to herein as HX008) disclosed in patent CN108299560A as an example, a conjugate based on the Fc-advanced fatty acid chain platform of the present application was constructed, wherein the HX008 antibody is of the IgG4 type, with the sequence as shown below:
The sequence of heavy chain of HX008 antibody is as follows: 
EVQLVQSGGGLVQPGGSLKLSCAASGFTFSSYGMSWVRQAPGKGLDWVATISGGGRDTYYPDSV
KGRFTISRDNSKNNLYLQMNSLRAEDTALYYCARQKGEAWFAYWGQGTLVTVSSASTKGPSVFPLAP
CSRSTSESTAALGCLVKDYFPEPVTVSWNSGALTSGVHTFPAVLQSSGLYSLSSVVTVPSSSLGTKT
YTCNVDHKPSNTKVDKRVESKYGPPCPPCPAPEFLGGPSVFLFPPKPKDTLMITRTPEVTCVVVDVS
QEDPEVQFNWYVDGVEVHNAKTKPREEQFNSTYRVVSVLTPLHQDWLNGKEYKCKVSNKGLPSSIEK
TISKAKGQPREPQVYTLPPSQEEMTKNQVSLTCLVKGFYPSDIAVEWESNGQPENNYKTTPPVLDSD
GSFFLYSRLTVDKSRWQEGNVFSCSVMHEALHAHYTQKSLSLSLGK (SEQ ID NO: 8)
The sequence of heavy chain variable region of HX008 antibody is as follows: 
EVQLVQSGGGLVQPGGSLKLSCAASGFTFSSYGMSWVRQAPGKGLDWVATISGGGRDTYYPDSV
KGRFTISRDNSKNNLYLQMNSLRAEDTALYYCARQKGEAWFAYWGQGTLVTVSS (SEQ ID NO: 9)
The sequence of Fc-region is as follows: 
VESKYGPPCPPCPAPEFLGGPSVFLFPPKPKDTLMITRTPEVTCVVVDVSQEDPEVQFNWYVDG
VEVHNAKTKPREEQFNSTYRVVSVLTPLHQDWLNGKEYKCKVSNKGLPSSIEKTISKAKGQPREPQV
YTLPPSQEEMTKNQVSLTCLVKGFYPSDIAVEWESNGQPENNYKTTPPVLDSDGSFFLYSRLTVDKS
RWQEGNVFSCSVMHEALHAHYTQKSLSLSLGK (SEQ ID NO: 10).
The sequence of light chain variable region of HX008 antibody is as follows: 
DIVLTQSPASLAVSPGQRATITCRASESVDNYGISFMNWFQQKPGQPPKLLIYAASNKGTGVPA
RFSGSGSGTDFTLNINPMEENDTAMYFCQQSKEVPWTFGGGTKLEI (SEQ ID NO: 11)
The sequence of light chain of HX008 antibody is as follows:

DIVLTQSPASLAVSPGQRATITCRASESVDNYGISFMNWFQQKPGQPPKLLIYAASNKGTGVPA
RFSGSGSGTDFTLNINPMEENDTAMYFCQQSKEVPWTFGGGTKLEIKRTVAAPSVFIFPPSDEQLKS
GTASVVCLLNNFYPREAKVQWKVDNALQSGNSQESVTEQDSKDSTYSLSSTLTLSKADYEKHKVYAC
EVTHQGLSSPVTKSFNRGEC (SEQ ID NO: 12)

4 mg HX008 antibody protein was exchanged into a reducing buffer (25 mM sodium borate, 30 mM NaCl, 5 mM EDTA, pH 8.0) via a 15 ml of 30KD ultrafiltration tube, repeat 4 times; the resulting sample with a final volume of about 1 ml was detected for the concentrations of proteins. To the antibody was added a 8-fold molar excess of TCEP and allowed to react in a water bath at 30°C for 2.5 h; followed by exchanging into a conjugation buffer (50 mM Tris, 150 mM NaCl, 5 mM EDTA, pH 7.5) via a 15 ml of 30KD ultrafiltration tube for 4 times. The sample was then detected for the concentrations of proteins and the number of free thiol groups.

To the fusion protein sample was added a 4-fold molar excess of TM1 according to the amount of proteins, the mixture was blended uniformly and allowed to react at 25°C for 2 h under shaking by a microplate shaker. The sample was purified by cation chromatography immediately after conjugation.

The purified protein was designated as HX008-TM1-2 and exchanged into a temporary buffer for further detection.

In this example, the conjugation ratio (DAR) of HX008 and TM1 in the molecule HX008-TM1-2 was determined by HIC-HPLC assay according to the method in Example 2.

The results were shown in FIG. 4, with an average DAR value of the TM1 conjugated to HX008 antibody in the molecule HX008-TM1-2 of 1.45.

### Example 14. Preparation of a sample conjugated by TM1 and HX008 antibody

In this example, a conjugation of HX008 antibody and TM1 was further prepared.

4 mg HX008 antibody protein was exchanged into a reducing buffer (25 mM sodium borate, 30 mM NaCl, 5 mM EDTA, pH8.0) via a 15 ml of 30KD ultrafiltration tube, repeat 4 times; the resulting sample with a final volume of about 1ml was detected for the concentrations of proteins. To the antibody was added a 10-fold molar excess of TCEP and allowed to react in a water bath at 30°C for 2.5 h; followed by exchanging into a conjugation buffer (50 mM Tris, 150 mM NaCl, 5 mM EDTA, pH 7.5) via a 15 ml of 30KD ultrafiltration tube for 4 times. The sample was then detected for the concentrations of proteins and the number of free thiol groups.

To the fusion protein sample was added a 5-fold molar excess of TM1 according to the amount of proteins, the mixture was blended uniformly and allowed to react at 25°C for 2 h under shaking by a microplate shaker. The sample was purified by cation chromatography immediately after conjugation.

The purified protein was designated as HX008-TM1-3 and exchanged into a temporary buffer for further detection.

In this example, the conjugation ratio (DAR) of HX008 and TM1 in the molecule HX008-TM1-3 was determined by HIC-HPLC assay according to the method in Example 2.

The results were shown in FIG. 4, with an average DAR value of the TM1 conjugated to HX008 antibody in the molecule HX008-TM1-3 of 2.0.

### Example 15. Binding activity of GLP-1-Fc-TM1 to HSA

The fatty acid chain contained in TM1 could bind to serum albumin (HSA), and the binding of fusion GLP-1-Fc-TM1 to HSA was detected by ELISA in this Example. 3 ELISA plates were coated with HSA-his that was diluted in a coating solution (carbonate buffer) to 0.5 µg/ml, and incubated overnight at 4°C. The plates were washed with PBST (PBS solution containing 0.05% Tween 20) for 3 times, then added with a blocking solution (PBST containing 1% BSA) at 200 µl/well, and incubated at 37°C for 1 h. The plates were washed with PBST for 4 times. GLP-1-Fc-TM1 was diluted with diluent (PBST solution containing 1‰ BSA) to 1000 nM as the initial concentration, and then 7 dilutions of 5-fold gradient were made, for a total of 8 gradients. Each of the dilutions was added in the plate at 100 µl/well and incubated at 37°C for 1 h. The plates were washed with PBST for 5 times, added with a working solution of HRP-Goat anti-Huamn 10000X at 100 µl/well, and incubated at 37°C for 1 h. The plates were washed with PBST for 6 times, added with a freshly prepared color-developing solution (5 ml substrate solution, 250 µl TMB stock solution, 16 µl 0.75% H₂O₂) at 100 µl/well, and incubated at 37°C for 10 min. The OD value was measured at 450 nm after adding a stop solution (2M H₂SO₄) at 50 µl/well.

The results were shown in FIG. 5. The results showed that the sample GLP1-Fc-TM1 after conjugating to TM1 exhibited strong binding to HSA-his.

### Example 16. Binding activity of GLP-1-Fc-C18-tert-butanol ester to HSA

The binding of fusion GLP-1-Fc-C18-tert-butanol ester to HSA was detected by ELISA in this Example. 3 ELISA plates were coated with HSA-his that was diluted in a coating solution to 0.5 µg/ml, and incubated overnight at 4°C. The plates were washed with PBST (PBS solution containing 0.05% Tween 20) for 3 times, then added with a blocking solution at 200 µl/well, and incubated at 37°C for 1 h. The plates were washed with PBST for 4 times. GLP-1-Fc-C18-tert-butanol ester was diluted with diluent to 1000 nM as the initial concentration, and then 7 dilutions of 5-fold gradient were made, for a total of 8 gradients. Each of the dilutions was added in the plate at 100 µl/well, and incubated at 37°C for 1 h. The plates were washed with PBST for 5 times, added with a working solution of HRP-Goat anti-Huamn 10000X at 100 µl/well, and incubated at 37°C for 1 h. The plates were washed with PBST for 6 times, added with a freshly prepared color-developing solution at 100 µl/well, and incubated at 37°C for 10 min. The OD value was measured at 450 nm after adding a stop solution (2M H₂SO₄) at 50 µl/well.

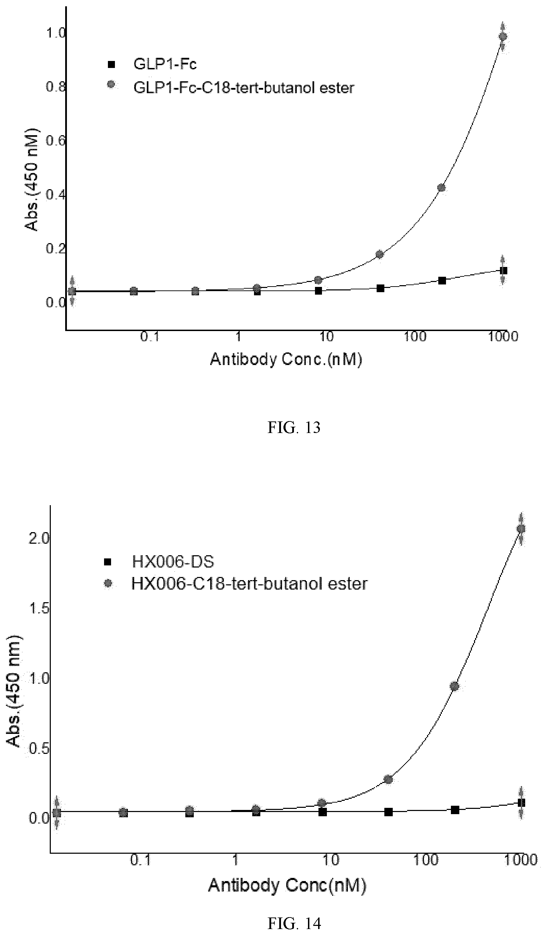

The results were shown in FIG. 13. The results showed that the sample GLP-1-Fc-C18-tert-butanol ester after conjugating to C18-tert-butanol este exhibited strong binding to HSA-his.

### Example 17. Binding activity of the HX006-TM1 to HSA

The binding of fusion HX006-TM1 to HSA was detected by ELISA in this Example. 8 ELISA plates were coated with HSA-his that was diluted in a coating solution to 0.5 µg/ml, and incubated overnight at 4°C. The plates were washed with PBST (0.05% Tween 20 in PBS) for 3 times, then added with a blocking solution at 200 µl/well, and incubated at 37°C for 1 h. The plates were washed with PBST for 4 times. HX006-TM1 was diluted with diluent to 1000 nM as the initial concentration, and then 7 dilutions of 5-fold gradient were made, for a total of 8 gradients. Each of the dilutions was added in the plate at 100 µl/well, and incubated at 37°C for 1 h. The plate was washed with PBST for 5 times, added with a working solution of HRP-Goat anti-Huamn 10000X at 100 µl/well, and incubated at 37°C for 1 h. The plates were washed with PBST for 6 times, added with a freshly prepared color-developing solution at 100 µl/well, and incubated at 37°C for 10 min. The OD value was measured at 450 nm after addiing a stop solution (2M H₂SO₄) at 50 µl/well.

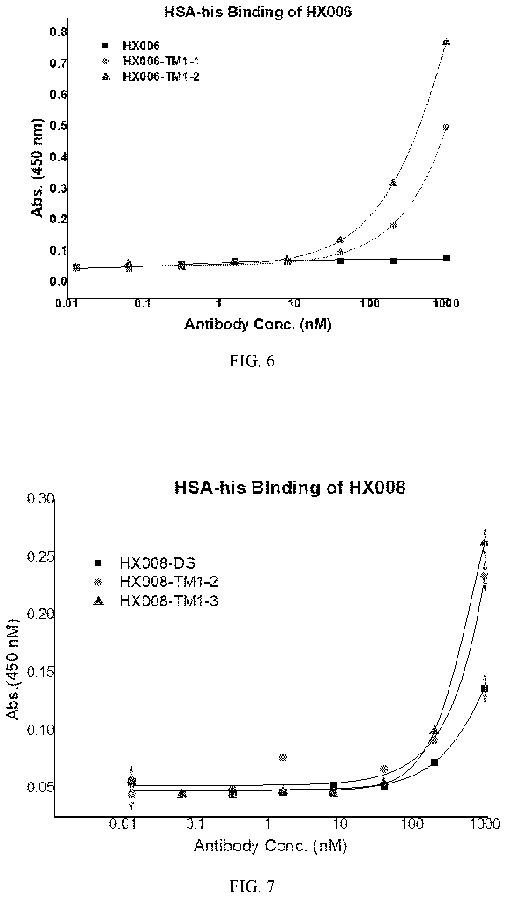

The results were shown in FIG. 6. The results showed that sample HX006-TM1 after conjugating to TM1 exhibited strong binding to HSA-his, and the binding activity increased with the increase of DAR value.

### Example 18. Binding activity of HX006-C18-tert-butanol ester to HSA

The binding of fusion HX006-C18-tert-butanol ester to HSA was detected by ELISA in this Example. 8 ELISA plates were coated with HSA-his that was diluted in a coating solution to 0.5 µg/ml, and incubated overnight at 4°C. The plates were washed with PBST (0.05% Tween 20 in PBS) for 3 times, then added with a blocking solution at 200 µl/well, and incubated at 37°C for 1 h. The plates were washed with PBST for 4 times. HX006-TM1 was diluted with diluent to 1000 nM as the initial concentration, and then 7 dilutions of 5-fold gradient were made, for a total of 8 gradients. Each of the dilutions was added in the plate at 100 µl/well, and incubated at 37°C for 1 h. The plates were washed with PBST for 5 times, added with a working solution of HRP-Goat anti-Huamn 10000X at 100 µl/well, and incubated at 37°C for 1 h. The plates were washed with PBST for 6 times, added with a freshly prepared color-developing solution at 100 µl/well, and incubated at 37°C for 10 min. The OD value was measured at 450 nm after adding a stop solution (2M H₂SO₄) at 50 µl/well.

The results were shown in FIG. 14. The results showed that the sample HX006-C18-tert-butanol ester after conjugating to C18-tert-butanol ester exhibited stronger binding to HSA-his, compared to naked antibody HX006-DS.

### Example 19. Binding activity of HX008-TM1 to HSA

The binding of fusion HX008-TM1 to HSA was detected by ELISA in this Example. See Example 15 for specific procedures, except that the test sample was changed to sample HX008-TM1-3. The results were shown in FIG. 7, wherein compared to naked antibody HX008-DS, the sample HX008-TM1 after conjugating to TM1 exhibited stronger binding to HSA-his, and the binding activity increased with the increase of DAR.

### Example 20. Effect of GLP-1-Fc-TM1 on the HEK293-CRE-Luc-GLP1R cell line

The biological activity of test substance I (GLP-1-Fc-TM1) was detected in cells HEK-293 (HEK293-CRE-Luc-GLP1R, available from Nanjing GenScript Biotechnology Co., Ltd.) that stably express the human GLP-1 receptor (GLP-1R) and the intracellular CRE-luciferase reporter gene (CRE4-luciferase). The biological activity of GLP-1-Fc-TM1 was detected by specifically binding of GLP-l-Fc to GLP-1R to produce cAMP, thereby activating the reporter gene. HEK293-CRE-Luc-GLP1R was recovered and then cultivated in DMEM (containing 10% FBS, 400 µg/ml G418, 200 µg/ml HygromycinB) in an incubator maintained at 37°C and 5% CO₂. The cells in the logarithmic growth phase were collected, counted, resuspended in complete medium, adjusted to the appropriate concentration, and seeded into a 384-well plate at 2×10³ cells/well, with 20 µl of cell suspension per well. The cells were incubated overnight in a 100% relative humidity incubator maintained at 37°C and 5% CO₂. The to-be-tested compound was diluted to the corresponding action concentration with the culture medium, and 30 µl of test substance solution was added per well (the final action concentration and dilution gradient of the to-be-tested compound depend on specific requirements), with a total of 9 concentration gradients, and 2 replicate wells for each concentration. Semaglutide (commercially available) and Dulaglutide (Homemade) were used as positive controls. The cells were incubated for 6 h in a 100% relative humidity incubator maintained at 37°C and 5% CO₂. The supernatant was discarded, an One-Glo test solution was added 40 µL/well and allowed to react in a Vortex shaker for 5 min, and luminescence (RLU) was measured on an ENVISION 2104 plate reader.

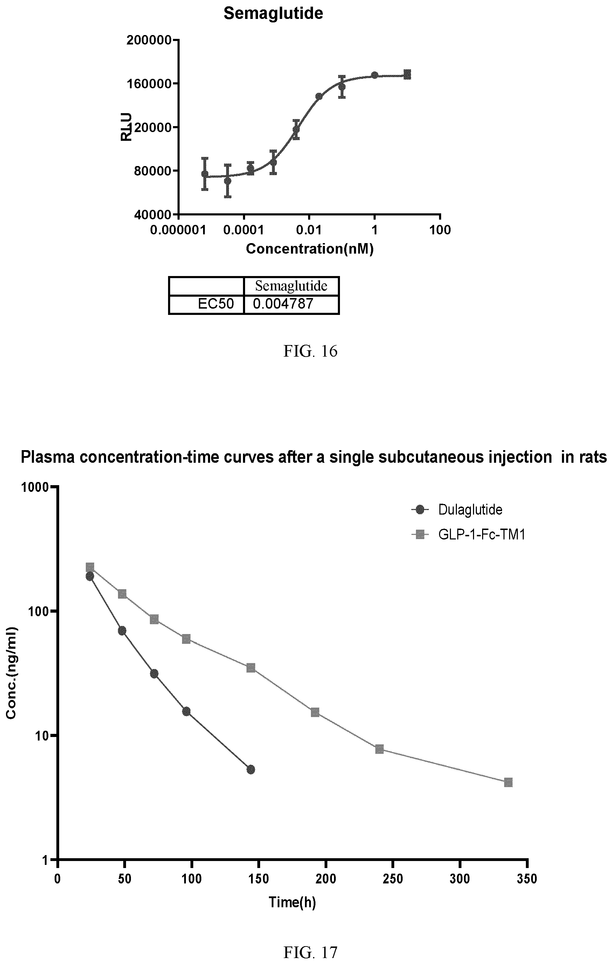

The results for the efficacy of activating CRE-luciferase reporter gene were shown in FIG. 16. Test substance I (GLP-1-Fc-TM1) has an EC₅₀ of 8.4×10⁻³nM , Dulaglutide has an EC₅₀ of 4.4×10⁻³nM, and Semaglutide has an EC₅₀ of 4.7×10⁻³nM, wherein the EC₅₀ of test substance I (GLP-1-Fc-TM1) was at the same order of magnitude as that of Dulaglutide and Semaglutide under the conditions of in vitro reporter gene assay, indicating that test substance I (GLP-1-Fc-TM1) had strong biological activity.

### Example 21. Interaction of a test substance with human serum albumin determined by surface plasmon resonance (SPR)

10 mM N- (2-hydroxyethyl) piperazine-N-2 sulfonic acid (HEPES), 150 mM sodium chloride (NaCl), 3 mM ethylenediaminetetraacetic acid (EDTA), and 0.005% Tween-20 were adjusted to pH 7.4 and used as the running reagents. A mouse anti-His antibody was diluted to 50 µg/mL with a fixing reagent (10 mM sodium acetate, pH 4.5). Firstly, the surface of the CM5 chip was activated with 400 mM EDC and 100 mM NHS at a flow rate of 10 µL/min for 420 s. Secondly, 50 µg/mL of the mouse anti-His antibody was injected into the channel at a flow rate of 10 µL/min for about 420 s, with a fixed amount of about 7000-15000 RU. Finally, the chip was blocked with 1M ethanolamine at 10 µL/min for 420 s. Human serum albumin (HSA) was subjected to buffer exchange using a desalting column and corresponding running reagents, and the concentration of the exchanged sample was measured using SPECTROstarNano. The ligands (test substances GLP-1-Fc, GLP-1-Fc-TM1, HX006-DS, HX006-TM1-1, HX006-TM1-2, HX008-DS, HX008-TM1-2, HX008-TM1-3) were diluted to 5 µg/mL with the running reagents and injected into the His capture chip assay channel (Fc2) at a flow rate of 10 µL/min, with an amount of about 400 RU. The capture of the ligands (test substances) was not required in the reference channel (Fc1). Human serum albumin (HSA) was subjected to 2-fold dilution with the running reagents, for a total of 7 concentrations, the diluted human serum albumin (HSA) was sequentially injected into the assay channel and the reference channel at a flow rate of 30 µL/min, with corresponding binding (120 s) and dissociation (300 s) times. The binding and dissociation steps were all performed in the running reagents. After each concentration analysis, the chip needed to be regenerated for 30 s with glycine hydrochloride at pH 1.5 at a flow rate of 20 µL/min, thus washing off the ligand and undissociated analyte. For the next concentration analysis, the same amount of ligands (test substances) needed to be recaptured in the assay channel. KD values for each sample were calculated using Biacore 8K analysis software Biacore Insight Evaluation Software. The reference channel (Fc1) was used for background subtraction.

The results showed the affinity of a test substance to human serum albumin detected by Biacore 8K as follows: a Kd value of the unmodified GLP-1-Fc, HX006-DS, or HX008-DS is 0, i.e., no binding; a Kd value of GLP-1-Fc-TM1, HX006-TM1-1, HX006-TM1-2, HX008-TM1-2, and HX008-TM1-3 is 1.01×10⁻² nM, 1.14×10⁻³ nM, 6.92×10⁻⁴ nM, 4.87×10⁻³ nM, and 4.05×10⁻³ nM, respectively, exhibiting strong affinity to human serum albumin.

### Example 22. Efficacy study of GLP-1-Fc-TM1 on type II diabetic db/db mice after multiple dosing

12 m/m mice (normal control) and 60 male db/db mice were purchased and adaptively raised; after the blood glucose of the db/db mice reached the standard (about 8-10 weeks old), 10 qualified m/m mice and 50 db/db mice were selected and divided into a normal control group (m/m mice), a model group, a positive control group (Dulaglutide), a low-dose test substance group, a medium-dose test substance group, and a high-dose test substance group, respectively, 10 mice per group. Mice were dosed after grouping, with the positive control group receiving 10 nmol/kg/dose of TRULICITY (Dulaglutide) by subcutaneous injection twice weekly for 4 weeks; the low-, medium-, and high-dose groups of the test substance respectively receiving 3, 10, and 30 nmol/kg/dose of test substance I (GLP-1-Fc-TM1) by subcutaneous injection twice weekly for 4 weeks; the db/db model group and the normal control group receiving corresponding vehicle (PBS) by subcutaneous injection twice weekly for 4 weeks. All groups were dosed at a volume of 5 ml/kg/dose. Clinical observations were done once daily, with serum insulin and glycated hemoglobin measured at the end of dosing, followed by an OGTT test. During the dosing period, the body weight and food intake were monitored twice weekly, random blood glucose was measured for 0 h (pre-dosing 0-60 min), 0.5 h, 2 h, 4h, 8 h, 24 h, 48 h, 72 h after the first dosing and the last dosing, and fasting blood glucose before dosing was measured once weekly (fasting for 4 h). At the end of the dosing period, random blood glucose, fasting blood glucose and serum insulin tests were measured.

The results showed that compared to the positive control drug (Dulaglutide), test substance I (GLP-1-Fc-TM1) exhibited overall better effects on reducing glycosylated hemoglobin, reducing random blood glucose and fasting blood glucose, stimulating the secretion of serum insulin, and reducing body weight and food intake; and low, medium, and high doses of test substance I (GLP-1-Fc-TM1) showed the efficacy being correlated with the dosage, indicating that test substance I (GLP-1-Fc-TM1) had a significant hypoglycemic effect.

Specifically, the test substance GLP-1-Fc-TM1 could dose-dependently reduce 4h-fasting blood glucose (FIG. 19-1), random blood glucose after the first and last dosing (FIG. 19-2 and FIG. 19-3), and blood glucose AUC₀₋₁₈₀ₘᵢₙ of OGTT test after the last dosing in db/db mice (FIG. 19-4 and FIG. 19-5), reduce the content of glycated hemoglobin in db/db mice (FIG. 19-6), raise insulin levels in db/db mice (FIG. 19-7), and reduce average daily intake in db/db mice (FIG. 19-8). At the same dose, the test substance GLP-1-Fc-TM1 showed better effect on the above indexes than that of the positive control Dulaglutide.

### Example 23. Efficacy study on the effect of GLP-1-Fc-TM1 on body weight of DIO model mice

72 male C57BL/6J mice were purchased, except 12 normal control mice (chow diet), the remaining 60 mice were given high-fat diet to prepare DIO model mice, which had free to food and water for about 10 weeks. During the molding period, food intake and body weight were monitored 2 times/week, the DIO model standard was reached when the body weight exceeded 20% of that of normal mice, followed by grouping. 10 qualified normal control group mice (chow diet) and 50 DIO model mice were selected and divided into groups including a normal control group (chow diet-fed mice), a model group, a positive control group (dulaglutide), a low-dose test substance group, a medium-dose test substance group, and a high-dose test substance group, 10 mice per group. Mice were dosed after grouping, with the positive control group receiving 10 nmol/kg/dose of TRULICITY (Dulaglutide) by subcutaneous injection twice weekly for 4 weeks; the low-, medium-, and high-dose groups of the test substance respectively receiving 3, 10, and 30 nmol/kg/dose of test substance I (GLP-1-Fc-TM1) by subcutaneous injection twice weekly for 4 weeks; the Obesity DIO model group and the normal control group receiving corresponding vehicle (PBS) by subcutaneous injection twice weekly for 4 weeks. All groups were dosed at a volume of 5 ml/kg/dose. All DIO mice continued to be fed with a high-fat diet during the dosing period, which lasted for the entire experimental period. Body weight and food intake were monitored 2 times per week during the dosing period. Before dosing, fasting blood glucose (fasting for 4 h) was measured, and blood was sampled to test four items of blood lipids and liver blood biochemistry. The first day of dosing recorded as D1, dosing for 28 day. After dosing (D28), 4h-fasting blood glucose was measured, blood was sampled and serum was isolated to test four items of blood lipids, liver blood biochemistry, and serum insulin levels. And finally, abdominal fat tissues were collected and weighed, the liver was harvested and weighed, and the organ coefficient was calculated by using the formula: organ coefficient = organ weight (g)/mouse weight (g). A part of the liver was harvested and fixed in formalin for histopathological examination (HE staining, oil red-O staining).

The results showed that compared with a positive control drug (dulaglutide), test substance I (GLP-1-Fc-TM1) exhibited overall better effects on reducing fasting blood glucose, improving four items of blood lipids, liver blood biochemical indexes and liver pathology, stimulating the secretion of serum insulin, and reducing weight and food intake; and the low, medium, and high doses of test substance I (GLP-1-Fc-TM1) showed the effects being correlated with the dosing, indicating that test substance I (GLP-1-Fc-TM1) had a significant hypoglycemic and antiobesity effects.

Specifically, GLP-1-Fc-TM1 could dose-dependently reduce the body weight of DIO mice (FIG. 20-1), reduce the food intake of DIO mice (FIG. 20-2), reduce the body fat content of DIO mice (FIG. 20-3), and reduce the fasting blood glucose (FIG. 20-4); in addition, the test substance GLP-1-Fc-TM1 could dose-dependently reduce the blood fat content (FIG. 20-5), and serum liver function indexes ALT and AST levels (FIG. 20-6), and showed the effect of improving hepatocyte balloon lesion and hepatocellular lipopathy in DIO mice (FIG. 20-7). Under the same dose, the test substance GLP-1-Fc-TM1 is more effective than the positive control Dulaglutide in improving hepatic steatosis.

### Example 24. Study of GLP-1-Fc-TM1 on Glucose Tolerance Test (IVGTT) of rats

58 male SD rats (6-8 weeks, weight 180-220 g) were purchased and adaptively raised for one week, 50 qualified animals were selected and divided into a vehicle control group, a positive control group (Dulaglutide), a low-dose test substance group, a medium-dose test substance group, and a high-dose test substance group, respectively, 10 mice per group. Mice were dosed after grouping, with the positive control group receiving a single dose of 10 nmol/kg of TRULICITY (Dulaglutide) by subcutaneous injection; the low-, medium-, and high-dose groups of the test substance respectively receiving a single dose of 3, 10, and 30 nmol/kg of test substance I (GLP-1-Fc-TM1) by subcutaneous injection; the vehicle control group receiving a single dose of corresponding vehicle (PBS) by subcutaneous injection. All groups were dosed at a volume of 5 ml/kg/dose. Rats were fasted (but not water deprivation) for 16 h, then dosed by intravenous injection, and after 24 h and 72 h, dosed with glucose (0.5 g/kg) by intravenous injection for stimulation; blood samples were collected at 2, 4, 6, 10, 20 and 30 min after glucose injection, respectively, blood glucose concentration and insulin level were measured, and AUC was calculated.

The results showed that compared with a positive control drug (Dulaglutide), test substance I (GLP-1-Fc-TM1) showed equivalent or no significant differences on reducing fasting blood glucose and stimulating the secretion of serum insulin; and the low, medium, and high doses of test substance I (GLP-1-Fc-TM1) showed the effects being correlated with the dose.

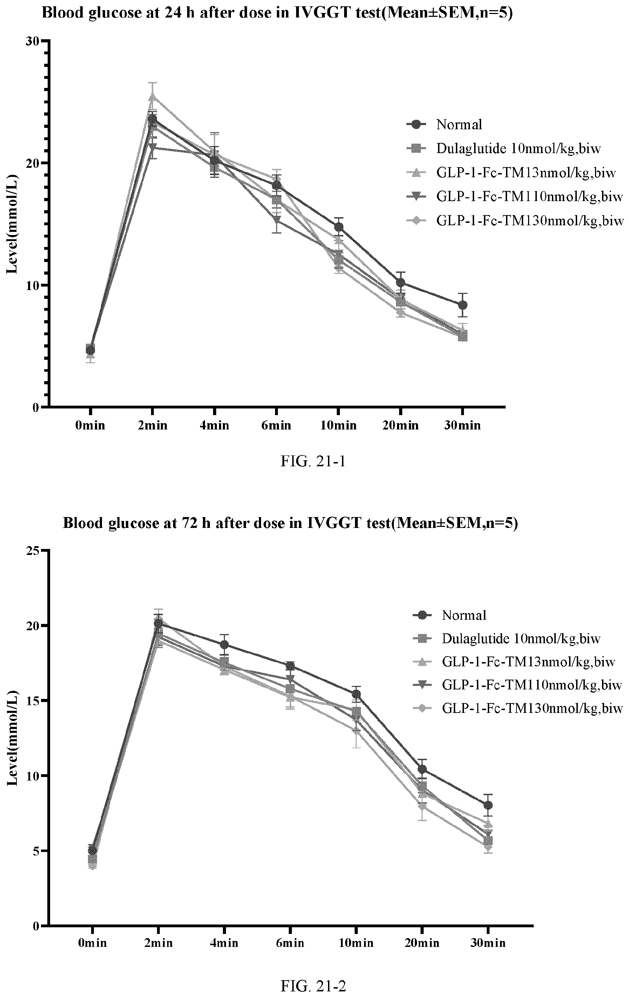

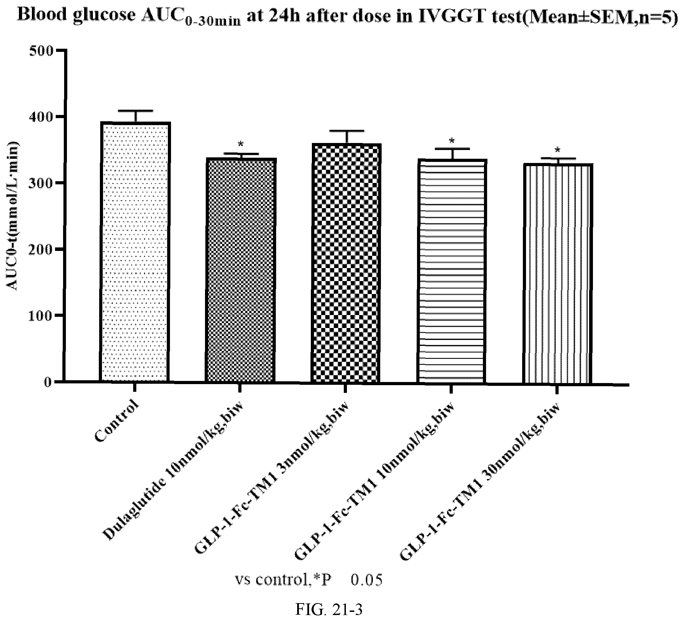

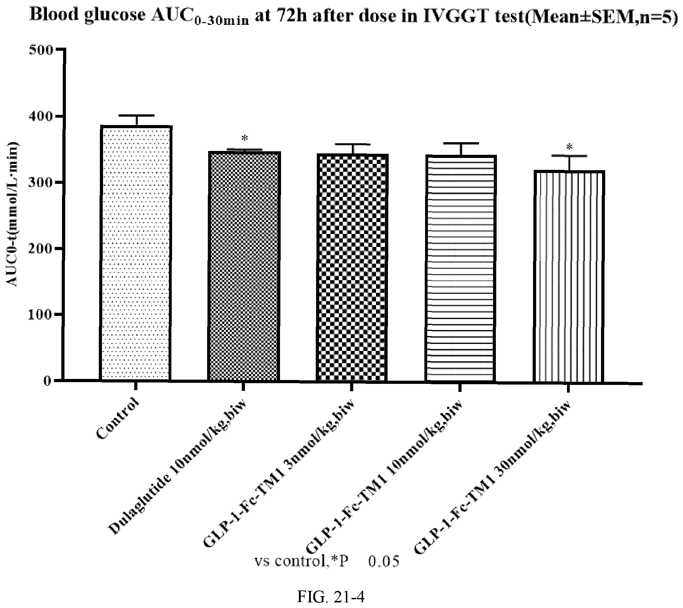

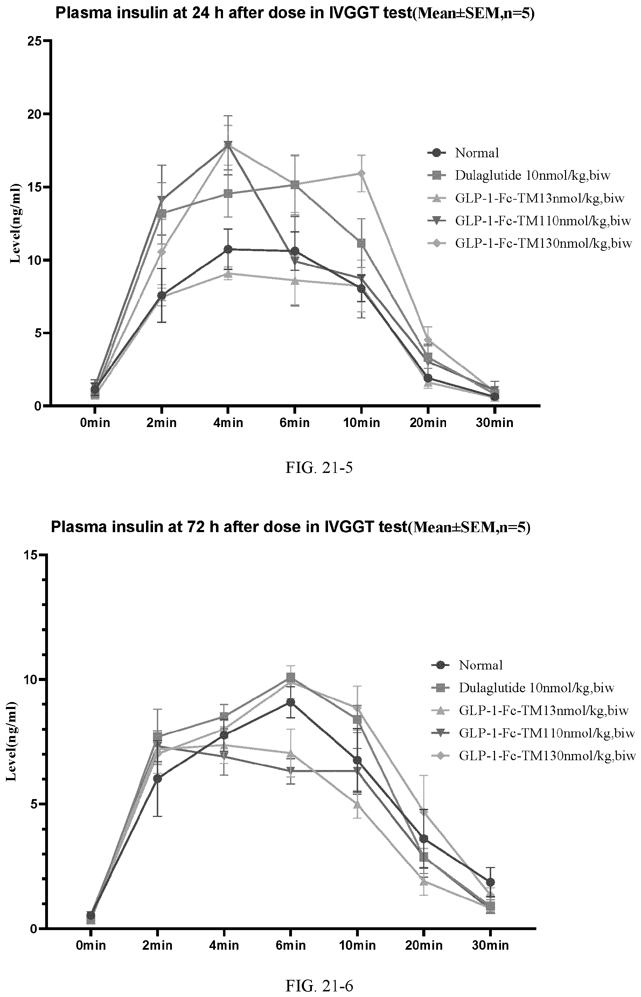

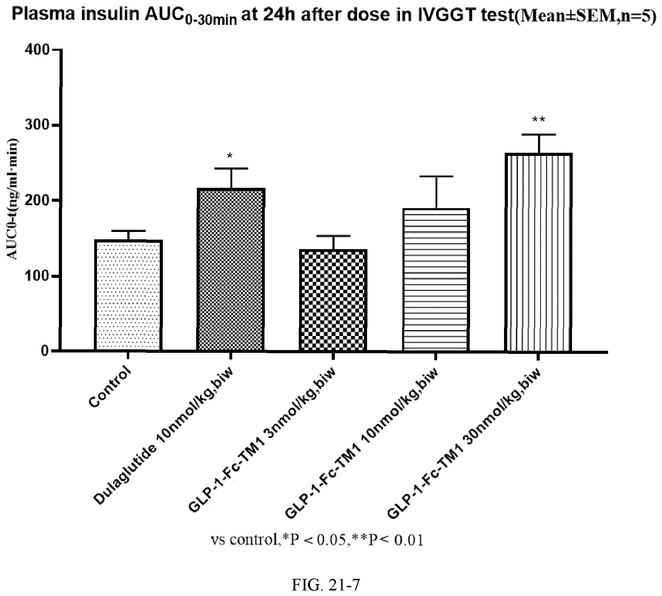

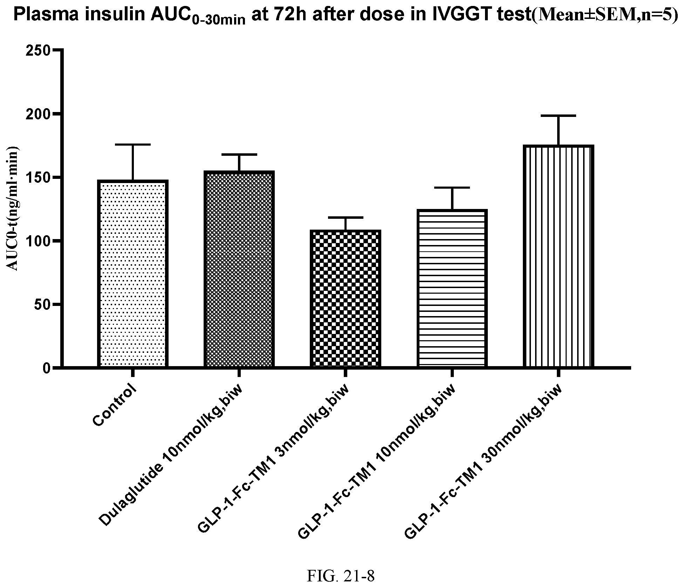

Specifically, the test substance GLP-1-Fc-TM1, which was administered in single dose of 3, 10, 30 nmol/kg, could both dose-dependently reduce the blood glucose AUC₀₋₃₀ₘᵢₙ (FIG. 21-1, FIG. 21-2, FIG. 21-3, and FIG. 21-4) and elevate the serum insulin AUC₀₋₃₀ₘᵢₙ (FIG. 21-5, FIG. 21-6, FIG. 21-7, and FIG. 21-8) in SD rats after administration 24 h and 72 h.

### Example 25. Pharmacokinetic study on single subcutaneous injection of test substance in SD rats

12 SD rats (6-8 weeks, weight 180-220 g) with half male and half female, were adaptively raised, then randomly divided into 2 groups, namely a test substance I (GLP-1-Fc-TM1) group and a positive control group (Dulaglutide, Homemade), 6 mice per group, with half male and female. A single subcutaneous injection of test substance I (GLP-1-Fc-TM1) and positive control (Dulaglutide) was administered at a dose of 0.1 mg/kg and a volume of 4 ml/kg. Approximately 0.5 mL of blood was collected from jugular vein before adminstration, and at 24 hours (± 10 minutes, day 1), 48 hours (± 10 minutes, day 2), 72 hours (± 15 minutes, day 3), 96 hours (± 15 minutes, day 4), 144 hours (± 15 minutes, day 6), 192 hours (± 30 minutes, day 8), 240 hours (± 30 minutes, day 10), and 336 hours (± 30 minutes, day 14) after administration, and anticoagulated with EDTA-K₂. The concentrations of test substance I (GLP-1-Fc-TM1) and positive control (Dulaglutide) were measured in plasma at each time point by ELISA. The standard curve range of 3 test substances ranged from 3.13 to 200 ng/mL, and the pharmacokinetic parameters were calculated by adopting Phoenix WinNonlin 8.2.

The results were shown in FIG. 17 and Table 1: after a single subcutaneous injection of 0.1 mg/kg of test substance I (GLP-1-Fc-TM1) and Dulaglutide in SD rats, mean Tₘₐₓ of test substance I (GLP-1-Fc-TM1) and Dulaglutide in plasma was 24 h, mean Cₘₐₓ of test substance I (GLP-1-Fc-TM1) and Dulaglutide was 225 and 191 ng/mL, mean T_{1/2} of test substance I (GLP-1-Fc-TM1) and Dulaglutide was 45 and 28 h, mean AUC₀₋ₜ of test substance I (GLP-1-Fc-TM1) and Dulaglutide was 15700 and 7540 h*ng/mL, mean AUC_{0-∞} of test substance I (GLP-1-Fc-TM1) and Dulaglutide was 16100 and 7840 h*ng/mL, mean Vz__{F_obs} of test substance I (GLP-1-Fc-TM1) and Dulaglutide was 428 and 533 mL/kg, mean Cl_{_F_obs} of test substance I (GLP-1-Fc-TM1) and Dulaglutide was 6.42 and 13.3 mL/h/kg, MRT₍₀₋ₜ₎ of test substance I (GLP-1-Fc-TM1) and Dulaglutide was 70 and 39 h, and MRT_{(0-∞)} of test substance I (GLP-1-Fc-TM1) and Dulaglutide was 77 and 44 h, respectively. The half-life T_{1/2}, Cₘₐₓ, AUCₗₐₛₜ, AUC_{0-∞}, Vz_{_F}, Cl, MRT₍₀₋ₜ₎, MRT_{(0-∞)} of test substance I (GLP-1-Fc-TM1) were respectively 1.61, 1.18, 2.08, 2.05, 0.80, 0.48, 1.79, and 1.75 times of those of Dulaglutide, indicating that test substance I (GLP-1-Fc-TM1) could extend the half-life by reducing the clearance rate.

| Table 1 Pharmacokinetic parameters after a single subcutaneous injection in SD rats (Mean±SD) | | | | |
|---|---|---|---|---|
| PK parameters | Unit | GLP-1-Fc-TM1 | Dulaglutide | Ratio (GLP-1-Fc-TM1/Dulaglutide) |
| Rsq_adjusted | | 0.99±0.01 | 0.92±0.07 | 1.08 |
| Tmax | hr | 24±0 | 24±0 | 1.00 |
| Cmax | ng/mL | 225±31 | 191±31 | 1.18 |
| T_{1/2} | hr | 45±12 | 28±5 | 1.61 |
| AUC₍₀₋ₜ₎ | hr*ng/mL | 15700±3120 | 7540±1890 | 2.08 |
| AUC_{(0-∞)} | hr*ng/mL | 16100±3010 | 7840±1880 | 2.05 |
| Vz_F_obs | mL/kg | 428±157 | 533±148 | 0.80 |
| Cl_F_obs | mL/hr/kg | 6.42±1.21 | 13.30±2.96 | 0.48 |
| MRT₍₀₋ₜ₎ | hr | 70±6 | 39±4.2 | 1.79 |
| MRT_{(0-∞)} | hr | 77±7 | 44±4 | 1.75 |

### Example 26. Pharmacokinetic study on single subcutaneous injection of test substance in Cynomolgus Macaques

4 Cynomolgus Macaques (5-6 years old, weight approximately 6.5 kg) with half male and half female, were adaptively raised, then randomly divided into 2 groups, namely a reference formulation control group (Dulaglutide, a commercially available drug, Trulicity) and a test substance I (GLP-1-Fc-TM1) group, 2 mice per group, with half male and female. A single subcutaneous injection of reference formulation control Dulaglutide and test substance I (GLP-1-Fc-TM1) was administered at a molar dose of 1.676 nmol/kg (i.e., Dulaglutide with a dose of 0.1 mg/kg and GLP-1-Fc-TM1 with a dose of 0.101mg/kg) and a volume of 4 ml/kg. Approximately 0.5 mL of blood was collected from jugular vein before administration and at 4 h, 8 h, 24 h, 48 h, 96 h, 144 h, 240 h, and 336 h after administration, and anticoagulated with EDTA-K₂. The concentrations of the reference formulation control Dulaglutide and test substance I (GLP-1-Fc-TM1) in the plasma at each time point were determined by ELISA. Wherein the standard curve of the test substance had a detection limit LLOQ =15.625 ng/mL, and the pharmacokinetic parameters were calculated by adopting Phoenix WinNonlin 8.2.

The results were shown in FIG. 18 and Table 2: after a single subcutaneous injection of 676 nmol/kg of test substance I (GLP-1-Fc-TM1) and reference formulation control Dulaglutide in Cynomolgus Macaques, mean Tₘₐₓ of test substance I (GLP-1-Fc-TM1) and Dulaglutide in plasma was 16 and 8 h, mean Cₘₐₓ of test substance I (GLP-1-Fc-TM1) and Dulaglutide was 760 and 602 ng/mL, mean T_{1/2} of test substance I (GLP-1-Fc-TM1) and Dulaglutide was 97.9 and 48.2 h, mean AUC₀₋ₜ of test substance I (GLP-1-Fc-TM1) and Dulaglutide was 98546 and 49118 h*ng/mL, mean AUC_{0-∞} of test substance I (GLP-1-Fc-TM1) and Dulaglutide was 140678 and 50947 h*ng/mL, mean Vz_F_obs of test substance I (GLP-1-Fc-TM1) and Dulaglutide was 95.7 and 136.8 mL/kg, mean Cl_F_obs of test substance I (GLP-1-Fc-TM1) and Dulaglutide was 0.74 and 1.97 mL/h/kg, MRT₍₀₋ₜ₎ of test substance I (GLP-1-Fc-TM1) and Dulaglutide was 88 and 68 h, and MRT_{(0-∞)} of test substance I (GLP-1-Fc-TM1) and Dulaglutide was 163 and 77 h, respectively. The half-life, Tₘₐₓ, Cₘₐₓ, T_{1/2}, AUC₀₋ₜ, AUC_{0-∞}, Vz-F, Cl, MRT₍₀₋ₜ₎, and MRT_{(0-∞)} of test substance I (GLP-1-Fc-TM1) were respectively 2.00, 1.26, 2.03, 2.01, 2.76, 0.70, 0.38, 1.29, and 2.12 times of those of Dulaglutide, indicating that test substance I (GLP-1-Fc-TM1) could extended the half-life by reducing the clearance rate.

| Table 2 Pharmacokinetic parameters after a single subcutaneous injection in Cynomolgus Macaques (Mean±SD) | | | | |
|---|---|---|---|---|
| PK parameters | Unit | GLP-1-Fc-TM1 | Dulaglutide | Ratio (GLP-1-Fc-TM1/Dulaglutide) |
| Rsq_adjusted | | 0.87±0.07 | 0.99±0.01 | 0.88 |
| Tmax | hr | 16±11.3 | 8±0 | 2.00 |
| Cmax | ng/mL | 760±54 | 602±154 | 1.26 |
| T_{1/2} | hr | 97.9±68.5 | 48.2±2.2 | 2.03 |
| AUC₍₀₋ₜ₎ | hr*ng/mL | 98546±22983 | 49118±3131 | 2.01 |
| AUC_{(0-∞)} | hr*ng/mL | 140678±33672 | 50947±3855 | 2.76 |
| Vz_F_obs | mL/kg | 95.7±48.1 | 136.8±4.1 | 0.70 |
| Cl_F_obs | mL/hr/kg | 0.74±0.18 | 1.97±0.15 | 0.38 |
| MRT₍₀₋ₜ₎ | hr | 88±32 | 68±9 | 1.29 |
| MRT_{(0-∞)} | hr | 163±67 | 77±11 | 2.12 |

## Claims

1. A conjugated molecule having a structure of "active molecule-Fc-Cn", wherein the active molecule is selected from any molecules beneficial to an organism, Fc is an immunoglobulin IgG Fc, and Cn is a modified moiety comprising a C₁₄-₂₄ fatty acid chain.

2. A conjugated molecule having a structure of "antibody-Cn", wherein Cn is a modified moiety comprising a C₁₄-₂₄ fatty acid chain.

3. A conjugated molecule having a structure of "active molecule-fusion protein-Cn", wherein the active molecule is selected from any molecules beneficial to an organism and Cn is a modified moiety comprising a C₁₄-₂₄ fatty acid chain.

4. The conjugated molecule according to any of claims 1 to 3, wherein Cn has the structure of Formula (I):
-Z-Y (I),
wherein
Z has the following structure:
-Z₁-Z₂-Z₃-Z₄-,
wherein Z₁ is a sulfur atom or a nitrogen atom or an oxygen atom in Fc,
Z₂ is -C( = O)- or 5-10 membered heterocyclyl, preferably containing 1 or 2 heteroatoms selected from N, S, and O;
Z₃ is selected from the group consisting of a bond, -C(=O)-, -C₁-C₁₀ alkylene-C(= O)-, -C₃-C₁₀ alkynylene-C(=O)-, -C₃-C₁₀ alkenylene-C(=O)-, -C₁-C₁₀ heteroalkylene-C(=O)-, -C₃-C₈ cycloalkylene-C( = O)-, -O-C₁-C₈ alkylene-C( = O)-, - arylidene-C( = O)-, -C₁-C₁₀ alkylene- arylidene-C( = O)-, -arylidene-C₁₋C₁₀ alkylene-C( = O)-, -C₁-C₁₀ alkylene-C₃-C₈ cycloalkylene-C( = O)-, -C₃-C₈ cycloalkylene-C₁-C₁₀ alkylene-C( = O)-, -C₃-C₈ heterocyclylene-C( = O)-, -C₁-C₁₀ alkylene-C₃-C₈ heterocyclylene-C( = O)-, -C₃-C₈ heterocyclylene-C₁-C₁₀ alkylene-C( = O)-, wherein the alkylene, alkynylene, alkenylene, heteroalkylene, cycloalkylene, arylidene, and heterocyclylene are substituted optionally;
Z₄ is a bond or a PEG unit represented by the following Formula, 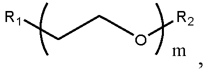 wherein R₁ is selected from the group consisting of C₁₋₄ alkylene, -NH-, -NH-C₁₋₄ alkylene-, -NH-C₁₋₄ alkylene-heteroaryl-, wherein heteroaryl is 5-membered or 6-membered nitrogen-containing heteroaryl; R₂ is-C(=O)-, -C₁₋₄ alkylene, -C₁₋₄ alkylene-C(=O)-, -C₁₋₄ alkylene-NH-C(=O)-(CH₂OCH₂)ₚ-C₁₋₄ alkylene-, -C₁₋₄ alkylene-C(=O)-NH-(CH₂OCH₂)ₚ-C₁₋₄ alkylene-, wherein m is an integer from 2 to 6, p is an integer from 1 to 3,
Y is 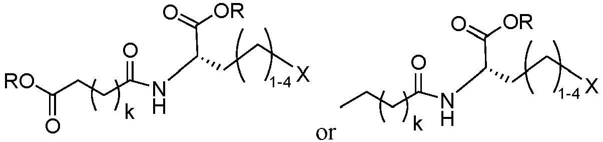
wherein Y is linked to Z₄ via X, k is an integer from 10 to 30,
wherein R independently represents hydrogen, C₁₋₆ alkyl, C₁₋₆ aminoalkyl, C₁₋₆ haloalkyl, C₁₋₆ hydroxyalkyl.

5. The conjugated molecule according to any one of claims 1 to 4, wherein Z₂ is maleimido group 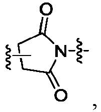 wherein the wavy line on the left side indicates the position to which Z₁ is linked; and the wavy line on the right side indicates the position to which Z3 is linked.

6. The conjugated molecule according to any one of claims 1 to 5, wherein Z₃ is-C₁-C₁₀ alkylene-C(=O)-, wherein the alkylene is optionally substituted and wherein Z₃ is linked to Z₄ via -C-(=O)-.

7. The conjugated molecule according to any one of claims 1 to 6, wherein Z₂ is a maleimido group and Z₃ is -C₁₋₆ alkylene-C(=O)-.

8. The conjugated molecule according to any one of claims 1 to 7, wherein Z₄ is a bond and Z₃ is directly linked to Y in Formula (I).

9. The conjugated molecule according to any of claims 1 to 8, wherein Z₄ is a PEG unit represented by the following Formula: 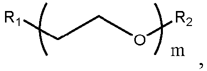 wherein R₁ is selected from the group consisting of -NH- and -NH-C₁₋₄ alkylene-; R₂ is -C₁₋₄ alkylene or -C₁₋₄ alkylene-NH-C(=O)-(CH₂OCH₂)ₚ-C₁₋₄ alkylene-, wherein m is an integer from 2 to 6 and p is an integer from 1 to 3.

10. The conjugated molecule according to any of claims 1 to 9, wherein Z₄ is a unit comprising 2 to 6 PEGs.

11. The conjugated molecule according to any of claims 1 to 10, wherein Z₄ is 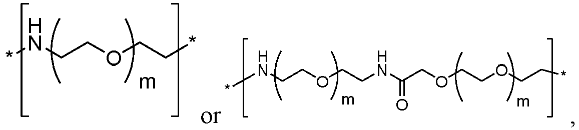 wherein m =1-4, the asterisk on the left side indicates the position to which Z₃ is linked; and the asterisk on the right side indicates the position to which Y is linked in Formula II.

12. The conjugated molecule according to any of claims 1 to 11, wherein Z in Formula (I) has the following structure: 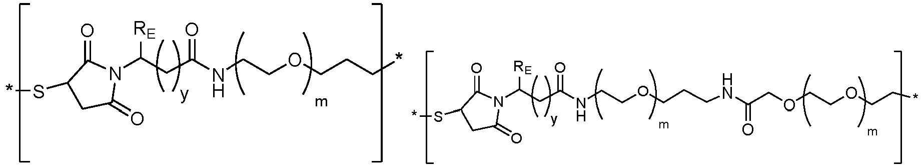 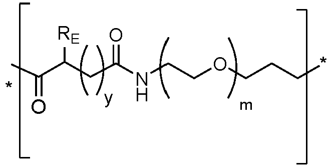 or 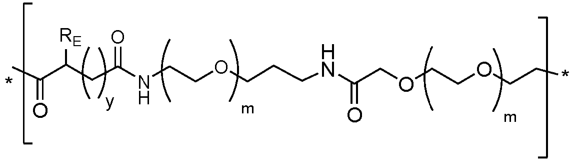 wherein R_{E} is hydrogen, C₁₋₆ alkyl, C₁₋₆ aminoalkyl, C₁₋₆ haloalkyl, C₁₋₆ hydroxyalkyl, wherein y =0-4, m =1-4, wherein the asterisk on the left side indicates the position to which Ab is linked and the asterisk on the right side indicates the position to which Y is linked.

13. The conjugated molecule according to any one of claims 1 to 12, wherein Y is linked to Z₄ via X, and X is -NH-(C=O)- or -(C=O)-NH-.

14. The conjugated molecule according to any one of claims 1 to 13, wherein Cn comprises a C₁₆ fatty acid chain, a C₁₈ fatty acid chain, or a C₂₀ fatty acid chain.

15. The conjugated molecule according to any of claims 1 to 14, wherein Cn comprises a C₁₈ fatty acid chain and is conjugated to the sulfur atom of the free thiol group of Fc.

16. The conjugated molecule according to any of claims 1 to 15, wherein Cn is selected from

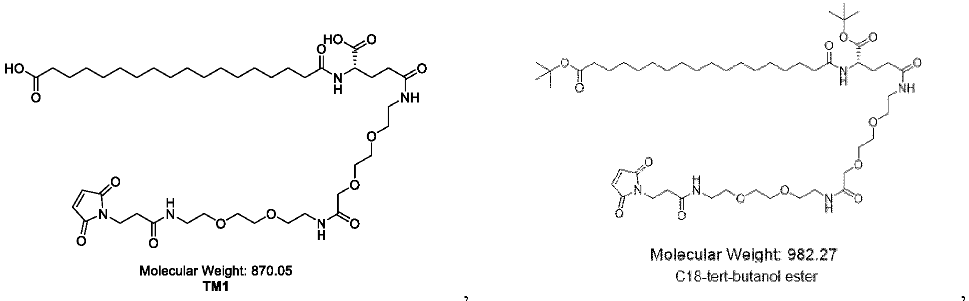

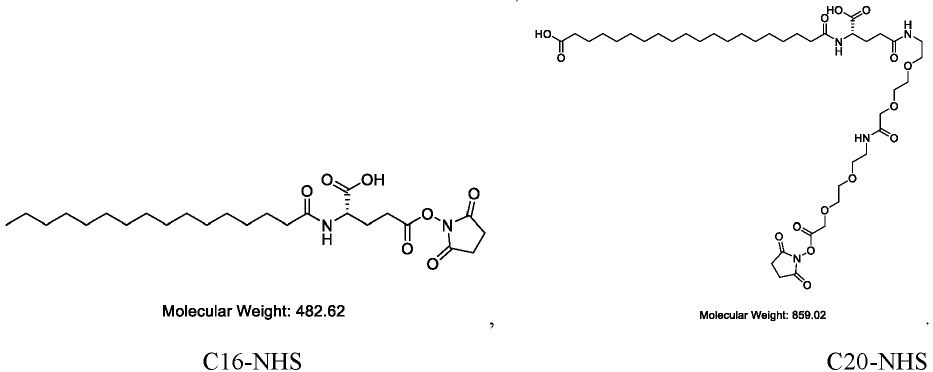

17. The conjugated molecule according to any one of claims 1 to 16, wherein the Fc is IgG1 Fc or IgG4 Fc, preferably human IgG1 Fc or human IgG4 Fc.

18. The conjugated molecule according to any of claims 1 to 16, wherein the Fc-region further comprises an immunoglobulin IgG hinge region.

19. The conjugated molecule according to any of claims 1 to 17, wherein the Fc-region has a modification at an amino acid selected from positions 228, 233, 234, 235, 252, 254, 256, 297, 307, 308, 311, 380, 385, 386, 389, 428, 434, and 447 (according to EU numbering).

20. The conjugated molecule according to claim 19, wherein the modification is to replace amino acids at positions 254, 308 and 434 with Thr, Pro and Ala, respectively (according to EU numbering).

21. The conjugated molecule according to claim 20, wherein the modification is S228P, F234A and L235A, or S228P, F234A, L235A and 447 deletion (according to EU numbering).

22. The conjugated molecule according to any one of claims 1 to 21, wherein the Fc-region comprises or consists of the sequence as set forth in SEQ ID NOs: 10, 15 or 16.

23. The conjugated molecule according to any one of claims 1 to 22, wherein the active molecule is an active peptidic molecule fused to the Fc-region either directly or via a peptide linker.

24. The conjugated molecule according to any one of claims 1 to 23, wherein the C-terminus of the active peptidic molecule is fused to the N-terminus of the Fc-region, or the N-terminus of the active peptidic molecule is fused to the C-terminus of the Fc-region.

25. The conjugated molecule according to any one of claims 1 to 24, wherein the active molecule is selected from an enzyme, an enzyme inhibitor, an antigen, an antibody or antibody fragment, a hormone, glucagon-like peptide-1 (GLP-1), glucagon, an interferon, a cytokine, a growth factor and/or a differentiation factor, a factor involved in cell motility or migration, a factor involved in bone formation/resorption, a chemokine, a plasma or interstitial adhesion molecule or extracellular matrix, a bactericidal or antifungal factor.

26. The conjugated molecule according to any one of claims 1 to 25, wherein the active molecule is selected from GLP-1, an antibody or an antigen-binding fragment thereof.

27. The conjugated molecule according to any one of claims 1 to 26, wherein the active molecule is selected from an anti-PD-1 antibody or an antigen-binding fragment thereof, an anti-VEGF antibody or an antigen-binding fragment thereof.

28. The conjugated molecule according to any one of claims 1 to 27, wherein the peptide linker comprises the amino acid sequence (G₄S)ₙ, wherein n is an integer equal to or greater than 1, preferably the peptide linker comprises(G₄S)₃, (G₄S)₄, (G₄S)₆, GS(G₄S)₄, DAAALEAAALDAAAREAAARDAAAL, NVDHLPSNTLVDLA, (G₃S)2, (G₄S)₂, (G₃S)₃, (G₄S)₃, (G₃S)₄, (G₄S)₄, (G₃S)₅, (G₄S)₅, (G₃S)₆, (G₄S)₆, GGG, DGGGS, TGEKP, GGRR, EGKSSGSGSESKVD, KESGSVSSEQLAQFRSLD, GGRRGGGS, LRQRDGERP, LRQKDGGGSERP, and GSTSGSGK PGSGEGSTKG.

29. The conjugated molecule according to any one of claims 1 to 28, wherein the Fc comprises the sequence as set forth in SEQ ID NOs: 10,15 or 16.

30. The conjugated molecule according to any one of claims 1 to 29, which is GLP-1-IgG4 Fc-TM1, GLP-1-IgG4 Fc-C18 tert-butanol ester, GLP-1-IgG4 Fc-C16-NHS, and GLP1-IgG4 Fc-C20-NHS.

31. The conjugated molecule according to claim 30, wherein IgG4 Fc comprises the sequence as set forth in SEQ ID NO: 16.

32. The conjugated molecule according to claim 30 or 31, wherein the GLP-1-IgG4 Fc moiety in GLP-1-IgG4 Fc-TM1, GLP-1-IgG4 Fc-C18 tert-butanol ester, GLP-1-IgG4 Fc-C16-NHS, and GLP1-IgG4 Fc-C20-NHS conjugated molecule comprises the amino acid sequence as set forth in SEQ ID NO: 1.

33. The conjugated molecule according to claim 27, wherein the anti-PD-1 antibody or antigen-binding fragment thereof comprises a heavy chain variable region as set forth in SEQ ID NO: 9 and a light chain variable region as set forth in SEQ ID NO: 11.

34. The conjugated molecule according to claim 33, wherein the anti-PD-1 antibody or antigen-binding fragment thereof comprises a heavy chain as set forth in SEQ ID NO: 8 and a light chain as set forth in SEQ ID NO: 12.

35. The conjugated molecule according to claim 33 or 34, wherein the Fc comprises the sequence as set forth in SEQ ID NO: 10.

36. The conjugated molecule according to claim 35, which is a HX008-TM1 conjugated molecule, preferably a HX008-TM1-2 conjugated molecule or a HX008-TM1-3 conjugated molecule.

37. The conjugated molecule according to claim 27, wherein the anti-VEGF antibody or antigen-binding fragment thereof comprises 3 heavy chain CDRs as set forth in SEQ ID NOs: 2, 3, and 4, and 3 light chain CDRs as set forth in SEQ ID NOs: 5, 6, and 7.

38. The conjugated molecule according to claim 37, wherein the anti-VEGF antibody or antigen-binding fragment thereof comprises a heavy chain as set forth in SEQ ID NO: 13 and a light chain as set forth in SEQ ID NO: 14.

39. The conjugated molecule according to claim 37 or 38, wherein the Fc comprises the sequence as set forth in SEQ ID NO: 15.

40. The conjugated molecule according to any one of claims 37 to 39, which is a HX006-TM1 conjugated molecule, a HX006-C18-tert-butanol ester conjugated molecule, a HX006-C16-NHS conjugated molecule, and a HX006-C20-NHS conjugated molecule.

41. A method for the preparation of a conjugated molecule having a structure of "active molecule-Fc-Cn", comprising
(a) linking an active molecule polypeptide to an immunoglobulin Fc-region to prepare an "active molecule-Fc" fusion; and
(b) subjecting the "active molecule-Fc" fusion and Cn containing a fatty acid chain to a conjugating reaction under the conditions that allow the conjugation of the Fc-region to Cn, resulting in an "active molecule-Fc-Cn" conjugated molecule.

42. A method for the preparation of a conjugated molecule having a structure of "active molecule-Fc-Cn", comprising the steps of
(a) linking the antibody Fab fragment to an immunoglobulin Fc-region to prepare a "Fab-Fc" fusion, and
(b) subjecting the "Fab-Fc" fusion to a conjugating reaction under the conditions that allow the conjugation of the Fc-region to Cn containing a fatty acid chain, resulting in a "Fab-Fc-Cn" conjugated molecule.

43. A method for the preparation of a conjugated molecule having a structure of "active molecule-Fc-Cn" or having a structure of "antibody-Cn", comprising the steps of subjecting a whole antibody to a conjugating reaction under the conditions that allow the conjugation of the Fc-region to Cn containing a fatty acid chain, resulting in an "active molecule-Fc-Cn" conjugated molecule.

44. A pharmaceutical composition comprising the conjugated molecule of any one of claims 1 to 40.

45. A method for effectively extending the serum half-life of an active molecule, comprising the step of constructing the active molecule as a conjugated molecule having a structure of "active molecule-Fc-Cn" or having a structure of "antibody-Cn" according to the method of any one of claims 41 to 43, thereby effectively increasing the serum half-life of the active molecule.

46. Use of the conjugated molecule of any one of claims 1 to 40 or the pharmaceutical composition of claim 44 in the manufacture of a medicament for the treatment of a human disease.

47. A method for the treatment of a human disease, comprising administering an effective amount of the conjugated molecule of any one of claims 1 to 40 or the pharmaceutical composition of claim 44 to a subject.
